# EUROPEAN PATENT APPLICATION

(11) **EP 1 627 887 A2**
(43) Date of publication of application: **22.02.2006**
(21) Application number: 05013872.6
(22) Date of filing: 28.06.2005
(51) Int. Cl.: C07K 14/72, C12N 15/12, C12N 15/63, G01N 33/53, A01K 67/027

(54) **Mouce trace amine associated receptors**

(30) Priority: 08.07.2004 EP 04103262
(71) Applicant: F.HOFFMANN-LA ROCHE AG, 4070 Basel (CH)
(72) Inventor: Ebeling, Martin, 79639 Grenzach-Wyhlen (DE); Hoener, Marius, 4056 Basel (CH); Lindemann, Lothar, 4057 Basel (CH)

(57) **Abstract**

The present invention provides a fingerprint sequence which is specific and selective for trace amine associated receptors (TAAR) forming a subfamily of G protein coupled receptors. The invention also provides the novel mouse polypeptides identified as members of this family, nucleic acids encoding said polypeptides, and vectors and host cells comprising the novel family members. In addition, the invention provides methods of identifying TAARs.

SEQUENCE LISTING

<110>  F.Hoffmann-La Roche

<120>  Mouse Trace Amine Receptors

<130>  22646

<160>  66

<170>  PatentIn version 3.2

<210>  1
<211>  16
<212>  PRT
<213>  Mus musculus


<220>
<221>  fingerprint
<222>  (1)..(16)
<223>  Zaa may be tyrosine or histidine

       Baa may be tyrosine or phenylalanine

<220>
<221>  misc_feature
<222>  (3)..(4)
<223>  Xaa can be any naturally occurring amino acid

<220>
<221>  misc_feature
<222>  (7)..(8)
<223>  Xaa can be any naturally occurring amino acid

<220>
<221>  misc_feature
<222>  (10)..(12)
<223>  Xaa can be any naturally occurring amino acid

<220>
<221>  misc_feature
<222>  (14)..(14)
<223>  Xaa can be any naturally occurring amino acid

<400>  1

Asn Ser Xaa Xaa Asn Pro Xaa Xaa Zaa Xaa Xaa Xaa Baa Xaa Trp Phe
1               5                   10                  15


<210>  2
<211>  999
<212>  DNA
<213>  Mus musculus


<220>
<221>  mTAAR1
<222>  (1)..(999)

<400>  2
atgcatcttt gccacgctat cacaaacatt tcccacagaa acagcgactg gtcaagagaa
60

gtccaagctt ccctgtacag cttaatgtca ctcataatcc tggccactct ggttggcaac
120

ttaatagtaa taatttccat atcccatttc aagcaacttc atacacccac caactggctc
180

cttcactcca tggccattgt cgactttctg ctgggctgtc tgataatgcc ctgcagcatg
240

gtgagaactg ttgagcgctg ttggtatttt ggggaaatcc tctgtaaagt tcacaccagc
300

accgatatca tgctgagctc cgcctccatt ttccacttag ctttcatttc cattgaccgc
360

tactgtgctg tgtgtgaccc tttgagatac aaagccaaga tcaatatctc cactattctt
420

gtgatgatcc tcgttagttg gagccttcct gctgtttatg catttgggat gatcttcctg
480

gaactgaact taaaaggagt ggaagagctg tatcgcagtc aggtcagcga cctgggcggc
540

tgttctccct tctttagtaa agtatctggg gtactggcgt tcatgacttc cttctatata
600

cctggatctg ttatgttatt tgtttactat aggatatatt tcatagctaa aggacaagca
660

aggtcaatca atcgtacgaa tgttcaagtt ggattggaag ggaaaagcca agcaccacaa
720

agcaaggaaa caaaagccgc gaagacctta gggatcatgg tgggcgtttt cctcgtatgc
780

tggtgcccgt tctttctctg cacggtcctg gaccctttcc tgggctatgt tatcccaccc
840

tctctgaatg acgcactgta ttggtttggg tacttgaatt ctgccctcaa tccgatggtt
900

tatgcctttt tctatccctg gttcagaaga gccttgaaga tggttctcct tggtaaaatt
960

ttccaaaaag attcatctag gtctaagcta tttttgtaa
999


<210>  3
<211>  332
<212>  PRT
<213>  Mus musculus


<220>
<221>  mTAAR1
<222>  (1)..(332)

<400>  3

Met His Leu Cys His Ala Ile Thr Asn Ile Ser His Arg Asn Ser Asp
1               5                   10                  15


Trp Ser Arg Glu Val Gln Ala Ser Leu Tyr Ser Leu Met Ser Leu Ile
            20                  25                  30


Ile Leu Ala Thr Leu Val Gly Asn Leu Ile Val Ile Ile Ser Ile Ser

        35                  40                  45

His Phe Lys Gln Leu His Thr Pro Thr Asn Trp Leu Leu His Ser Met
    50                  55                  60

Ala Ile Val Asp Phe Leu Leu Gly Cys Leu Ile Met Pro Cys Ser Met
65                  70                  75                  80

Val Arg Thr Val Glu Arg Cys Trp Tyr Phe Gly Glu Ile Leu Cys Lys
                85                  90                  95

Val His Thr Ser Thr Asp Ile Met Leu Ser Ser Ala Ser Ile Phe His
            100                 105                 110

Leu Ala Phe Ile Ser Ile Asp Arg Tyr Cys Ala Val Cys Asp Pro Leu
        115                 120                 125

Arg Tyr Lys Ala Lys Ile Asn Ile Ser Thr Ile Leu Val Met Ile Leu
    130                 135                 140

Val Ser Trp Ser Leu Pro Ala Val Tyr Ala Phe Gly Met Ile Phe Leu
145                 150                 155                 160

Glu Leu Asn Leu Lys Gly Val Glu Glu Leu Tyr Arg Ser Gln Val Ser
                165                 170                 175

Asp Leu Gly Gly Cys Ser Pro Phe Phe Ser Lys Val Ser Gly Val Leu
            180                 185                 190

Ala Phe Met Thr Ser Phe Tyr Ile Pro Gly Ser Val Met Leu Phe Val
        195                 200                 205

Tyr Tyr Arg Ile Tyr Phe Ile Ala Lys Gly Gln Ala Arg Ser Ile Asn
    210                 215                 220

Arg Thr Asn Val Gln Val Gly Leu Glu Gly Lys Ser Gln Ala Pro Gln
225                 230                 235                 240

Ser Lys Glu Thr Lys Ala Ala Lys Thr Leu Gly Ile Met Val Gly Val
                245                 250                 255

Phe Leu Val Cys Trp Cys Pro Phe Phe Leu Cys Thr Val Leu Asp Pro
            260                 265                 270

Phe Leu Gly Tyr Val Ile Pro Pro Ser Leu Asn Asp Ala Leu Tyr Trp
        275                 280                 285

Phe Gly Tyr Leu Asn Ser Ala Leu Asn Pro Met Val Tyr Ala Phe Phe
    290                 295                 300

Tyr Pro Trp Phe Arg Arg Ala Leu Lys Met Val Leu Leu Gly Lys Ile
305                 310                 315                 320

Phe Gln Lys Asp Ser Ser Arg Ser Lys Leu Phe Leu
                325                 330

<210>  4
<211>  1020
<212>  DNA
<213>  Mus musculus

<220>
<221>  mTAAR2
<222>  (1)..(1020)

<400>  4
   atggcatctt ttgaagccca gcaggaaacc tttgattgtt cagagtacgg aaatggatct
   60
   tgcccagaga atgaaagatc tctcggcgtc cgagctgcca tgtactcgtt gatggcctgc
   120
   gccatattca tcacgatctt tggcaacctg gccatgatca tttccatttc ctacttcaaa
   180
   cagcttcaca caccgaccaa cctcctcatt ctctccatgg ccgtcaccga cttccttctc
   240
   ggattcacca tcatgccata tagtatggtc aggtcagtgg aaaactgctg gtatttcgga
   300
   cttacgtttt gcaagatcca ttatagcttc gacctgatgc ttagcataac gtccattttc
   360
   cacctttgct cggtggccgt cgatagattt tatgccatct gtcacccttt gcattattgc
   420
   accaaaatga ctatcccggt cgttaggcgg ctgctgctcg tctgctggtc agtccccggg
   480
   gcgtttgcct ttggggtggt cttctccgag gcttacgctg atggaattga aggctatgac
   540
   attttggttg catgttccag ttcctgccca gtcatgttca acaagctatg ggggaccacc
   600
   ttgtttgtgg caggcttttt cactcctagc tcgatgatgg tggggattta cggcaaaatt
   660
   ttcgctgtat ccaaaaaaca cgctcgcgta attgacaact tgccagaaaa tcaaaacaat
   720
   caaatgagga aggacaaaaa agcagccaaa actttaggga tagtgatggg tgttttctta
   780
   ttgtgttggt ttccatgttt cttcaccatc ctgttagatc cgtttctgaa tttctctacg
   840
   cctgcagttc tgtttgatgc cttaacatgg ttcggttatt ttaattccac atgtaatccc
   900
   ttaatttatg gtttcttcta cccttggttt cgcagagcac tcaagtacat tttactgggt
   960
   aaaattttca gctcgcattt ccataatact aatttgttta ctcaaaaaga aacagaatag
   1020

<210> 5
<211> 339
<212> PRT
<213> Mus musculus

<220>
<221> mTAAR2
<222> (1)..(339)

<400> 5

Met Ala Ser Phe Glu Ala Gln Gln Glu Thr Phe Asp Cys Ser Glu Tyr
1               5                   10                  15

Gly Asn Gly Ser Cys Pro Glu Asn Glu Arg Ser Leu Gly Val Arg Ala
            20                  25                  30

Ala Met Tyr Ser Leu Met Ala Cys Ala Ile Phe Ile Thr Ile Phe Gly
        35                  40                  45

Asn Leu Ala Met Ile Ile Ser Ile Ser Tyr Phe Lys Gln Leu His Thr
    50                  55                  60

Pro Thr Asn Leu Leu Ile Leu Ser Met Ala Val Thr Asp Phe Leu Leu
65                  70                  75                  80

Gly Phe Thr Ile Met Pro Tyr Ser Met Val Arg Ser Val Glu Asn Cys
                85                  90                  95

Trp Tyr Phe Gly Leu Thr Phe Cys Lys Ile His Tyr Ser Phe Asp Leu
            100                 105                 110

Met Leu Ser Ile Thr Ser Ile Phe His Leu Cys Ser Val Ala Val Asp
        115                 120                 125

Arg Phe Tyr Ala Ile Cys His Pro Leu His Tyr Cys Thr Lys Met Thr
    130                 135                 140

Ile Pro Val Val Arg Arg Leu Leu Leu Val Cys Trp Ser Val Pro Gly
145                 150                 155                 160

Ala Phe Ala Phe Gly Val Val Phe Ser Glu Ala Tyr Ala Asp Gly Ile
                165                 170                 175

Glu Gly Tyr Asp Ile Leu Val Ala Cys Ser Ser Ser Cys Pro Val Met
            180                 185                 190

Phe Asn Lys Leu Trp Gly Thr Thr Leu Phe Val Ala Gly Phe Phe Thr
        195                 200                 205

Pro Ser Ser Met Met Val Gly Ile Tyr Gly Lys Ile Phe Ala Val Ser
    210                 215                 220

Lys Lys His Ala Arg Val Ile Asp Asn Leu Pro Glu Asn Gln Asn Asn
225                 230                 235                 240

Gln Met Arg Lys Asp Lys Lys Ala Ala Lys Thr Leu Gly Ile Val Met
                245                 250                 255

Gly Val Phe Leu Leu Cys Trp Phe Pro Cys Phe Phe Thr Ile Leu Leu
            260                 265                 270

Asp Pro Phe Leu Asn Phe Ser Thr Pro Ala Val Leu Phe Asp Ala Leu
        275                 280                 285

Thr Trp Phe Gly Tyr Phe Asn Ser Thr Cys Asn Pro Leu Ile Tyr Gly
    290                 295                 300

Phe Phe Tyr Pro Trp Phe Arg Arg Ala Leu Lys Tyr Ile Leu Leu Gly
305                 310                 315                 320

Lys Ile Phe Ser Ser His Phe His Asn Thr Asn Leu Phe Thr Gln Lys
                325                 330                 335

Glu Thr Glu

<210>  6
<211>  1032
<212>  DNA
<213>  Mus musculus

<220>
<221>  mTAAR3
<222>  (1)..(1032)

<400>  6
atggatctaa tatacattcc cgaagactta tccagctgtc caaaatttgg aaataaatcc
60

tgccctccca ccaatcgctc tttccgtgtc cgcatgataa tgtacctgtt tatgactgga
120

gccatggtta tcaccatctt tggaaactta gtgataatca tttccatatc gcatttcaaa
180

cagctacatt ctcccaccaa ctttctgatc ctctccatgg caaccacgga cttcctgctg
240

ggttttgtca tcatgcctta cagtatggtg agatcagtgg agagctgctg gtatttcgga
300

gacagctttt gcaaattcca cgcgagcttc gacatgatgc taagcctgac gtccatattc
360

cacctgtgct ccatcgcgat cgaccggttt tatgcagtgt gtgacccttt gcactacacc
420

accaccatga cggtatccat gatcaagaga ctgctggctt tttgctgggc agccccagct
480

ctcttttcgt ttggtttagt tctgtcagag gccaatgttt ctggtatgca gagctacgag
540

attcttgttg cttgcttcaa tttctgtgcg cttacgttca acaagttttg gggaccata
600

ctgttcacta cctgcttctt cactcctggc tccatcatgg ttggtattta tggtaaaatt
660

tttattgttt ctagacgaca tgctcgagcc ctcagcgata tgcctgcgaa cacaaaagga
720

gcagtaggga aaaacctgtc taagaaaaag gacaggaaag cagctaagac cctgggcatc
780

gtcatggggg tgtttctagc gtgctggctg ccttgcttcc tggctgtttt gattgatcca
840

tatttagact actccacgcc catcatagtg ctggatcttc tggtatggct cgggtacttc
900

aactctactt gcaacccccct catccatggc tttttttacc cgtggtttcg gaaagctctt
960

cagtttatag tgtcaggaaa aatatttcgc tctaattcag acactgcaaa tctttttcct
1020

gaagcacatt aa
1032

<210> 7
<211> 343
<212> PRT
<213> Mus musculus

<220>
<221> mTAAR3
<222> (1)..(343)

<400> 7

Met Asp Leu Ile Tyr Ile Pro Glu Asp Leu Ser Ser Cys Pro Lys Phe
1               5                   10                  15

Gly Asn Lys Ser Cys Pro Pro Thr Asn Arg Ser Phe Arg Val Arg Met
            20                  25                  30

Ile Met Tyr Leu Phe Met Thr Gly Ala Met Val Ile Thr Ile Phe Gly
        35                  40                  45

Asn Leu Val Ile Ile Ile Ser Ile Ser His Phe Lys Gln Leu His Ser
    50                  55                  60

Pro Thr Asn Phe Leu Ile Leu Ser Met Ala Thr Thr Asp Phe Leu Leu
65                  70                  75                  80

Gly Phe Val Ile Met Pro Tyr Ser Met Val Arg Ser Val Glu Ser Cys
                85                  90                  95

Trp Tyr Phe Gly Asp Ser Phe Cys Lys Phe His Ala Ser Phe Asp Met
            100                 105                 110

Met Leu Ser Leu Thr Ser Ile Phe His Leu Cys Ser Ile Ala Ile Asp
        115                 120                 125

Arg Phe Tyr Ala Val Cys Asp Pro Leu His Tyr Thr Thr Thr Met Thr
    130                 135                 140

Val Ser Met Ile Lys Arg Leu Leu Ala Phe Cys Trp Ala Ala Pro Ala
145                 150                 155                 160

Leu Phe Ser Phe Gly Leu Val Leu Ser Glu Ala Asn Val Ser Gly Met
                165                 170                 175

Gln Ser Tyr Glu Ile Leu Val Ala Cys Phe Asn Phe Cys Ala Leu Thr
            180                 185                 190

Phe Asn Lys Phe Trp Gly Thr Ile Leu Phe Thr Thr Cys Phe Phe Thr
        195                 200                 205

Pro Gly Ser Ile Met Val Gly Ile Tyr Gly Lys Ile Phe Ile Val Ser
    210                 215                 220

Arg Arg His Ala Arg Ala Leu Ser Asp Met Pro Ala Asn Thr Lys Gly
225                 230                 235                 240

Ala Val Gly Lys Asn Leu Ser Lys Lys Lys Asp Arg Lys Ala Ala Lys
                245                 250                 255

Thr Leu Gly Ile Val Met Gly Val Phe Leu Ala Cys Trp Leu Pro Cys
            260                 265                 270

Phe Leu Ala Val Leu Ile Asp Pro Tyr Leu Asp Tyr Ser Thr Pro Ile
        275                 280                 285

Ile Val Leu Asp Leu Leu Val Trp Leu Gly Tyr Phe Asn Ser Thr Cys
    290                 295                 300

Asn Pro Leu Ile His Gly Phe Phe Tyr Pro Trp Phe Arg Lys Ala Leu
305                 310                 315                 320

Gln Phe Ile Val Ser Gly Lys Ile Phe Arg Ser Asn Ser Asp Thr Ala
                325                 330                 335

Asn Leu Phe Pro Glu Ala His

            340

<210> 8
<211> 1044
<212> DNA
<213> Mus musculus

<220>
<221> mTAAR4
<222> (1)..(1044)

<400> 8
atgaatacac ccgacccctg gagctcccca gaagtacagt tttgctttgc tgctgcaaac
60

agttcttgcc caaggaaggc gaggcctgcg ctcgttgtct gtgccatgta cctcatcatg
120

attggagcga tagtgatgac catgctggga aacatggctg tgatcatctc tattgcccac
180

ttcaagcagc tccactcccc aaccaacttc cttattctct ccatggctac cacagacttc
240

ctgttgagtt gcgtggtcat gcccttcagt atgatccggt ccatcgagtc atgctggtac
300

ttcggagacc tcttttgcaa agttcacagc tgctgtgaca tcatgctctg taccacatcc
360

attttccacc tctgcttcat ctcagttgac cgccactatg ccgtctgcga ccctttgcac
420

tacgtcaccc aaatcacgac ccgggtggta ggggtctttc tactcatcag ttggtctgtt
480

cccatctttt ttgcctttgg cttggtattc tcagaattaa atctgattgg tgctgaggat
540

tttgtcgcag ccattgactg tacaggtttg tgtgtgttga tatttaacaa gctctgggga
600

gttctggctt ccttcatagc tttctttctg cctgggacag tcatggtagg gatttacata
660

cacattttca cagttgccca gaaacatgcc aggcagattg gtacaggtcc taggacaaaa
720

caggccctct cagaaagcaa aatgaaggcc acatcaaaaa aggaaagcaa ggccaccaag
780

actttaagca ttgtcatggg agtgtttgtg ttgtgttggc tacccttttt tgtcttgaca
840

atcacagacc ctttcattga ttttacaacc cctgaagatt tgtataatgt tttcctttgg
900

cttggttatt ttaattccac tttcaatccc atcatatatg gcatgttcta tccctggttt
960

cgaaaggccc tgaggatgat agtcacagga acgatcttcc gctctgactc ttctacctca
1020

agcctgcatc ctgcacatcc ttag
1044

<210> 9
<211> 347
<212> PRT
<213> Mus musculus

<220>
<221> mTAAR4
<222> (1)..(347)

<400> 9

Met Asn Thr Pro Asp Pro Trp Ser Ser Pro Glu Val Gln Phe Cys Phe
1               5                   10                  15

Ala Ala Ala Asn Ser Ser Cys Pro Arg Lys Ala Arg Pro Ala Leu Val
            20                  25                  30

Val Cys Ala Met Tyr Leu Ile Met Ile Gly Ala Ile Val Met Thr Met
        35                  40                  45

Leu Gly Asn Met Ala Val Ile Ile Ser Ile Ala His Phe Lys Gln Leu
    50                  55                  60

His Ser Pro Thr Asn Phe Leu Ile Leu Ser Met Ala Thr Thr Asp Phe
65                  70                  75                  80

Leu Leu Ser Cys Val Val Met Pro Phe Ser Met Ile Arg Ser Ile Glu
                85                  90                  95

Ser Cys Trp Tyr Phe Gly Asp Leu Phe Cys Lys Val His Ser Cys Cys
            100                 105                 110

Asp Ile Met Leu Cys Thr Thr Ser Ile Phe His Leu Cys Phe Ile Ser
        115                 120                 125

Val Asp Arg His Tyr Ala Val Cys Asp Pro Leu His Tyr Val Thr Gln
    130                 135                 140

Ile Thr Thr Arg Val Val Gly Val Phe Leu Leu Ile Ser Trp Ser Val
145                 150                 155                 160

Pro Ile Phe Phe Ala Phe Gly Leu Val Phe Ser Glu Leu Asn Leu Ile
                165                 170                 175

Gly Ala Glu Asp Phe Val Ala Ala Ile Asp Cys Thr Gly Leu Cys Val
            180                 185                 190

Leu Ile Phe Asn Lys Leu Trp Gly Val Leu Ala Ser Phe Ile Ala Phe
        195                 200                 205

Phe Leu Pro Gly Thr Val Met Val Gly Ile Tyr Ile His Ile Phe Thr

210                 215                 220

Val Ala Gln Lys His Ala Arg Gln Ile Gly Thr Gly Pro Arg Thr Lys
225                 230                 235                 240

Gln Ala Leu Ser Glu Ser Lys Met Lys Ala Thr Ser Lys Lys Glu Ser
                245                 250                 255

Lys Ala Thr Lys Thr Leu Ser Ile Val Met Gly Val Phe Val Leu Cys
            260                 265                 270

Trp Leu Pro Phe Phe Val Leu Thr Ile Thr Asp Pro Phe Ile Asp Phe
        275                 280                 285

Thr Thr Pro Glu Asp Leu Tyr Asn Val Phe Leu Trp Leu Gly Tyr Phe
    290                 295                 300

Asn Ser Thr Phe Asn Pro Ile Ile Tyr Gly Met Phe Tyr Pro Trp Phe
305                 310                 315                 320

Arg Lys Ala Leu Arg Met Ile Val Thr Gly Thr Ile Phe Arg Ser Asp
                325                 330                 335

Ser Ser Thr Ser Ser Leu His Pro Ala His Pro
            340                 345

<210>  10
<211>  1014
<212>  DNA
<213>  Mus musculus

<220>
<221>  mTAAR5
<222>  (1)..(1014)

<400>  10
   atgagagctg tcctcctccc gggctctgga gagcagccta cagcgttctg ctaccaggtg
   60
   aatgggtctt gccccaggac agtccacccg ctggccatcc aggtcgtcat ctatctggcc
   120
   tgcgcagtgg gtgtgctgat cacagtcctg gggaatttgt ttgtggtgtt tgcagtgtcc
   180
   tactttaaag tgctccacac gcccaccaac ttcctgctgc tctccctggc cctggcagac
   240
   atgttgctag gtctgctggt gctgcccctg agcacagtcc gctctgtgga gagctgctgg
   300
   ttctttggag acttcctgtg ccgtctgcat acctatctgg acacgctgtt ctgtctcacc
   360
   tccatcttcc atctctgttt tatttccatt gaccgtcatt gtgccatctg tgaccccctg
   420
   ctctatccct ccaagttcac agtcaggaca gccctccgct acatcgtggc agggtggggg
   480
   ataccagcag cctacactgc cttcttcctc tacacagatg tggtagagag agcactcagc
   540
   cagtggctag aagagatgcc ttgtgtgggc agctgccagc tgctgttcaa taagttctgg
   600
   ggttggttaa acttccctgc attttttgtt ccctgcctaa tcatgatcag cttgtacctg
   660
   aagatctttg tggtcgccac caggcaggct cagcagatca gaactctgag ccaaagcttg
   720
   gctggggctg tcaagcggga aagaaaagct gccaagacgc tgggcattgc tgtgggcatc
   780
   taccttgtgt gttggcttcc cttcactgtt gacacactag tggacagcct tcttaacttc
   840
   atcaccccac ccctggtctt tgacatcttt atctggtttg cttatttcaa ttcagcctgt
   900
   aatcccatca tctacgtctt ctcctaccgg tggtttagga aggcactgaa actgctcctg
   960
   agccgggaga tcttctcacc aaggacaccg actgttgatt tataccatga ctga
   1014

<210> 11
<211> 337
<212> PRT
<213> Mus musculus

<220>
<221> mTAAR5
<222> (1)..(337)

<400> 11

Met Arg Ala Val Leu Leu Pro Gly Ser Gly Glu Gln Pro Thr Ala Phe
1               5                   10                  15

Cys Tyr Gln Val Asn Gly Ser Cys Pro Arg Thr Val His Pro Leu Ala
            20                  25                  30

Ile Gln Val Val Ile Tyr Leu Ala Cys Ala Val Gly Val Leu Ile Thr
        35                  40                  45

Val Leu Gly Asn Leu Phe Val Val Phe Ala Val Ser Tyr Phe Lys Val
    50                  55                  60

Leu His Thr Pro Thr Asn Phe Leu Leu Leu Ser Leu Ala Leu Ala Asp
65                  70                  75                  80

Met Leu Leu Gly Leu Leu Val Leu Pro Leu Ser Thr Val Arg Ser Val
                85                  90                  95

Glu Ser Cys Trp Phe Phe Gly Asp Phe Leu Cys Arg Leu His Thr Tyr
            100                 105                 110

Leu Asp Thr Leu Phe Cys Leu Thr Ser Ile Phe His Leu Cys Phe Ile
        115                 120                 125

Ser Ile Asp Arg His Cys Ala Ile Cys Asp Pro Leu Leu Tyr Pro Ser
    130                 135                 140

Lys Phe Thr Val Arg Thr Ala Leu Arg Tyr Ile Val Ala Gly Trp Gly
145                 150                 155                 160

Ile Pro Ala Ala Tyr Thr Ala Phe Phe Leu Tyr Thr Asp Val Val Glu
                165                 170                 175

Arg Ala Leu Ser Gln Trp Leu Glu Glu Met Pro Cys Val Gly Ser Cys
            180                 185                 190

Gln Leu Leu Phe Asn Lys Phe Trp Gly Trp Leu Asn Phe Pro Ala Phe
        195                 200                 205

Phe Val Pro Cys Leu Ile Met Ile Ser Leu Tyr Leu Lys Ile Phe Val
    210                 215                 220

Val Ala Thr Arg Gln Ala Gln Gln Ile Arg Thr Leu Ser Gln Ser Leu
225                 230                 235                 240

Ala Gly Ala Val Lys Arg Glu Arg Lys Ala Ala Lys Thr Leu Gly Ile
                245                 250                 255

Ala Val Gly Ile Tyr Leu Val Cys Trp Leu Pro Phe Thr Val Asp Thr
            260                 265                 270

Leu Val Asp Ser Leu Leu Asn Phe Ile Thr Pro Pro Leu Val Phe Asp
        275                 280                 285

Ile Phe Ile Trp Phe Ala Tyr Phe Asn Ser Ala Cys Asn Pro Ile Ile
    290                 295                 300

Tyr Val Phe Ser Tyr Arg Trp Phe Arg Lys Ala Leu Lys Leu Leu Leu
305                 310                 315                 320

Ser Arg Glu Ile Phe Ser Pro Arg Thr Pro Thr Val Asp Leu Tyr His
                325                 330                 335

Asp

<210>  12
<211>  1038
<212>  DNA

<213>  Mus musculus

<220>
<221>  mTAAR6
<222>  (1)..(1038)

<400>  12
atgggcagta actcgtctcc gcccaccgtc ctgcagctct gctatgagaa tgtgaccggg
60

tcttgtgtga aaacccccta ctctcctggg tcgagggtca tcctctatgc agtctttggc
120

tttggggctg tgctggcagt gtttggaaac ctcatggtga tgatatccat cctccatttc
180

aagcagctgc actctcccac caatttcctc attgcatcct tggcctgtgc tgactttggt
240

gtgggcatct ctgtgatgcc cttcagcatg gtcaggtcca tcgagagctg ctggtacttt
300

ggtagaagtt tctgcacttt ccacacctgc tgtgatgtgg cgttctgtta ctcttctctc
360

ttccacctga gcttcatctc catcgacagg tacatcgctg tcacggaccc tctggtctat
420

cccaccaagt tcacggtgtc tgtgtctgga atttgcatcg gcgtctcctg gattctgccc
480

ctggtgtaca gcggcgctgt gttctacaca ggggtttatg atgacggcct ggaggaatta
540

tccagtgccc tcaactgcgt agggggctgt caggtagttg tgaatcagaa ctgggtgctg
600

atagattttc tttccttctt aatacctaca cttgttatga taattctgta tggtaatatt
660

tttctggtag ctagacaaca ggctaaaaag atagaaaata ttgggagcaa aacagaatca
720

tcctctgaga gctacaaagc cagagtggcg agaagagaaa gaaaagcagc caaaaccctg
780

gggattaccg tggtagcatt catgatatca tggttgccct acagcattga ttcattagtt
840

gatgctttta tgggcttcat cacccctgcc tacatttatg aaatctgtgt ttggtgtgct
900

tattacaact cagccatgaa ccctctgatc tacgctttat tttatccatg gtttaagaaa
960

gctattaagg ttattatgag tggccaggtg ttcaagaaca gctcagccac tatgaacttg
1020

ttctctgagc aaatataa
1038


<210>  13
<211>  345
<212>  PRT
<213>  Mus musculus

<220>
<221>  mTAAR6
<222>  (1)..(345)

<400>  13

Met Gly Ser Asn Ser Ser Pro Pro Thr Val Leu Gln Leu Cys Tyr Glu
1               5                   10                  15

Asn Val Thr Gly Ser Cys Val Lys Thr Pro Tyr Ser Pro Gly Ser Arg
            20                  25                  30

Val Ile Leu Tyr Ala Val Phe Gly Phe Gly Ala Val Leu Ala Val Phe
        35                  40                  45

Gly Asn Leu Met Val Met Ile Ser Ile Leu His Phe Lys Gln Leu His
    50                  55                  60

Ser Pro Thr Asn Phe Leu Ile Ala Ser Leu Ala Cys Ala Asp Phe Gly
65                  70                  75                  80

Val Gly Ile Ser Val Met Pro Phe Ser Met Val Arg Ser Ile Glu Ser
                85                  90                  95

Cys Trp Tyr Phe Gly Arg Ser Phe Cys Thr Phe His Thr Cys Cys Asp
            100                 105                 110

Val Ala Phe Cys Tyr Ser Ser Leu Phe His Leu Ser Phe Ile Ser Ile
        115                 120                 125

Asp Arg Tyr Ile Ala Val Thr Asp Pro Leu Val Tyr Pro Thr Lys Phe
    130                 135                 140

Thr Val Ser Val Ser Gly Ile Cys Ile Gly Val Ser Trp Ile Leu Pro
145                 150                 155                 160

Leu Val Tyr Ser Gly Ala Val Phe Tyr Thr Gly Val Tyr Asp Asp Gly
                165                 170                 175

Leu Glu Glu Leu Ser Ser Ala Leu Asn Cys Val Gly Gly Cys Gln Val
            180                 185                 190

Val Val Asn Gln Asn Trp Val Leu Ile Asp Phe Leu Ser Phe Leu Ile
        195                 200                 205

Pro Thr Leu Val Met Ile Ile Leu Tyr Gly Asn Ile Phe Leu Val Ala
    210                 215                 220

Arg Gln Gln Ala Lys Lys Ile Glu Asn Ile Gly Ser Lys Thr Glu Ser
225                 230                 235                 240

Ser Ser Glu Ser Tyr Lys Ala Arg Val Ala Arg Arg Glu Arg Lys Ala
                245                 250                 255

Ala Lys Thr Leu Gly Ile Thr Val Val Ala Phe Met Ile Ser Trp Leu
            260                 265                 270

Pro Tyr Ser Ile Asp Ser Leu Val Asp Ala Phe Met Gly Phe Ile Thr
        275                 280                 285

Pro Ala Tyr Ile Tyr Glu Ile Cys Val Trp Cys Ala Tyr Tyr Asn Ser
    290                 295                 300

Ala Met Asn Pro Leu Ile Tyr Ala Leu Phe Tyr Pro Trp Phe Lys Lys
305                 310                 315                 320

Ala Ile Lys Val Ile Met Ser Gly Gln Val Phe Lys Asn Ser Ser Ala
                325                 330                 335

Thr Met Asn Leu Phe Ser Glu Gln Ile
            340                 345

<210>  14
<211>  1077
<212>  DNA
<213>  Mus musculus

<220>
<221>  mTAAR7a
<222>  (1)..(1077)

<400>  14
atggacaaat tggttgacca ttttctcagt gatcaatcta gaaccatgaa tgaagatctg
60

ttctctgcca catctacaga gctgtgctat gagaacctga acaggtcctg tgtcaggagc
120

ccatactccc caggccctcg cctcatcctc tatgcagtct ttggctttgg ggctgcgctg
180

gctgtgtgtg gaaacctcct ggtgatgaca tcaattcttc atttcaggca gctgcactct
240

cctgccaact tcctggtggc atccctggcc tgtgctgatt tcttagtggg tctgactgtg
300

atgcccttca gcacggtgag gtctgtggag ggctgctggt actttggaga gagttattgt
360

aaattccatt cttgttttga agggtcattc tgttactctt ctatcttcca cttgtgcttc
420

atctctgttg atagatatat tgcagtcagt gaccccctga cctaccccac caggttcact
480

gcttctgttt ctggcaagtg catcaccttc tcctggctcc tgtccatcat ctacagcttt
540

tccctccttt acacaggagc caacgaagct gggctggagg atctagtgag tgtcctcacc
600

tgtgtgggtg gctgtcaaat tgcagtgaat caaagctggg tctttatcaa ctttctatta
660

tttcttatcc ccactcttgt gatgatgact gtctactcta agattttcct cattgctaaa
720

cagcaggctc agaacattga gaagatgagc aagcagactg ccagggcatc agaaagctac
780

aaggacaggg tggccaagag ggagaggaaa gcagccaaaa ccctgggcat tgcagtggct
840

gccttcctcc tttcatggct gccatacttc attgactcca tcattgatgc cttcctaggg
900

ttcatcacgc ccacgtatgt gtatgaaatc ctagtttgga tcgcttacta taactcagcc
960

atgaaccctt tgatttatgc tttcttttat ccttggtttc gaaaagccat caagctcatt
1020

gtcactggca aaatcttgag ggagaattcc tcaaccacca acttgtttcc tgagtag
1077


<210> 15
<211> 358
<212> PRT
<213> Mus musculus


<220>
<221> mTAAR7a
<222> (1)..(358)

<400> 15

Met Asp Lys Leu Val Asp His Phe Leu Ser Asp Gln Ser Arg Thr Met
1               5                   10                  15


Asn Glu Asp Leu Phe Ser Ala Thr Ser Thr Glu Leu Cys Tyr Glu Asn
            20                  25                  30


Leu Asn Arg Ser Cys Val Arg Ser Pro Tyr Ser Pro Gly Pro Arg Leu
        35                  40                  45


Ile Leu Tyr Ala Val Phe Gly Phe Gly Ala Ala Leu Ala Val Cys Gly
    50                  55                  60


Asn Leu Leu Val Met Thr Ser Ile Leu His Phe Arg Gln Leu His Ser
65                  70                  75                  80


Pro Ala Asn Phe Leu Val Ala Ser Leu Ala Cys Ala Asp Phe Leu Val
                85                  90                  95


Gly Leu Thr Val Met Pro Phe Ser Thr Val Arg Ser Val Glu Gly Cys
            100                 105                 110

Trp Tyr Phe Gly Glu Ser Tyr Cys Lys Phe His Ser Cys Phe Glu Gly
        115                 120                 125

Ser Phe Cys Tyr Ser Ser Ile Phe His Leu Cys Phe Ile Ser Val Asp
    130                 135                 140

Arg Tyr Ile Ala Val Ser Asp Pro Leu Thr Tyr Pro Thr Arg Phe Thr
145                 150                 155                 160

Ala Ser Val Ser Gly Lys Cys Ile Thr Phe Ser Trp Leu Leu Ser Ile
                165                 170                 175

Ile Tyr Ser Phe Ser Leu Leu Tyr Thr Gly Ala Asn Glu Ala Gly Leu
            180                 185                 190

Glu Asp Leu Val Ser Val Leu Thr Cys Val Gly Gly Cys Gln Ile Ala
        195                 200                 205

Val Asn Gln Ser Trp Val Phe Ile Asn Phe Leu Leu Phe Leu Ile Pro
    210                 215                 220

Thr Leu Val Met Met Thr Val Tyr Ser Lys Ile Phe Leu Ile Ala Lys
225                 230                 235                 240

Gln Gln Ala Gln Asn Ile Glu Lys Met Ser Lys Gln Thr Ala Arg Ala
                245                 250                 255

Ser Glu Ser Tyr Lys Asp Arg Val Ala Lys Arg Glu Arg Lys Ala Ala
            260                 265                 270

Lys Thr Leu Gly Ile Ala Val Ala Ala Phe Leu Leu Ser Trp Leu Pro
        275                 280                 285

Tyr Phe Ile Asp Ser Ile Ile Asp Ala Phe Leu Gly Phe Ile Thr Pro
    290                 295                 300

Thr Tyr Val Tyr Glu Ile Leu Val Trp Ile Ala Tyr Tyr Asn Ser Ala
305                 310                 315                 320

Met Asn Pro Leu Ile Tyr Ala Phe Phe Tyr Pro Trp Phe Arg Lys Ala
                325                 330                 335

Ile Lys Leu Ile Val Thr Gly Lys Ile Leu Arg Glu Asn Ser Ser Thr
            340                 345                 350

Thr Asn Leu Phe Pro Glu
        355

<210>  16
<211>  1077

<212>  DNA
<213>  Mus musculus


<220>
<221>  mTAAR7b
<222>  (1)..(1077)

<400>  16
atggctacag ataatgacag ttttccctgg gatcaagaca gcatcctgag cagtgatatg
60

ttctctgcca catctacaga gctgtgctat gagaacctga acaggtcctg tgtcaggagc
120

ccatattccc caggccctcg cctcatcctc tatgcagtct ttggctttgg ggctgcgctg
180

gctgtgtgtg gaaacctcct ggtgatgaca tcaattcttc atttcaggca gctacactcc
240

cctgccaact tcctggtggt gtccttggcc tgtgctgatt tcttggtggg attgactgtg
300

atgcccttca gcacagtgag gtctgtggag ggatgctggt actttgggga gagttactgt
360

aaattacaca cttgttttga tgtatctttc tgctattgtt ctatcttcca cttgtgcttc
420

atctctgttg atagatatat tgcagtcagt gaccccctga cctaccccac caggtttact
480

gcatttgttt ctggcaagtg catcaccttc tcctggctcc tgtccaccat ttatgggttt
540

tctctccttt atacaggggc taatgaagct gggctggagg atctagtgag tgccctcacc
600

tgtgtgggtg gctgtcaact tgcagtgaat caaagctggg tcttcatcaa tttcctatta
660

tttcttatcc ccacccttgt gatgataact gtctactcta agattttcct cattgctaaa
720

cagcaggctc agaacattga gaagatgagc aagcagactg ccagggcatc agacagctac
780

aaggacaggg tggccaagag agagaggaaa gcagccaaaa ccctgggcat cgcagtggct
840

gccttcctcc tttcatggct gccatacttc attgactcca tcattgatgc cttcctaggg
900

ttcatcacgc ccacgtatgt gtatgaaatc ctagtttgga tcgcttacta taactcagcc
960

atgaaccctt tgatttatgc tttcttttat ccttggtttc gaaaagccat caaactcatt
1020

gtctctggca aagtcttgag ggagaattcc tcaaccacca acttgtttcc tgagtag
1077


<210>  17
<211>  358
<212>  PRT

<213>  Mus musculus

<220>
<221>  mTAAR7b
<222>  (1)..(358)

<400>  17

Met Ala Thr Asp Asn Asp Ser Phe Pro Trp Asp Gln Asp Ser Ile Leu
1               5                   10                  15

Ser Ser Asp Met Phe Ser Ala Thr Ser Thr Glu Leu Cys Tyr Glu Asn
            20                  25                  30

Leu Asn Arg Ser Cys Val Arg Ser Pro Tyr Ser Pro Gly Pro Arg Leu
        35                  40                  45

Ile Leu Tyr Ala Val Phe Gly Phe Gly Ala Ala Leu Ala Val Cys Gly
    50                  55                  60

Asn Leu Leu Val Met Thr Ser Ile Leu His Phe Arg Gln Leu His Ser
65                  70                  75                  80

Pro Ala Asn Phe Leu Val Val Ser Leu Ala Cys Ala Asp Phe Leu Val
                85                  90                  95

Gly Leu Thr Val Met Pro Phe Ser Thr Val Arg Ser Val Glu Gly Cys
            100                 105                 110

Trp Tyr Phe Gly Glu Ser Tyr Cys Lys Leu His Thr Cys Phe Asp Val
        115                 120                 125

Ser Phe Cys Tyr Cys Ser Ile Phe His Leu Cys Phe Ile Ser Val Asp
    130                 135                 140

Arg Tyr Ile Ala Val Ser Asp Pro Leu Thr Tyr Pro Thr Arg Phe Thr
145                 150                 155                 160

Ala Phe Val Ser Gly Lys Cys Ile Thr Phe Ser Trp Leu Leu Ser Thr
                165                 170                 175

Ile Tyr Gly Phe Ser Leu Leu Tyr Thr Gly Ala Asn Glu Ala Gly Leu
            180                 185                 190

Glu Asp Leu Val Ser Ala Leu Thr Cys Val Gly Gly Cys Gln Leu Ala
        195                 200                 205

Val Asn Gln Ser Trp Val Phe Ile Asn Phe Leu Leu Phe Leu Ile Pro
    210                 215                 220

Thr Leu Val Met Ile Thr Val Tyr Ser Lys Ile Phe Leu Ile Ala Lys
225                 230                 235                 240

Gln Gln Ala Gln Asn Ile Glu Lys Met Ser Lys Gln Thr Ala Arg Ala
                245                 250                 255

Ser Asp Ser Tyr Lys Asp Arg Val Ala Lys Arg Glu Arg Lys Ala Ala
            260                 265                 270

Lys Thr Leu Gly Ile Ala Val Ala Ala Phe Leu Leu Ser Trp Leu Pro
        275                 280                 285

Tyr Phe Ile Asp Ser Ile Ile Asp Ala Phe Leu Gly Phe Ile Thr Pro
    290                 295                 300

Thr Tyr Val Tyr Glu Ile Leu Val Trp Ile Ala Tyr Tyr Asn Ser Ala
305                 310                 315                 320

Met Asn Pro Leu Ile Tyr Ala Phe Phe Tyr Pro Trp Phe Arg Lys Ala
                325                 330                 335

Ile Lys Leu Ile Val Ser Gly Lys Val Leu Arg Glu Asn Ser Ser Thr
            340                 345                 350

Thr Asn Leu Phe Pro Glu
        355

<210>  18
<211>  1055
<212>  DNA
<213>  Mus musculus

<220>
<221>  mTAAR7c pseudogene
<222>  (1)..(1055)

<400>  18
atgactacag gtgatgacag ttttccttgg aaccaagaca gcatcctgag cagagatctg
60

ttctctgcca catctgcaga gctgtgctat gagaacctga acaggtcctg tgtcaggagc
120

ccatactcct caggccctcg cctcatcctc tatgcagtct ttggctttgg ggctgtgctg
180

gctgtgtgtg gaaacctcct ggtgatgaca tcaattcttc atttcaggca gctgcactct
240

cctgccaact tcctggtggc atccctggcc tgtgctgact tcttggtagg ggtgatggtg
300

atgcccttta gcacagtgag gtctgtggag ggctgctggt actttgggga gatttactgt
360

aaattccatt cttgttttga agggtcattc tgttactctt ctatcttcca cttgtgtttc
420

atctctattg atagatatat tgcagtcagt gaccccctga tctaccccac caggtttact
480

gcatctgttt ctggcaagtg catcaccttc tcctgtccac catctacagc ttttccctcc
540

tttatacagg ggccaatgaa gctgggctga aggatctagt gagtgccctc acctgtgtgg
600

gtggctgtca agtcccagtg aatcaaagct gggtcttcat caacttccta ttatttctta
660

tccccaccct tgtgatgata actgtctact ctaagatttt cctcattgct aaacagcagg
720

ctcagaacat tgagaagatg agcaagcaga ctgccagggc atcagacagc tacaaggaca
780

gggtggccaa gagggagagg aaagcagcca aaacgctggg cattgcagtg gctgccttcc
840

tcctttcatg gctgccatac ttcattgact cctttattga tgccttccta gggtatgtgt
900

atgaaatcct agtttggatc gcttactata actcagccat gaaccctttg atttatgctt
960

tcttttatcc ttggtttcga aaagccatca aactcattgt cactggcaaa atcctgagag
1020

tgaattcctc aaccaccaac ctgtttcccg aatag
1055

<210> 19
<211> 1055
<212> DNA
<213> Mus musculus

<220>
<221> mTAAR7c fixed pseudogen
<222> (1)..(1055)

<400> 19
atgactacag gtgatgacag ttttccttgg aaccaagaca gcatcctgag cagagatctg
60

ttctctgcca catctgcaga gctgtgctat gagaacctga acaggtcctg tgtcaggagc
120

ccatactcct caggccctcg cctcatcctc tatgcagtct ttggctttgg ggctgtgctg
180

gctgtgtgtg gaaacctcct ggtgatgaca tcaattcttc atttcaggca gctgcactct
240

cctgccaact tcctggtggc atccctggcc tgtgctgact tcttggtagg ggtgatggtg
300

atgcccttta gcacagtgag gtctgtggag ggctgctggt actttgggga gatttactgt
360

aaattccatt cttgttttga agggtcattc tgttactctt ctatcttcca cttgtgtttc
420

atctctattg atagatatat tgcagtcagt gaccccctga tctaccccac caggtttact
480

gcatctgttt ctggcaagtg catcaccttc tcctgtccac catctacagc ttttccctcc
540

tttatacagg ggccaatgaa gctgggctga aggatctagt gagtgccctc acctgtgtgg
600

gtggctgtca agtcccagtg aatcaaagct gggtcttcat caacttccta ttatttctta
660

tccccaccct tgtgatgata actgtctact ctaagatttt cctcattgct aaacagcagg
720

ctcagaacat tgagaagatg agcaagcaga ctgccagggc atcagacagc tacaaggaca
780

gggtggccaa gagggagagg aaagcagcca aaacgctggg cattgcagtg gctgccttcc
840

tcctttcatg gctgccatac ttcattgact cctttattga tgccttccta gggtatgtgt
900

atgaaatcct agtttggatc gcttactata actcagccat gaaccctttg atttatgctt
960

tcttttatcc ttggtttcga aaagccatca aactcattgt cactggcaaa atcctgagag
1020

tgaattcctc aaccaccaac ctgtttcccg aatag
1055


<210>  20
<211>  351
<212>  PRT
<213>  Mus musculus


<220>
<221>  mTAAR7c fixed
<222>  (1)..(351)

<400>  20

Met Thr Thr Gly Asp Asp Ser Phe Pro Trp Asn Gln Asp Ser Ile Leu
1               5                   10                  15


Ser Arg Asp Leu Phe Ser Ala Thr Ser Ala Glu Leu Cys Tyr Glu Asn
            20                  25                  30


Leu Asn Arg Ser Cys Val Arg Ser Pro Tyr Ser Ser Gly Pro Arg Leu
        35                  40                  45


Ile Leu Tyr Ala Val Phe Gly Phe Gly Ala Val Leu Ala Val Cys Gly
    50                  55                  60


Asn Leu Leu Val Met Thr Ser Ile Leu His Phe Arg Gln Leu His Ser
65                  70                  75                  80


Pro Ala Asn Phe Leu Val Ala Ser Leu Ala Cys Ala Asp Phe Leu Val

                85                  90                  95

Gly Val Met Val Met Pro Phe Ser Thr Val Arg Ser Val Glu Gly Cys
            100                 105                 110

Trp Tyr Phe Gly Glu Ile Tyr Cys Lys Phe His Ser Cys Phe Glu Gly
        115                 120                 125

Ser Phe Cys Tyr Ser Ser Ile Phe His Leu Cys Phe Ile Ser Ile Asp
    130                 135                 140

Arg Tyr Ile Ala Val Ser Asp Pro Leu Ile Tyr Pro Thr Arg Phe Thr
145                 150                 155                 160

Ala Ser Val Ser Gly Lys Cys Ile Thr Phe Ser Leu Ser Thr Ile Tyr
                165                 170                 175

Ser Phe Ser Leu Leu Tyr Thr Gly Ala Asn Glu Ala Gly Leu Lys Asp
            180                 185                 190

Leu Val Ser Ala Leu Thr Cys Val Gly Gly Cys Gln Val Pro Val Asn
        195                 200                 205

Gln Ser Trp Val Phe Ile Asn Phe Leu Leu Phe Leu Ile Pro Thr Leu
    210                 215                 220

Val Met Ile Thr Val Tyr Ser Lys Ile Phe Leu Ile Ala Lys Gln Gln
225                 230                 235                 240

Ala Gln Asn Ile Glu Lys Met Ser Lys Gln Thr Ala Arg Ala Ser Asp
                245                 250                 255

Ser Tyr Lys Asp Arg Val Ala Lys Arg Glu Arg Lys Ala Ala Lys Thr
            260                 265                 270

Leu Gly Ile Ala Val Ala Ala Phe Leu Leu Ser Trp Leu Pro Tyr Phe
        275                 280                 285

Ile Asp Ser Phe Ile Asp Ala Phe Leu Gly Tyr Val Tyr Glu Ile Leu
    290                 295                 300

Val Trp Ile Ala Tyr Tyr Asn Ser Ala Met Asn Pro Leu Ile Tyr Ala
305                 310                 315                 320

Phe Phe Tyr Pro Trp Phe Arg Lys Ala Ile Lys Leu Ile Val Thr Gly
                325                 330                 335

Lys Ile Leu Arg Val Asn Ser Ser Thr Thr Asn Leu Phe Pro Glu
            340                 345                 350

<210> 21
<211> 1055
<212> DNA
<213> Mus musculus


<220>
<221> mTAAR7d
<222> (1)..(1055)

<400> 21
   atgactacag gtgatgacag ttttccttgg aaccaagaca gcatcctgag cagagatctg
   60
   ttctctgcca catctgcaga gctgtgctat gagaacctga acaggtcctg tgtcaggagc
   120
   ccatactcct caggccctcg cctcatcctc tatgcagtct ttggctttgg ggctgtgctg
   180
   gctgtgtgtg gaaacctcct ggtgatgaca tcaattcttc atttcaggca gctgcactct
   240
   cctgccaact tcctggtggc atccctggcc tgtgctgact tcttggtagg ggtgatggtg
   300
   atgcccttta gcacagtgag gtctgtggag ggctgctggt actttgggga gatttactgt
   360
   aaattccatt cttgttttga agggtcattc tgttactctt ctatcttcca cttgtgtttc
   420
   atctctattg atagatatat tgcagtcagt gaccccctga tctaccccac caggtttact
   480
   gcatctgttt ctggcaagtg catcaccttc tcctgtccac catctacagc ttttccctcc
   540
   tttatacagg ggccaatgaa gctgggctga aggatctagt gagtgccctc acctgtgtgg
   600
   gtggctgtca agtcccagtg aatcaaagct gggtcttcat caacttccta ttatttctta
   660
   tccccaccct tgtgatgata actgtctact ctaagatttt cctcattgct aaacagcagg
   720
   ctcagaacat tgagaagatg agcaagcaga ctgccagggc atcagacagc tacaaggaca
   780
   gggtggccaa gagggagagg aaagcagcca aaacgctggg cattgcagtg gctgccttcc
   840
   tcctttcatg gctgccatac ttcattgact cctttattga tgccttccta gggtatgtgt
   900
   atgaaatcct agtttggatc gcttactata actcagccat gaaccctttg atttatgctt
   960
   tcttttatcc ttggtttcga aaagccatca aactcattgt cactggcaaa atcctgagag
   1020
   tgaattcctc aaccaccaac ctgtttcccg aatag
   1055


<210> 22

<211>  358
<212>  PRT
<213>  Mus musculus

<220>
<221>  mTAAR7d
<222>  (1)..(358)

<400>  22

Met Ala Thr Gly Asp Asp Ser Phe Pro Trp Asp Gln Asp Ser Ile Leu
1               5                   10                  15

Ser Arg Asp Leu Phe Ser Ala Thr Ser Thr Glu Leu Cys Tyr Glu Asn
            20                  25                  30

Leu Asn Arg Ser Cys Val Arg Ser Pro Tyr Ser Pro Gly Pro Arg Leu
        35                  40                  45

Ile Leu Tyr Ala Val Phe Gly Phe Gly Ala Val Leu Ala Val Cys Gly
    50                  55                  60

Asn Leu Leu Val Met Thr Ser Ile Leu His Phe Arg Gln Leu His Ser
65                  70                  75                  80

Pro Ala Asn Phe Leu Val Ala Ser Leu Ala Cys Ala Asp Phe Leu Val
                85                  90                  95

Gly Val Met Val Met Pro Phe Ser Met Val Arg Ser Val Glu Gly Cys
            100                 105                 110

Trp Tyr Phe Gly Glu Ser Tyr Cys Lys Phe His Ser Cys Phe Glu Gly
        115                 120                 125

Ser Phe Cys Tyr Ser Ser Leu Phe His Leu Cys Phe Ile Ser Val Asp
    130                 135                 140

Arg Tyr Ile Ala Val Ser Asp Pro Leu Thr Tyr Pro Thr Arg Phe Thr
145                 150                 155                 160

Ala Ser Val Ser Gly Lys Cys Ile Thr Phe Ser Trp Leu Leu Ser Ile
                165                 170                 175

Ile Tyr Ser Phe Ser Leu Leu Tyr Thr Gly Ala Asn Asp Ala Gly Leu
            180                 185                 190

Glu Asp Leu Val Ser Ala Leu Thr Cys Val Gly Gly Cys Gln Ile Ala
        195                 200                 205

Val Asn Gln Thr Trp Val Phe Ile Asn Phe Leu Leu Phe Leu Ile Pro
    210                 215                 220

Thr Leu Val Met Ile Thr Val Tyr Ser Lys Ile Phe Leu Ile Ala Lys
225                 230                 235                 240

Gln Gln Ala Gln Asn Ile Glu Lys Met Ser Lys Gln Thr Ala Arg Ala
                245                 250                 255

Ser Glu Ser Tyr Lys Asp Arg Val Thr Lys Arg Glu Arg Lys Ala Ala
            260                 265                 270

Lys Thr Leu Gly Ile Ala Val Ala Ala Phe Leu Leu Ser Trp Leu Pro
        275                 280                 285

Tyr Phe Ile Asp Ser Ile Ile Asp Ala Phe Leu Gly Phe Ile Thr Pro
    290                 295                 300

Thr Tyr Val Tyr Glu Ile Leu Val Trp Ile Val Tyr Tyr Asn Ser Ala
305                 310                 315                 320

Met Asn Pro Leu Ile Tyr Ala Phe Phe Tyr Ser Trp Phe Arg Lys Ala
                325                 330                 335

Ile Lys Leu Ile Val Ser Gly Lys Ile Leu Arg Glu Asn Ser Ser Thr
            340                 345                 350

Thr Asn Leu Phe Pro Glu
        355

<210>  23
<211>  1077
<212>  DNA
<213>  Mus musculus

<220>
<221>  mTAAR7e
<222>  (1)..(1077)

<400>  23
atggctacag gtgatgacag ttttctctgg gaccaagaca gcatcctgag cagagatctg
60

ttctctgcca catctgcaga gctgtgctat gagaacctga acaggtcctg tgtcagaagc
120

ccatactccc caggccctcg cctcatcctc tatgcagtct ttggctttgg ggctgtgctg
180

gctgtgtgtg gaaacctcct ggtgatgaca tcaattcttc atttcaggca gctgcactct
240

cctgccaact tcctggtggc atccctggcc tgtgctgact tcttggtggg tctgactgtg
300

atgcccttca gcacagtgag gtctgtggag ggctgctggt actttgggga gatttactgt
360

aaattacaca cttgttttga tgtatctttc tgtagttctt ctattttcca cttgtgcttc
420

atctctgttg atagatatat tgcagtcagt gaccccctga tctaccccac caggtttact
480

gcatctgttt ctaacaagtg catcactttc tcctggcttc tgtccatcag ctatgggttt
540

tcccttattt acacaggagc aagtgaagct gggctagagg atctagtgag tgccctcacc
600

tgtgtgggtg gctgtcaact tgcagtgaat caaagctggg tctttatcaa ctttctatta
660

tttcttatcc ccacccttgt gatgataact gtctactcta agattttcct cattgctaaa
720

cagcaggctc agaacattga gaagatgagc aagcagactg ccagggcatc agacagctac
780

aaggacaggg tggccaagag ggagaggaaa gcagccaaaa ccctgggcat cgcagtggct
840

gccttcctcc tttcatggct gccatacttc attgactcct tcattgatgc cttcctgggg
900

ttcatcacgc ccacgtatgt gtatgaaatc ctagtttgga tcgcttacta taactcagcc
960

atgaaccctt tgatttatgc tttcttttat ccttggtttc gaaaagccat caaactcact
1020

gttactggca aaatcttgag ggaaaattcc tcaaccacca acctgtttcc tgagtag
1077

<210> 24
<211> 358
<212> PRT
<213> Mus musculus

<220>
<221> mTAAR7e
<222> (1)..(358)

<400> 24

Met Ala Thr Gly Asp Asp Ser Phe Leu Trp Asp Gln Asp Ser Ile Leu
1               5                   10                  15

Ser Arg Asp Leu Phe Ser Ala Thr Ser Ala Glu Leu Cys Tyr Glu Asn
            20                  25                  30

Leu Asn Arg Ser Cys Val Arg Ser Pro Tyr Ser Pro Gly Pro Arg Leu
        35                  40                  45

Ile Leu Tyr Ala Val Phe Gly Phe Gly Ala Val Leu Ala Val Cys Gly
    50                  55                  60

Asn Leu Leu Val Met Thr Ser Ile Leu His Phe Arg Gln Leu His Ser
65                  70                  75                  80

Pro Ala Asn Phe Leu Val Ala Ser Leu Ala Cys Ala Asp Phe Leu Val
                85                  90                  95

Gly Leu Thr Val Met Pro Phe Ser Thr Val Arg Ser Val Glu Gly Cys
            100                 105                 110

Trp Tyr Phe Gly Glu Ile Tyr Cys Lys Leu His Thr Cys Phe Asp Val
        115                 120                 125

Ser Phe Cys Ser Ser Ser Ile Phe His Leu Cys Phe Ile Ser Val Asp
    130                 135                 140

Arg Tyr Ile Ala Val Ser Asp Pro Leu Ile Tyr Pro Thr Arg Phe Thr
145                 150                 155                 160

Ala Ser Val Ser Asn Lys Cys Ile Thr Phe Ser Trp Leu Leu Ser Ile
                165                 170                 175

Ser Tyr Gly Phe Ser Leu Ile Tyr Thr Gly Ala Ser Glu Ala Gly Leu
            180                 185                 190

Glu Asp Leu Val Ser Ala Leu Thr Cys Val Gly Gly Cys Gln Leu Ala
        195                 200                 205

Val Asn Gln Ser Trp Val Phe Ile Asn Phe Leu Leu Phe Leu Ile Pro
    210                 215                 220

Thr Leu Val Met Ile Thr Val Tyr Ser Lys Ile Phe Leu Ile Ala Lys
225                 230                 235                 240

Gln Gln Ala Gln Asn Ile Glu Lys Met Ser Lys Gln Thr Ala Arg Ala
                245                 250                 255

Ser Asp Ser Tyr Lys Asp Arg Val Ala Lys Arg Glu Arg Lys Ala Ala
            260                 265                 270

Lys Thr Leu Gly Ile Ala Val Ala Ala Phe Leu Leu Ser Trp Leu Pro
        275                 280                 285

Tyr Phe Ile Asp Ser Phe Ile Asp Ala Phe Leu Gly Phe Ile Thr Pro
    290                 295                 300

Thr Tyr Val Tyr Glu Ile Leu Val Trp Ile Ala Tyr Tyr Asn Ser Ala
305                 310                 315                 320

Met Asn Pro Leu Ile Tyr Ala Phe Phe Tyr Pro Trp Phe Arg Lys Ala
                325                 330                 335

Ile Lys Leu Thr Val Thr Gly Lys Ile Leu Arg Glu Asn Ser Ser Thr
            340                 345                 350

Thr Asn Leu Phe Pro Glu
        355

<210>  25
<211>  1077
<212>  DNA
<213>  Mus musculus

<220>
<221>  mTAAR7f
<222>  (1)..(1077)

<400>  25
atgtctatag ctgatgaaac tgtttcctgg aaccaagaca gcatcctgag cagagatctg
60

ttctctgcca catctgcaga gctgtgctat gagaacctga acaggtcctg tgtcagaagc
120

ccatactccc caggccctcg cctcatcctc tatgcagtct ttggctttgg ggctgtgctg
180

gctgtgtgtg gaaacctcct ggtgatgaca tcaattcttc atttcaggca gctgcactct
240

cctgccaact tcctggtggc atccctggcc tgtgctgact tcttggtagg ggtgatggtg
300

atgcccttta gcatggtgag gtctgtggag ggctgctggt actttggaga cagttactgt
360

aaattacaca cttgttttga tgtatctttc tgctattgtt ctctcttcca cttgtgcttc
420

atctctgttg atagatatat tgcagtcagt gaccccctgg cctaccccac caggtttact
480

gcatctgttt ctggcaagtg catcaccttc tcctggctcc tgtccatcag ctatgggttt
540

tccctcattt acacaggagc aagtgaagct gggctggagg atctagtgag ttccctcacc
600

tgtgtgggtg gctgtcaaat cgcagtgaat caaacctggg tctttatcaa cttctcagta
660

tttctcattc ccacccttgt gatgataact gtctactcta agattttcct cattgctaaa
720

cagcaggctc agaacattga gaagatgagc aagcagactg ccagggcatc agacagctac
780

aaggacaggg tggccaagag ggagaggaaa gcagccaaaa ccctgggcat cgcagtggct
840

gccttcctcc tttcatggct gccatacttc attgactcct ttattgatgc cttcctaggg
900

ttcatcacgc ccacgtatgt gtatgaaatc ctagtttgga ttgtttacta taactcagcc
960

atgaaccctt tgatttatgc tttcttttat ccttggtttc gaaaagccat caaactcact
1020

gttactggca aaatcttgag ggaaaattcc tcaaccacca atctgttttc tgagtag
1077

<210> 26
<211> 358
<212> PRT
<213> Mus musculus

<220>
<221> mTAAR7f
<222> (1)..(358)

<400> 26

Met Ser Ile Ala Asp Glu Thr Val Ser Trp Asn Gln Asp Ser Ile Leu
1               5                   10                  15

Ser Arg Asp Leu Phe Ser Ala Thr Ser Ala Glu Leu Cys Tyr Glu Asn
            20                  25                  30

Leu Asn Arg Ser Cys Val Arg Ser Pro Tyr Ser Pro Gly Pro Arg Leu
        35                  40                  45

Ile Leu Tyr Ala Val Phe Gly Phe Gly Ala Val Leu Ala Val Cys Gly
    50                  55                  60

Asn Leu Leu Val Met Thr Ser Ile Leu His Phe Arg Gln Leu His Ser
65                  70                  75                  80

Pro Ala Asn Phe Leu Val Ala Ser Leu Ala Cys Ala Asp Phe Leu Val
                85                  90                  95

Gly Val Met Val Met Pro Phe Ser Met Val Arg Ser Val Glu Gly Cys
            100                 105                 110

Trp Tyr Phe Gly Asp Ser Tyr Cys Lys Leu His Thr Cys Phe Asp Val
        115                 120                 125

Ser Phe Cys Tyr Cys Ser Leu Phe His Leu Cys Phe Ile Ser Val Asp
    130                 135                 140

Arg Tyr Ile Ala Val Ser Asp Pro Leu Ala Tyr Pro Thr Arg Phe Thr
145                 150                 155                 160

Ala Ser Val Ser Gly Lys Cys Ile Thr Phe Ser Trp Leu Leu Ser Ile
                165                 170                 175

Ser Tyr Gly Phe Ser Leu Ile Tyr Thr Gly Ala Ser Glu Ala Gly Leu
            180                 185                 190

Glu Asp Leu Val Ser Ser Leu Thr Cys Val Gly Gly Cys Gln Ile Ala
        195                 200                 205

Val Asn Gln Thr Trp Val Phe Ile Asn Phe Ser Val Phe Leu Ile Pro
    210                 215                 220

Thr Leu Val Met Ile Thr Val Tyr Ser Lys Ile Phe Leu Ile Ala Lys
225                 230                 235                 240

Gln Gln Ala Gln Asn Ile Glu Lys Met Ser Lys Gln Thr Ala Arg Ala
                245                 250                 255

Ser Asp Ser Tyr Lys Asp Arg Val Ala Lys Arg Glu Arg Lys Ala Ala
            260                 265                 270

Lys Thr Leu Gly Ile Ala Val Ala Ala Phe Leu Leu Ser Trp Leu Pro
        275                 280                 285

Tyr Phe Ile Asp Ser Phe Ile Asp Ala Phe Leu Gly Phe Ile Thr Pro
    290                 295                 300

Thr Tyr Val Tyr Glu Ile Leu Val Trp Ile Val Tyr Tyr Asn Ser Ala
305                 310                 315                 320

Met Asn Pro Leu Ile Tyr Ala Phe Phe Tyr Pro Trp Phe Arg Lys Ala
                325                 330                 335

Ile Lys Leu Thr Val Thr Gly Lys Ile Leu Arg Glu Asn Ser Ser Thr
            340                 345                 350

Thr Asn Leu Phe Ser Glu
        355

<210>  27
<211>  1035
<212>  DNA
<213>  Mus musculus

<220>
<221>  mTAAR8a
<222>  (1)..(1035)

<400>  27
   atgaccagca acttttccca accagccctg cagctctgct atgagaacac gaatggatcc
   60
   tgtattaaaa ctccctattc tccagggccc cgggtcatcc tgtacatggt ctatggcttt
   120
   ggggctgtgc tggcagtgtg tgggaacctc ctggtggtga tttcagttct ccatttcaag
   180
   cagctgcact ctccagccaa ttttctcatc gcctctctgg ccagtgctga cttcttggtg
   240
   ggcatctctg tgatgccctt cagcatggtc aggtccattg agagctgctg gtactttgga
   300
   gatgcatttt gtagccttca cagttgttgt gatgtggctt tttgttactc ttctgtcctc
   360
   cacctctgct tcatctcagt ggacaggtac atcgctgtca cggaccctct ggtctatccc
   420
   accaagttca cagtgtctgt gtctggaatt tgcatcagca tctcctggat tctgcccctg
   480
   gtgtacagca gtgcagtgtt ttacacaggc atcagtgcta aggggattga aagcttagta
   540
   agtgctctaa attgtgtagg gggctgccaa attgttatca atcaagactt tgttttgata
   600
   agttttcttc tattcttcat acctaccctt gttatgatca ttctttacag caaaatattt
   660
   ttggtagcca aacagcaagc tgtaaaaatt gaaacttctg taagtggcaa cagaggtgaa
   720
   tcatcctcag agagtcacaa agccagagtg gccaagagag agaggaaggc tgcaaaaacc
   780
   ttgggggtca ctgtggtggc ttttatggtc tcgtggctcc catacacaat tgacgcattg
   840
   gttgatgctt tcatgggctt catcactcct gcctatgtct atgaaatctg ttgctggggt
   900
   acctattata actcagccat gaaccctttg atttatgctt tcttttttcc ttggtttaag
   960
   aaagccataa agcttatttt aagtggggaa attctaaagg gacattcatc aactgcaaat
   1020
   ttattttcag agtaa
   1035


<210>  28
<211>  344
<212>  PRT
<213>  Mus musculus


<220>
<221>  mTAAR8a
<222>  (1)..(344)

<400>  28

Met Thr Ser Asn Phe Ser Gln Pro Ala Leu Gln Leu Cys Tyr Glu Asn
1               5                   10                  15


Thr Asn Gly Ser Cys Ile Lys Thr Pro Tyr Ser Pro Gly Pro Arg Val
            20                  25                  30


Ile Leu Tyr Met Val Tyr Gly Phe Gly Ala Val Leu Ala Val Cys Gly
        35                  40                  45


Asn Leu Leu Val Val Ile Ser Val Leu His Phe Lys Gln Leu His Ser
    50                  55                  60

Pro Ala Asn Phe Leu Ile Ala Ser Leu Ala Ser Ala Asp Phe Leu Val
65                  70                  75                  80

Gly Ile Ser Val Met Pro Phe Ser Met Val Arg Ser Ile Glu Ser Cys
                85                  90                  95

Trp Tyr Phe Gly Asp Ala Phe Cys Ser Leu His Ser Cys Cys Asp Val
            100                 105                 110

Ala Phe Cys Tyr Ser Ser Val Leu His Leu Cys Phe Ile Ser Val Asp
        115                 120                 125

Arg Tyr Ile Ala Val Thr Asp Pro Leu Val Tyr Pro Thr Lys Phe Thr
    130                 135                 140

Val Ser Val Ser Gly Ile Cys Ile Ser Ile Ser Trp Ile Leu Pro Leu
145                 150                 155                 160

Val Tyr Ser Ser Ala Val Phe Tyr Thr Gly Ile Ser Ala Lys Gly Ile
                165                 170                 175

Glu Ser Leu Val Ser Ala Leu Asn Cys Val Gly Gly Cys Gln Ile Val
            180                 185                 190

Ile Asn Gln Asp Phe Val Leu Ile Ser Phe Leu Leu Phe Phe Ile Pro
        195                 200                 205

Thr Leu Val Met Ile Ile Leu Tyr Ser Lys Ile Phe Leu Val Ala Lys
    210                 215                 220

Gln Gln Ala Val Lys Ile Glu Thr Ser Val Ser Gly Asn Arg Gly Glu
225                 230                 235                 240

Ser Ser Ser Glu Ser His Lys Ala Arg Val Ala Lys Arg Glu Arg Lys
                245                 250                 255

Ala Ala Lys Thr Leu Gly Val Thr Val Val Ala Phe Met Val Ser Trp
            260                 265                 270

Leu Pro Tyr Thr Ile Asp Ala Leu Val Asp Ala Phe Met Gly Phe Ile
        275                 280                 285

Thr Pro Ala Tyr Val Tyr Glu Ile Cys Cys Trp Gly Thr Tyr Tyr Asn
    290                 295                 300

Ser Ala Met Asn Pro Leu Ile Tyr Ala Phe Phe Phe Pro Trp Phe Lys
305                 310                 315                 320

Lys Ala Ile Lys Leu Ile Leu Ser Gly Glu Ile Leu Lys Gly His Ser
                325                 330                 335

Ser Thr Ala Asn Leu Phe Ser Glu
            340

<210> 29
<211> 1035
<212> DNA
<213> Mus musculus

<220>
<221> mTAAR8b
<222> (1)..(1035)

<400> 29
atgaccagca acttttccca accagccctg cagctctgct atgagaacac gaatggatcc
60

tgtattaaaa ctccctattc tccagggccc cgggtcatcc tgtacatggt ctttggcttt
120

ggggctgtgc tggcagtgtg tgggaacctc ctggtggtga tttcagttct ccatttcaag
180

cagctgcact ctccagccaa ttttctcatc gcctctctgg ccagcgctga cttcttggtg
240

ggcatctctg tgatgccctt cagcatggtc aggtccattg agagctgctg gtactttgga
300

gatgcatttt gtagccttca cagttgctgt gatgtggctt tttgttactc ttctgccctc
360

cacctgtgct tcatctcagt ggacaggtac attgctgtca cagaccctct ggtctatccc
420

accaagttca cagtgtctgt gtctggaatt tgcatcagca tctcctggat tctgcccctg
480

gtatacagca gtgcagtgtt ctacacaggc atcagtgcta aggggattga aagcttagta
540

agtgctctga attgtgtagg gggctgccaa atcgttgtca atcaagactg ggttttgata
600

gattttcttc tatttttcat acctaccctt gttatgatca ttctttacag caaaatattt
660

ttggtagcca aacagcaagc tgtaaaaatt gaaacttctg taagtgacaa cagaggtgaa
720

tcatcctcag agagtcacaa agccagagtg gccaagagag agaggaaggc tgcaaaaacc
780

ttgggggtca ctgtggtggc ttttatggtc tcgtggctcc catacacaat tgactcattg
840

gttgatgctt tcgtgggctt cattactcct gcctatgtct atgaaatctg ttgctggagt
900

gcctattata actcagccat gaaccctttg atttatgctt tcttttatcc ttggtttagg
960

aaagccataa agcttatttt aagtggggag attctaaaga gtcattcatc taccatgagt
1020

ttgttttcag agtaa
1035

<210> 30
<211> 344
<212> PRT
<213> Mus musculus

<220>
<221> mTAAR8b
<222> (1)..(344)

<400> 30

Met Thr Ser Asn Phe Ser Gln Pro Ala Leu Gln Leu Cys Tyr Glu Asn
1               5                   10                  15

Thr Asn Gly Ser Cys Ile Lys Thr Pro Tyr Ser Pro Gly Pro Arg Val
            20                  25                  30

Ile Leu Tyr Met Val Phe Gly Phe Gly Ala Val Leu Ala Val Cys Gly
        35                  40                  45

Asn Leu Leu Val Val Ile Ser Val Leu His Phe Lys Gln Leu His Ser
    50                  55                  60

Pro Ala Asn Phe Leu Ile Ala Ser Leu Ala Ser Ala Asp Phe Leu Val
65                  70                  75                  80

Gly Ile Ser Val Met Pro Phe Ser Met Val Arg Ser Ile Glu Ser Cys
                85                  90                  95

Trp Tyr Phe Gly Asp Ala Phe Cys Ser Leu His Ser Cys Cys Asp Val
            100                 105                 110

Ala Phe Cys Tyr Ser Ser Ala Leu His Leu Cys Phe Ile Ser Val Asp
        115                 120                 125

Arg Tyr Ile Ala Val Thr Asp Pro Leu Val Tyr Pro Thr Lys Phe Thr
    130                 135                 140

Val Ser Val Ser Gly Ile Cys Ile Ser Ile Ser Trp Ile Leu Pro Leu
145                 150                 155                 160

Val Tyr Ser Ser Ala Val Phe Tyr Thr Gly Ile Ser Ala Lys Gly Ile
                165                 170                 175

Glu Ser Leu Val Ser Ala Leu Asn Cys Val Gly Gly Cys Gln Ile Val
            180                 185                 190

Val Asn Gln Asp Trp Val Leu Ile Asp Phe Leu Leu Phe Phe Ile Pro
        195                 200                 205

Thr Leu Val Met Ile Ile Leu Tyr Ser Lys Ile Phe Leu Val Ala Lys
    210                 215                 220

Gln Gln Ala Val Lys Ile Glu Thr Ser Val Ser Asp Asn Arg Gly Glu
225                 230                 235                 240

Ser Ser Ser Glu Ser His Lys Ala Arg Val Ala Lys Arg Glu Arg Lys
                245                 250                 255

Ala Ala Lys Thr Leu Gly Val Thr Val Val Ala Phe Met Val Ser Trp
            260                 265                 270

Leu Pro Tyr Thr Ile Asp Ser Leu Val Asp Ala Phe Val Gly Phe Ile
        275                 280                 285

Thr Pro Ala Tyr Val Tyr Glu Ile Cys Cys Trp Ser Ala Tyr Tyr Asn
    290                 295                 300

Ser Ala Met Asn Pro Leu Ile Tyr Ala Phe Phe Tyr Pro Trp Phe Arg
305                 310                 315                 320

Lys Ala Ile Lys Leu Ile Leu Ser Gly Glu Ile Leu Lys Ser His Ser
                325                 330                 335

Ser Thr Met Ser Leu Phe Ser Glu
            340

<210>  31
<211>  1035
<212>  DNA
<213>  Mus musculus

<220>
<221>  mTAAR8c
<222>  (1)..(1035)

<400>  31
atgaccagca acttttccca accagccctg cagctctgct atgagaacac gaatggatcc
60

tgtattaaaa ctccctattc tccagggccc cgggtcatcc tgtacatggt ctatggcttt
120

ggggctgtgc tggcagtgtg tgggaacctc ctggtggtga tttcagttct ccatttcaag
180

cagctgcact ctccagccaa ttttctcatc gcctctctgg ccagtgctga cttcttggtg
240

ggcatctctg tgatgccctt cagcatggtc aggtccattg agagctgctg gtactttgga
300

gatgcatttt gtagccttca cagttgctgt gatgtggctt tttgttactc ttctgccctc
360

cacctgtgct tcatctcagt ggacaggtac atagctgtca cggaccctct ggtctatccc
420

accaagttca cagtgtctgt gtctggaatt tgcatcagca tctcctggat tctgcccctg
480

gtatacagca gtgcagtgtt ctacacaggc atcagtgcta aggggattga aagcttagta
540

agtgctctga attgtgtagg gggctgccaa gttgttgtca atcaagactg ggttttgata
600

agttttcttt tattcttcat acctaccgtt gttatgatca ttctttacag caaaatattt
660

ttggtagcca aacagcaagc tgtaaaaatt gaaacttctg taagtggcaa cagaggtgaa
720

tcatcctcag agagtcacaa agccagagtg gccaagagag agaggaaggc tgcaaaaacc
780

ttgggggtca ctgtggtggc ttttatggtc tcgtggctcc catacacaat tgatgcattg
840

gttgatgctt tcatgggctt catcactcct gcctatgtct atgaaatctg ttgctggagt
900

gcctattata actcagccat gaaccctttg atttatgctt tcttttatcc ttggtttagg
960

aaagccataa agcttatttt aagtgggaaa attctaaagg gacattcatc aactacaaat
1020

ttattttcag agtaa
1035

<210>  32
<211>  344
<212>  PRT
<213>  Mus musculus

<220>
<221>  mTAAR8c
<222>  (1)..(344)

<400>  32

Met Thr Ser Asn Phe Ser Gln Pro Ala Leu Gln Leu Cys Tyr Glu Asn
1               5                   10                  15

Thr Asn Gly Ser Cys Ile Lys Thr Pro Tyr Ser Pro Gly Pro Arg Val
            20                  25                  30

Ile Leu Tyr Met Val Tyr Gly Phe Gly Ala Val Leu Ala Val Cys Gly
        35                  40                  45

Asn Leu Leu Val Val Ile Ser Val Leu His Phe Lys Gln Leu His Ser
    50                  55                  60

Pro Ala Asn Phe Leu Ile Ala Ser Leu Ala Ser Ala Asp Phe Leu Val
65                  70                  75                  80

Gly Ile Ser Val Met Pro Phe Ser Met Val Arg Ser Ile Glu Ser Cys
                85                  90                  95

Trp Tyr Phe Gly Asp Ala Phe Cys Ser Leu His Ser Cys Cys Asp Val
            100                 105                 110

Ala Phe Cys Tyr Ser Ser Ala Leu His Leu Cys Phe Ile Ser Val Asp
        115                 120                 125

Arg Tyr Ile Ala Val Thr Asp Pro Leu Val Tyr Pro Thr Lys Phe Thr
    130                 135                 140

Val Ser Val Ser Gly Ile Cys Ile Ser Ile Ser Trp Ile Leu Pro Leu
145                 150                 155                 160

Val Tyr Ser Ser Ala Val Phe Tyr Thr Gly Ile Ser Ala Lys Gly Ile
                165                 170                 175

Glu Ser Leu Val Ser Ala Leu Asn Cys Val Gly Gly Cys Gln Val Val
            180                 185                 190

Val Asn Gln Asp Trp Val Leu Ile Ser Phe Leu Leu Phe Phe Ile Pro
        195                 200                 205

Thr Val Val Met Ile Ile Leu Tyr Ser Lys Ile Phe Leu Val Ala Lys
    210                 215                 220

Gln Gln Ala Val Lys Ile Glu Thr Ser Val Ser Gly Asn Arg Gly Glu
225                 230                 235                 240

Ser Ser Ser Glu Ser His Lys Ala Arg Val Ala Lys Arg Glu Arg Lys
                245                 250                 255

Ala Ala Lys Thr Leu Gly Val Thr Val Val Ala Phe Met Val Ser Trp
            260                 265                 270

Leu Pro Tyr Thr Ile Asp Ala Leu Val Asp Ala Phe Met Gly Phe Ile
        275                 280                 285

Thr Pro Ala Tyr Val Tyr Glu Ile Cys Cys Trp Ser Ala Tyr Tyr Asn
    290                 295                 300

Ser Ala Met Asn Pro Leu Ile Tyr Ala Phe Phe Tyr Pro Trp Phe Arg
305                 310                 315                 320

Lys Ala Ile Lys Leu Ile Leu Ser Gly Lys Ile Leu Lys Gly His Ser
                325                 330                 335

Ser Thr Thr Asn Leu Phe Ser Glu

            340

<210>  33
<211>  1047
<212>  DNA
<213>  Mus musculus


<220>
<221>  mTAAR9
<222>  (1)..(1047)

<400>  33
atgacaagcg acttctcccc agaaccgccc atggagctct gctatgagaa tgtgaacgga
60

tcctgcatta aaagctctta cgcaccctgg cctcgagcca tcctctatgg ggtcctcggt
120

ttgggagccc tgctggcagt gtttgggaac ttactggtca tcattgccat cctccacttt
180

aaacagctgc acacgcctac gaacttcctg gtggcctccc tggcctgtgc tgacttcttg
240

gtgggggtga cagtgatgcc cttcagcacg gtgcgttctg tggagagctg ctggtacttt
300

ggggagagtt actgtaagtt ccacacgtgt ttcgacacct ccttctgttt tgcgtctctg
360

tttcatttat gctgcatctc cattgaccgg tacattgcag ttacagatcc actgacctat
420

ccgaccaagt tcactgtatc ggtttctgga ctgtgcattg ctctctcttg gttcttttct
480

gtcacataca gcttttccat cttttacacg ggagccaatg aggaagggat tgaggaatta
540

gtggttgctc tgacctgtgt gggaggctgc caggctccac tgaatcaaaa ttgggtttta
600

ctttgtttcc ttttgttctt tctgcccact gtggtcatgg tgtttctata tggtcggata
660

tttttggtgg caaagtatca ggctaggaag atagagggta cagccaacca agctcaggcc
720

tcctctgaga gctataagga aagagtagcc aaaagagaga ggaaggctgc caagaccttg
780

gggatcgcca tggctgcatt tctcgtgtcc tggctgccat acattattga tgcggttatt
840

gacgcttaca tgaacttcat aactcctgcg tacgtctatg agatattagt gtggtgtgtt
900

tactacaatt cagctatgaa ccccttgata tacgcctttt tttatccttg gtttcgtaag
960

gcaataaaac ttattgtgag tggcaaagtc ttcagggctg attcatcaac aactaatcta
1020

ttctctgaag aggcaggtgc aggttaa
1047

<210>  34
<211>  348
<212>  PRT
<213>  Mus musculus

<220>
<221>  mTAAR9
<222>  (1)..(348)

<400>  34

Met Thr Ser Asp Phe Ser Pro Glu Pro Pro Met Glu Leu Cys Tyr Glu
1               5                   10                  15

Asn Val Asn Gly Ser Cys Ile Lys Ser Ser Tyr Ala Pro Trp Pro Arg
            20                  25                  30

Ala Ile Leu Tyr Gly Val Leu Gly Leu Gly Ala Leu Leu Ala Val Phe
        35                  40                  45

Gly Asn Leu Leu Val Ile Ile Ala Ile Leu His Phe Lys Gln Leu His
    50                  55                  60

Thr Pro Thr Asn Phe Leu Val Ala Ser Leu Ala Cys Ala Asp Phe Leu
65                  70                  75                  80

Val Gly Val Thr Val Met Pro Phe Ser Thr Val Arg Ser Val Glu Ser
                85                  90                  95

Cys Trp Tyr Phe Gly Glu Ser Tyr Cys Lys Phe His Thr Cys Phe Asp
            100                 105                 110

Thr Ser Phe Cys Phe Ala Ser Leu Phe His Leu Cys Cys Ile Ser Ile
        115                 120                 125

Asp Arg Tyr Ile Ala Val Thr Asp Pro Leu Thr Tyr Pro Thr Lys Phe
    130                 135                 140

Thr Val Ser Val Ser Gly Leu Cys Ile Ala Leu Ser Trp Phe Phe Ser
145                 150                 155                 160

Val Thr Tyr Ser Phe Ser Ile Phe Tyr Thr Gly Ala Asn Glu Glu Gly
                165                 170                 175

Ile Glu Glu Leu Val Val Ala Leu Thr Cys Val Gly Gly Cys Gln Ala
            180                 185                 190

Pro Leu Asn Gln Asn Trp Val Leu Leu Cys Phe Leu Leu Phe Phe Leu
        195                 200                 205

Pro Thr Val Val Met Val Phe Leu Tyr Gly Arg Ile Phe Leu Val Ala

210                 215                 220

Lys Tyr Gln Ala Arg Lys Ile Glu Gly Thr Ala Asn Gln Ala Gln Ala
225                 230                 235                 240

Ser Ser Glu Ser Tyr Lys Glu Arg Val Ala Lys Arg Glu Arg Lys Ala
                245                 250                 255

Ala Lys Thr Leu Gly Ile Ala Met Ala Ala Phe Leu Val Ser Trp Leu
            260                 265                 270

Pro Tyr Ile Ile Asp Ala Val Ile Asp Ala Tyr Met Asn Phe Ile Thr
        275                 280                 285

Pro Ala Tyr Val Tyr Glu Ile Leu Val Trp Cys Val Tyr Tyr Asn Ser
    290                 295                 300

Ala Met Asn Pro Leu Ile Tyr Ala Phe Phe Tyr Pro Trp Phe Arg Lys
305                 310                 315                 320

Ala Ile Lys Leu Ile Val Ser Gly Lys Val Phe Arg Ala Asp Ser Ser
                325                 330                 335

Thr Thr Asn Leu Phe Ser Glu Glu Ala Gly Ala Gly
            340                 345

<210>  35
<211>  18
<212>  DNA
<213>  Mus musculus

<220>
<221>  musTAR2_5_01
<222>  (1)..(18)
<223>  primer

<400>  35
atgaatacac ccgacccc
18

<210>  36
<211>  19
<212>  DNA
<213>  Mus musculus

<220>
<221>  musTAR2_3_01
<222>  (1)..(19)
<223>  primer

<400>  36
ctaaggatgt gcaggatgc
19

<210> 37
<211> 18
<212> DNA
<213> Mus musculus

<220>
<221> musGPR58_5_01
<222> (1)..(18)
<223> primer

<400> 37
atggcatctt ttgaagcc
18

<210> 38
<211> 27
<212> DNA
<213> Mus musculus

<220>
<221> musGPR58_3_01
<222> (1)..(27)
<223> primer

<400> 38
ctattctgtt tctttttgag taaacaa
27

<210> 39
<211> 25
<212> DNA
<213> Mus musculus

<220>
<221> musGPR57_5_01
<222> (1)..(25)
<223> primer

<400> 39
atggatctaa tatacattcc cgaag
25

<210> 40
<211> 22
<212> DNA
<213> Mus musculus

<220>
<221> musGPR57_3_01
<222> (1)..(22)
<223> primer

<400> 40
ttaatgtgct tcaggaaaaa ga
22

<210> 41
<211> 18
<212> DNA

<213>  Mus musculus

<220>
<221>  musTAR2_5_01
<222>  (1)..(18)
<223>  primer

<400>  41
atgaatacac ccgacccc
18

<210>  42
<211>  19
<212>  DNA
<213>  Mus musculus

<220>
<221>  musTAR2_3_01
<222>  (1)..(19)
<223>  primer

<400>  42
ctaaggatgt gcaggatgc
19

<210>  43
<211>  19
<212>  DNA
<213>  Mus musculus

<220>
<221>  musPNR_5_01
<222>  (1)..(19)
<223>  primer

<400>  43
atgagagctg tcctcctcc
19

<210>  44
<211>  25
<212>  DNA
<213>  Mus musculus

<220>
<221>  musPNR_3_01
<222>  (1)..(25)
<223>  primer

<400>  44
tcagtcatgg tataaatcaa cagtc
25

<210>  45
<211>  20
<212>  DNA
<213>  Mus musculus

<220>
<221>  musTAR4_5_01
<222>  (1)..(20)
<223>  primer

<400>  45
atgggcagta actcgtctcc
20


<210>  46
<211>  25
<212>  DNA
<213>  Mus musculus


<220>
<221>  musTAR4_3_01
<222>  (1)..(25)
<223>  primer

<400>  46
ttatatttgc tcagagaaca agttg
25


<210>  47
<211>  19
<212>  DNA
<213>  Mus musculus


<220>
<221>  musTAR8_5_01
<222>  (1)..(19)
<223>  primer

<400>  47
atggacaaat tggttgacc
19


<210>  48
<211>  22
<212>  DNA
<213>  Mus musculus


<220>
<221>  musTAR8_3_01
<222>  (1)..(22)
<223>  primer

<400>  48
ctactcagga aacaagttgg tg
22


<210>  49
<211>  24
<212>  DNA
<213>  Mus musculus


<220>
<221>  musTAR12_5_01
<222>  (1)..(24)

<223>  primer

<400>  49
tggctacaga taatgacagt tttc
24


<210>  50
<211>  22
<212>  DNA
<213>  Mus musculus


<220>
<221>  musTAR12_3_01
<222>  (1)..(22)
<223>  primer

<400>  50
ctactcagga aacaagttgg tg
22


<210>  51
<211>  25
<212>  DNA
<213>  Mus musculus


<220>
<221>  musTAR17_5_01
<222>  (1)..(25)
<223>  primer

<400>  51
atgactacag gtgatgacag ttttc
25


<210>  52
<211>  20
<212>  DNA
<213>  Mus musculus


<220>
<221>  musTAR17_3_01
<222>  (1)..(20)
<223>  primer

<400>  52
ctattcggga aacaggttgg
20


<210>  53
<211>  20
<212>  DNA
<213>  Mus musculus


<220>
<221>  musTAR14_5_01
<222>  (1)..(20)
<223>  primer

<400>  53

atggctacag gtgatgacag
20

<210> 54
<211> 20
<212> DNA
<213> Mus musculus

<220>
<221> musTAR14_3_01
<222> (1)..(20)
<223> primer

<400> 54
ctattcagga aacaggttgg
20

<210> 55
<211> 20
<212> DNA
<213> Mus musculus

<220>
<221> musTAR9_5_01
<222> (1)..(20)
<223> primer

<400> 55
atggctacag gtgatgacag
20

<210> 56
<211> 20
<212> DNA
<213> Mus musculus

<220>
<221> musTAR9_3_01
<222> (1)..(20)
<223> primer

<400> 56
ctattcagga aacaggttgg
20

<210> 57
<211> 26
<212> DNA
<213> Mus musculus

<220>
<221> musTAR6_5_01
<222> (1)..(26)
<223> primer

<400> 57
atgtctatag ctgatgaaac tgtttc
26

<210>  58
<211>  23
<212>  DNA
<213>  Mus musculus


<220>
<221>  musTAR6_3_01
<222>  (1)..(23)
<223>  primer

<400>  58
ctactcagaa aacagattgg tgg
23


<210>  59
<211>  19
<212>  DNA
<213>  Mus musculus


<220>
<221>  musTAR11_5_01
<222>  (1)..(19)
<223>  primer

<400>  59
atgaccagca acttttccc
19


<210>  60
<211>  26
<212>  DNA
<213>  Mus musculus


<220>
<221>  musTAR11_3_01
<222>  (1)..(26)
<223>  primer

<400>  60
ttactctgaa aataaatttg cagttg
26


<210>  61
<211>  19
<212>  DNA
<213>  Mus musculus


<220>
<221>  musTAR7_5_01
<222>  (1)..(19)
<223>  primer

<400>  61
atgaccagca acttttccc
19


<210>  62
<211>  26

<212>  DNA
<213>  Mus musculus


<220>
<221>  musTAR7_3_01
<222>  (1)..(26)
<223>  primer

<400>  62
ttactctgaa aacaaactca tggtag
26


<210>  63
<211>  19
<212>  DNA
<213>  Mus musculus


<220>
<221>  musTAR10_5_01
<222>  (1)..(19)
<223>  primer

<400>  63
atgaccagca acttttccc
19


<210>  64
<211>  28
<212>  DNA
<213>  Mus musculus


<220>
<221>  musTAR10_3_01
<222>  (1)..(28)
<223>  primer

<400>  64
ttactctgaa aataaatttg tagttgat
28


<210>  65
<211>  19
<212>  DNA
<213>  Mus musculus


<220>
<221>  musTAR3_5_01
<222>  (1)..(19)
<223>  primer

<400>  65
atgacaagcg acttctccc
19


<210>  66
<211>  18
<212>  DNA
<213>  Mus musculus

<220>
<221>  musTAR3_3_01
<222>  (1)..(18)
<223>  primer

<400>  66
ttaacctgca cctgcctc
18

### Annex to the application documents - subsequently filed sequences listing

SEQUENCE LISTING

<110>  F.Hoffmann-La Roche

<120>  Mouse Trace Amine Associated Receptors

<130>  22646

<140>  EP05013872.6
<141>  2005-06-28

<160>  66

<170>  PatentIn version 3.3

<210>  1
<211>  16
<212>  PRT
<213>  Mus musculus


<220>
<221>  VARIANT
<222>  (3)..(4)
<223>  Xaa may be any naturally occuring amino acid

<220>
<221>  VARIANT
<222>  (7)..(8)
<223>  Xaa may be any naturally occuring amino acid

<220>
<221>  VARIANT
<222>  (9)..(9)
<223>  Zaa may be tyrosine or histidine

<220>
<221>  VARIANT
<222>  (10)..(12)
<223>  Xaa may be any naturally occuring amino acid

<220>
<221>  VARIANT
<222>  (13)..(13)
<223>  Baa may be tyrosine or phenylalanine

<220>
<221>  VARIANT
<222>  (14)..(14)
<223>  Xaa may be any naturally occuring amino acid

<400>  1

Asn Ser Xaa Xaa Asn Pro Xaa Xaa Glx Xaa Xaa Xaa Asx Xaa Trp Phe
1               5                   10                  15


<210>  2
<211>  999
<212>  DNA
<213>  Mus musculus

<400>  2
atgcatcttt gccacgctat cacaaacatt tcccacagaa acagcgactg gtcaagagaa      60

gtccaagctt ccctgtacag cttaatgtca ctcataatcc tggccactct ggttggcaac     120
ttaatagtaa taatttccat atcccatttc aagcaacttc atacacccac caactggctc     180
cttcactcca tggccattgt cgactttctg ctgggctgtc tgataatgcc ctgcagcatg     240
gtgagaactg ttgagcgctg ttggtatttt ggggaaatcc tctgtaaagt tcacaccagc     300
accgatatca tgctgagctc cgcctccatt ttccacttag ctttcatttc cattgaccgc     360
tactgtgctg tgtgtgaccc tttgagatac aaagccaaga tcaatatctc cactattctt     420
gtgatgatcc tcgttagttg gagccttcct gctgtttatg catttgggat gatcttcctg     480
gaactgaact taaaaggagt ggaagagctg tatcgcagtc aggtcagcga cctgggcggc     540
tgttctccct tctttagtaa agtatctggg gtactggcgt tcatgacttc cttctatata     600
cctggatctg ttatgttatt tgtttactat aggatatatt tcatagctaa aggacaagca     660
aggtcaatca atcgtacgaa tgttcaagtt ggattggaag ggaaaagcca agcaccacaa     720
agcaaggaaa caaaagccgc gaagacctta gggatcatgg tgggcgtttt cctcgtatgc     780
tggtgcccgt tctttctctg cacggtcctg gaccctttcc tgggctatgt tatcccaccc     840
tctctgaatg acgcactgta ttggtttggg tacttgaatt ctgccctcaa tccgatggtt     900
tatgcctttt tctatccctg gttcagaaga gccttgaaga tggttctcct tggtaaaatt     960
ttccaaaaag attcatctag gtctaagcta tttttgtaa                            999

<210>  3
<211>  332
<212>  PRT
<213>  Mus musculus

<400>  3

Met His Leu Cys His Ala Ile Thr Asn Ile Ser His Arg Asn Ser Asp
1               5                   10                  15

Trp Ser Arg Glu Val Gln Ala Ser Leu Tyr Ser Leu Met Ser Leu Ile
            20                  25                  30

Ile Leu Ala Thr Leu Val Gly Asn Leu Ile Val Ile Ile Ser Ile Ser
        35                  40                  45

His Phe Lys Gln Leu His Thr Pro Thr Asn Trp Leu Leu His Ser Met
    50                  55                  60

Ala Ile Val Asp Phe Leu Leu Gly Cys Leu Ile Met Pro Cys Ser Met
65                  70                  75                  80

Val Arg Thr Val Glu Arg Cys Trp Tyr Phe Gly Glu Ile Leu Cys Lys
                85                  90                  95

Val His Thr Ser Thr Asp Ile Met Leu Ser Ser Ala Ser Ile Phe His
            100                 105                 110

Leu Ala Phe Ile Ser Ile Asp Arg Tyr Cys Ala Val Cys Asp Pro Leu
        115                 120                 125

Arg Tyr Lys Ala Lys Ile Asn Ile Ser Thr Ile Leu Val Met Ile Leu
    130                 135                 140

Val Ser Trp Ser Leu Pro Ala Val Tyr Ala Phe Gly Met Ile Phe Leu
145                 150                 155                 160

Glu Leu Asn Leu Lys Gly Val Glu Glu Leu Tyr Arg Ser Gln Val Ser
                165                 170                 175

Asp Leu Gly Gly Cys Ser Pro Phe Phe Ser Lys Val Ser Gly Val Leu
            180                 185                 190

Ala Phe Met Thr Ser Phe Tyr Ile Pro Gly Ser Val Met Leu Phe Val
        195                 200                 205

Tyr Tyr Arg Ile Tyr Phe Ile Ala Lys Gly Gln Ala Arg Ser Ile Asn
    210                 215                 220

Arg Thr Asn Val Gln Val Gly Leu Glu Gly Lys Ser Gln Ala Pro Gln
225                 230                 235                 240

Ser Lys Glu Thr Lys Ala Ala Lys Thr Leu Gly Ile Met Val Gly Val
                245                 250                 255

Phe Leu Val Cys Trp Cys Pro Phe Phe Leu Cys Thr Val Leu Asp Pro
            260                 265                 270

Phe Leu Gly Tyr Val Ile Pro Pro Ser Leu Asn Asp Ala Leu Tyr Trp
        275                 280                 285

Phe Gly Tyr Leu Asn Ser Ala Leu Asn Pro Met Val Tyr Ala Phe Phe
    290                 295                 300

Tyr Pro Trp Phe Arg Arg Ala Leu Lys Met Val Leu Leu Gly Lys Ile
305                 310                 315                 320

Phe Gln Lys Asp Ser Ser Arg Ser Lys Leu Phe Leu
                325                 330

<210> 4
<211> 1020
<212> DNA

<213>  Mus musculus

<400>  4
atggcatctt ttgaagccca gcaggaaacc tttgattgtt cagagtacgg aaatggatct      60

tgcccagaga atgaaagatc tctcggcgtc cgagctgcca tgtactcgtt gatggcctgc     120

gccatattca tcacgatctt tggcaacctg gccatgatca tttccatttc ctacttcaaa     180

cagcttcaca caccgaccaa cctcctcatt ctctccatgg ccgtcaccga cttccttctc     240

ggattcacca tcatgccata tagtatggtc aggtcagtgg aaaactgctg gtatttcgga     300

cttacgtttt gcaagatcca ttatagcttc gacctgatgc ttagcataac gtccattttc     360

cacctttgct cggtggccgt cgatagattt tatgccatct gtcacccttt gcattattgc     420

accaaaatga ctatcccggt cgttaggcgg ctgctgctcg tctgctggtc agtccccggg     480

gcgtttgcct ttggggtggt cttctccgag gcttacgctg atggaattga aggctatgac     540

attttggttg catgttccag ttcctgccca gtcatgttca acaagctatg ggggaccacc     600

ttgtttgtgg caggcttttt cactcctagc tcgatgatgg tggggattta cggcaaaatt     660

ttcgctgtat ccaaaaaaca cgctcgcgta attgacaact tgccagaaaa tcaaaacaat     720

caaatgagga aggacaaaaa agcagccaaa actttaggga tagtgatggg tgttttctta     780

ttgtgttggt ttccatgttt cttcaccatc ctgttagatc cgtttctgaa tttctctacg     840

cctgcagttc tgtttgatgc cttaacatgg ttcggttatt ttaattccac atgtaatccc     900

ttaatttatg gtttcttcta cccttggttt cgcagagcac tcaagtacat tttactgggt     960

aaaattttca gctcgcattt ccataatact aatttgttta ctcaaaaaga aacagaatag    1020

<210>  5
<211>  339
<212>  PRT
<213>  Mus musculus

<400>  5

Met Ala Ser Phe Glu Ala Gln Gln Glu Thr Phe Asp Cys Ser Glu Tyr
1               5                   10                  15

Gly Asn Gly Ser Cys Pro Glu Asn Glu Arg Ser Leu Gly Val Arg Ala
            20                  25                  30

Ala Met Tyr Ser Leu Met Ala Cys Ala Ile Phe Ile Thr Ile Phe Gly
        35                  40                  45

Asn Leu Ala Met Ile Ile Ser Ile Ser Tyr Phe Lys Gln Leu His Thr
    50                  55                  60

Pro Thr Asn Leu Leu Ile Leu Ser Met Ala Val Thr Asp Phe Leu Leu
65                  70                  75                  80

Gly Phe Thr Ile Met Pro Tyr Ser Met Val Arg Ser Val Glu Asn Cys
                85                  90                  95

Trp Tyr Phe Gly Leu Thr Phe Cys Lys Ile His Tyr Ser Phe Asp Leu
            100                 105                 110

Met Leu Ser Ile Thr Ser Ile Phe His Leu Cys Ser Val Ala Val Asp
        115                 120                 125

Arg Phe Tyr Ala Ile Cys His Pro Leu His Tyr Cys Thr Lys Met Thr
    130                 135                 140

Ile Pro Val Val Arg Arg Leu Leu Leu Val Cys Trp Ser Val Pro Gly
145                 150                 155                 160

Ala Phe Ala Phe Gly Val Val Phe Ser Glu Ala Tyr Ala Asp Gly Ile
                165                 170                 175

Glu Gly Tyr Asp Ile Leu Val Ala Cys Ser Ser Ser Cys Pro Val Met
            180                 185                 190

Phe Asn Lys Leu Trp Gly Thr Thr Leu Phe Val Ala Gly Phe Phe Thr
        195                 200                 205

Pro Ser Ser Met Met Val Gly Ile Tyr Gly Lys Ile Phe Ala Val Ser
    210                 215                 220

Lys Lys His Ala Arg Val Ile Asp Asn Leu Pro Glu Asn Gln Asn Asn
225                 230                 235                 240

Gln Met Arg Lys Asp Lys Lys Ala Ala Lys Thr Leu Gly Ile Val Met
                245                 250                 255

Gly Val Phe Leu Leu Cys Trp Phe Pro Cys Phe Phe Thr Ile Leu Leu
            260                 265                 270

Asp Pro Phe Leu Asn Phe Ser Thr Pro Ala Val Leu Phe Asp Ala Leu
        275                 280                 285

Thr Trp Phe Gly Tyr Phe Asn Ser Thr Cys Asn Pro Leu Ile Tyr Gly
    290                 295                 300

Phe Phe Tyr Pro Trp Phe Arg Arg Ala Leu Lys Tyr Ile Leu Leu Gly
305                 310                 315                 320

Lys Ile Phe Ser Ser His Phe His Asn Thr Asn Leu Phe Thr Gln Lys
                325                 330                 335

Glu Thr Glu

<210> 6
<211> 1032
<212> DNA
<213> Mus musculus

<400> 6
atggatctaa tatacattcc cgaagactta tccagctgtc caaaatttgg aaataaatcc 60

tgccctccca ccaatcgctc tttccgtgtc cgcatgataa tgtacctgtt tatgactgga 120

gccatggtta tcaccatctt tggaaactta gtgataatca tttccatatc gcatttcaaa 180

cagctacatt ctcccaccaa ctttctgatc ctctccatgg caaccacgga cttcctgctg 240

ggttttgtca tcatgcctta cagtatggtg agatcagtgg agagctgctg gtatttcgga 300

gacagctttt gcaaattcca cgcgagcttc gacatgatgc taagcctgac gtccatattc 360

cacctgtgct ccatcgcgat cgaccggttt tatgcagtgt gtgacccttt gcactacacc 420

accaccatga cggtatccat gatcaagaga ctgctggctt tttgctgggc agccccagct 480

ctcttttcgt ttggtttagt tctgtcagag gccaatgttt ctggtatgca gagctacgag 540

attcttgttg cttgcttcaa tttctgtgcg cttacgttca acaagttttg ggggaccata 600

ctgttcacta cctgcttctt cactcctggc tccatcatgg ttggtattta tggtaaaatt 660

tttattgttt ctagacgaca tgctcgagcc ctcagcgata tgcctgcgaa cacaaaagga 720

gcagtaggga aaaacctgtc taagaaaaag gacaggaaag cagctaagac cctgggcatc 780

gtcatggggg tgtttctagc gtgctggctg ccttgcttcc tggctgtttt gattgatcca 840

tatttagact actccacgcc catcatagtg ctggatcttc tggtatggct cgggtacttc 900

aactctactt gcaacccccct catccatggc ttttttaccc cgtggtttcg gaaagctctt 960

cagtttatag tgtcaggaaa aatatttcgc tctaattcag acactgcaaa tctttttcct 1020

gaagcacatt aa 1032

<210> 7
<211> 343
<212> PRT
<213> Mus musculus

<400> 7

Met Asp Leu Ile Tyr Ile Pro Glu Asp Leu Ser Ser Cys Pro Lys Phe
1               5                   10                  15

Gly Asn Lys Ser Cys Pro Pro Thr Asn Arg Ser Phe Arg Val Arg Met
            20                  25                  30

Ile Met Tyr Leu Phe Met Thr Gly Ala Met Val Ile Thr Ile Phe Gly

        35                  40                  45

Asn Leu Val Ile Ile Ile Ser Ile Ser His Phe Lys Gln Leu His Ser
    50                  55                  60

Pro Thr Asn Phe Leu Ile Leu Ser Met Ala Thr Thr Asp Phe Leu Leu
65                  70                  75                  80

Gly Phe Val Ile Met Pro Tyr Ser Met Val Arg Ser Val Glu Ser Cys
                85                  90                  95

Trp Tyr Phe Gly Asp Ser Phe Cys Lys Phe His Ala Ser Phe Asp Met
            100                 105                 110

Met Leu Ser Leu Thr Ser Ile Phe His Leu Cys Ser Ile Ala Ile Asp
        115                 120                 125

Arg Phe Tyr Ala Val Cys Asp Pro Leu His Tyr Thr Thr Thr Met Thr
    130                 135                 140

Val Ser Met Ile Lys Arg Leu Leu Ala Phe Cys Trp Ala Ala Pro Ala
145                 150                 155                 160

Leu Phe Ser Phe Gly Leu Val Leu Ser Glu Ala Asn Val Ser Gly Met
                165                 170                 175

Gln Ser Tyr Glu Ile Leu Val Ala Cys Phe Asn Phe Cys Ala Leu Thr
            180                 185                 190

Phe Asn Lys Phe Trp Gly Thr Ile Leu Phe Thr Thr Cys Phe Phe Thr
        195                 200                 205

Pro Gly Ser Ile Met Val Gly Ile Tyr Gly Lys Ile Phe Ile Val Ser
    210                 215                 220

Arg Arg His Ala Arg Ala Leu Ser Asp Met Pro Ala Asn Thr Lys Gly
225                 230                 235                 240

Ala Val Gly Lys Asn Leu Ser Lys Lys Lys Asp Arg Lys Ala Ala Lys
                245                 250                 255

Thr Leu Gly Ile Val Met Gly Val Phe Leu Ala Cys Trp Leu Pro Cys
            260                 265                 270

Phe Leu Ala Val Leu Ile Asp Pro Tyr Leu Asp Tyr Ser Thr Pro Ile
        275                 280                 285

Ile Val Leu Asp Leu Leu Val Trp Leu Gly Tyr Phe Asn Ser Thr Cys

290                 295                 300

Asn Pro Leu Ile His Gly Phe Phe Tyr Pro Trp Phe Arg Lys Ala Leu
305                 310                 315                 320

Gln Phe Ile Val Ser Gly Lys Ile Phe Arg Ser Asn Ser Asp Thr Ala
                325                 330                 335

Asn Leu Phe Pro Glu Ala His
            340

<210>  8
<211>  1044
<212>  DNA
<213>  Mus musculus

<400>  8
atgaatacac ccgacccctg gagctcccca gaagtacagt tttgctttgc tgctgcaaac      60

agttcttgcc caaggaaggc gaggcctgcg ctcgttgtct gtgccatgta cctcatcatg     120

attggagcga tagtgatgac catgctggga aacatggctg tgatcatctc tattgcccac     180

ttcaagcagc tccactcccc aaccaacttc cttattctct ccatggctac cacagacttc     240

ctgttgagtt gcgtggtcat gcccttcagt atgatccggt ccatcgagtc atgctggtac     300

ttcggagacc tcttttgcaa agttcacagc tgctgtgaca tcatgctctg taccacatcc     360

attttccacc tctgcttcat ctcagttgac cgccactatg ccgtctgcga ccctttgcac     420

tacgtcaccc aaatcacgac ccgggtggta ggggtctttc tactcatcag ttggtctgtt     480

cccatctttt ttgcctttgg cttggtattc tcagaattaa atctgattgg tgctgaggat     540

tttgtcgcag ccattgactg tacaggtttg tgtgtgttga tatttaacaa gctctgggga     600

gttctggctt ccttcatagc tttctttctg cctgggacag tcatggtagg gatttacata     660

cacattttca cagttgccca gaaacatgcc aggcagattg gtacaggtcc taggacaaaa     720

caggccctct cagaaagcaa aatgaaggcc acatcaaaaa aggaaagcaa ggccaccaag     780

actttaagca ttgtcatggg agtgtttgtg ttgtgttggc tacccttttt tgtcttgaca     840

atcacagacc ctttcattga ttttacaacc cctgaagatt tgtataatgt tttcctttgg     900

cttggttatt ttaattccac tttcaatccc atcatatatg gcatgttcta tccctggttt     960

cgaaaggccc tgaggatgat agtcacagga acgatcttcc gctctgactc ttctacctca    1020

agcctgcatc ctgcacatcc ttag                                           1044

<210>  9
<211>  347
<212>  PRT
<213>  Mus musculus

<400>  9

Met Asn Thr Pro Asp Pro Trp Ser Ser Pro Glu Val Gln Phe Cys Phe
1               5                   10                  15

Ala Ala Ala Asn Ser Ser Cys Pro Arg Lys Ala Arg Pro Ala Leu Val
            20                  25                  30

Val Cys Ala Met Tyr Leu Ile Met Ile Gly Ala Ile Val Met Thr Met
        35                  40                  45

Leu Gly Asn Met Ala Val Ile Ile Ser Ile Ala His Phe Lys Gln Leu
    50                  55                  60

His Ser Pro Thr Asn Phe Leu Ile Leu Ser Met Ala Thr Thr Asp Phe
65                  70                  75                  80

Leu Leu Ser Cys Val Val Met Pro Phe Ser Met Ile Arg Ser Ile Glu
                85                  90                  95

Ser Cys Trp Tyr Phe Gly Asp Leu Phe Cys Lys Val His Ser Cys Cys
            100                 105                 110

Asp Ile Met Leu Cys Thr Thr Ser Ile Phe His Leu Cys Phe Ile Ser
        115                 120                 125

Val Asp Arg His Tyr Ala Val Cys Asp Pro Leu His Tyr Val Thr Gln
    130                 135                 140

Ile Thr Thr Arg Val Val Gly Val Phe Leu Leu Ile Ser Trp Ser Val
145                 150                 155                 160

Pro Ile Phe Phe Ala Phe Gly Leu Val Phe Ser Glu Leu Asn Leu Ile
                165                 170                 175

Gly Ala Glu Asp Phe Val Ala Ala Ile Asp Cys Thr Gly Leu Cys Val
            180                 185                 190

Leu Ile Phe Asn Lys Leu Trp Gly Val Leu Ala Ser Phe Ile Ala Phe
        195                 200                 205

Phe Leu Pro Gly Thr Val Met Val Gly Ile Tyr Ile His Ile Phe Thr
    210                 215                 220

Val Ala Gln Lys His Ala Arg Gln Ile Gly Thr Gly Pro Arg Thr Lys
225                 230                 235                 240

Gln Ala Leu Ser Glu Ser Lys Met Lys Ala Thr Ser Lys Lys Glu Ser
                245                 250                 255

Lys Ala Thr Lys Thr Leu Ser Ile Val Met Gly Val Phe Val Leu Cys
            260                 265                 270

Trp Leu Pro Phe Phe Val Leu Thr Ile Thr Asp Pro Phe Ile Asp Phe
        275                 280                 285

Thr Thr Pro Glu Asp Leu Tyr Asn Val Phe Leu Trp Leu Gly Tyr Phe
    290                 295                 300

Asn Ser Thr Phe Asn Pro Ile Ile Tyr Gly Met Phe Tyr Pro Trp Phe
305                 310                 315                 320

Arg Lys Ala Leu Arg Met Ile Val Thr Gly Thr Ile Phe Arg Ser Asp
                325                 330                 335

Ser Ser Thr Ser Ser Leu His Pro Ala His Pro
            340                 345

<210>  10
<211>  1014
<212>  DNA
<213>  Mus musculus

<400>  10
atgagagctg tcctcctccc gggctctgga gagcagccta cagcgttctg ctaccaggtg      60

aatgggtctt gccccaggac agtccacccg ctggccatcc aggtcgtcat ctatctggcc     120

tgcgcagtgg gtgtgctgat cacagtcctg gggaatttgt ttgtggtgtt tgcagtgtcc     180

tactttaaag tgctccacac gcccaccaac ttcctgctgc tctccctggc cctggcagac     240

atgttgctag gtctgctggt gctgcccctg agcacagtcc gctctgtgga gagctgctgg     300

ttctttggag acttcctgtg ccgtctgcat acctatctgg acacgctgtt ctgtctcacc     360

tccatcttcc atctctgttt tatttccatt gaccgtcatt gtgccatctg tgaccccctg     420

ctctatccct ccaagttcac agtcaggaca gccctccgct acatcgtggc agggtggggg     480

ataccagcag cctacactgc cttcttcctc tacacagatg tggtagagag agcactcagc     540

cagtggctag aagagatgcc ttgtgtgggc agctgccagc tgctgttcaa taagttctgg     600

ggttggttaa acttccctgc attttttgtt ccctgcctaa tcatgatcag cttgtacctg     660

aagatctttg tggtcgccac caggcaggct cagcagatca gaactctgag ccaaagcttg     720

gctggggctg tcaagcggga aagaaaagct gccaagacgc tgggcattgc tgtgggcatc     780

taccttgtgt gttggcttcc cttcactgtt gacacactag tggacagcct tcttaacttc     840

atcaccccac ccctggtctt tgacatcttt atctggtttg cttatttcaa ttcagcctgt     900

aatcccatca tctacgtctt ctcctaccgg tggtttagga aggcactgaa actgctcctg     960

agccgggaga tcttctcacc aaggacaccg actgttgatt tataccatga ctga          1014

<210> 11
<211> 337
<212> PRT
<213> Mus musculus

<400> 11

Met Arg Ala Val Leu Leu Pro Gly Ser Gly Glu Gln Pro Thr Ala Phe
1               5                   10                  15

Cys Tyr Gln Val Asn Gly Ser Cys Pro Arg Thr Val His Pro Leu Ala
            20                  25                  30

Ile Gln Val Val Ile Tyr Leu Ala Cys Ala Val Gly Val Leu Ile Thr
        35                  40                  45

Val Leu Gly Asn Leu Phe Val Val Phe Ala Val Ser Tyr Phe Lys Val
    50                  55                  60

Leu His Thr Pro Thr Asn Phe Leu Leu Leu Ser Leu Ala Leu Ala Asp
65                  70                  75                  80

Met Leu Leu Gly Leu Leu Val Leu Pro Leu Ser Thr Val Arg Ser Val
                85                  90                  95

Glu Ser Cys Trp Phe Phe Gly Asp Phe Leu Cys Arg Leu His Thr Tyr
            100                 105                 110

Leu Asp Thr Leu Phe Cys Leu Thr Ser Ile Phe His Leu Cys Phe Ile
        115                 120                 125

Ser Ile Asp Arg His Cys Ala Ile Cys Asp Pro Leu Leu Tyr Pro Ser
    130                 135                 140

Lys Phe Thr Val Arg Thr Ala Leu Arg Tyr Ile Val Ala Gly Trp Gly
145                 150                 155                 160

Ile Pro Ala Ala Tyr Thr Ala Phe Phe Leu Tyr Thr Asp Val Val Glu
                165                 170                 175

Arg Ala Leu Ser Gln Trp Leu Glu Glu Met Pro Cys Val Gly Ser Cys
            180                 185                 190

Gln Leu Leu Phe Asn Lys Phe Trp Gly Trp Leu Asn Phe Pro Ala Phe
        195                 200                 205

Phe Val Pro Cys Leu Ile Met Ile Ser Leu Tyr Leu Lys Ile Phe Val
    210                 215                 220

Val Ala Thr Arg Gln Ala Gln Gln Ile Arg Thr Leu Ser Gln Ser Leu
225                 230                 235                 240

Ala Gly Ala Val Lys Arg Glu Arg Lys Ala Ala Lys Thr Leu Gly Ile
                245                 250                 255

Ala Val Gly Ile Tyr Leu Val Cys Trp Leu Pro Phe Thr Val Asp Thr
            260                 265                 270

Leu Val Asp Ser Leu Leu Asn Phe Ile Thr Pro Pro Leu Val Phe Asp
        275                 280                 285

Ile Phe Ile Trp Phe Ala Tyr Phe Asn Ser Ala Cys Asn Pro Ile Ile
    290                 295                 300

Tyr Val Phe Ser Tyr Arg Trp Phe Arg Lys Ala Leu Lys Leu Leu Leu
305                 310                 315                 320

Ser Arg Glu Ile Phe Ser Pro Arg Thr Pro Thr Val Asp Leu Tyr His
                325                 330                 335

Asp

<210>  12
<211>  1038
<212>  DNA
<213>  Mus musculus

<400>  12
atgggcagta actcgtctcc gcccaccgtc ctgcagctct gctatgagaa tgtgaccggg      60

tcttgtgtga aaacccccta ctctcctggg tcgagggtca tcctctatgc agtctttggc     120

tttggggctg tgctggcagt gtttggaaac ctcatggtga tgatatccat cctccatttc     180

aagcagctgc actctcccac caatttcctc attgcatcct tggcctgtgc tgactttggt     240

gtgggcatct ctgtgatgcc cttcagcatg gtcaggtcca tcgagagctg ctggtacttt     300

ggtagaagtt tctgcacttt ccacacctgc tgtgatgtgg cgttctgtta ctcttctctc     360

ttccacctga gcttcatctc catcgacagg tacatcgctg tcacggaccc tctggtctat     420

cccaccaagt tcacggtgtc tgtgtctgga atttgcatcg gcgtctcctg gattctgccc     480

ctggtgtaca gcggcgctgt gttctacaca ggggtttatg atgacggcct ggaggaatta     540

tccagtgccc tcaactgcgt agggggctgt caggtagttg tgaatcagaa ctgggtgctg     600

atagattttc tttccttctt aatacctaca cttgttatga taattctgta tggtaatatt     660

tttctggtag ctagacaaca ggctaaaaag atagaaaata ttgggagcaa aacagaatca     720

tcctctgaga gctacaaagc cagagtggcg agaagagaaa gaaaagcagc caaaaccctg     780

gggattaccg tggtagcatt catgatatca tggttgccct acagcattga ttcattagtt 840

gatgctttta tgggcttcat cacccctgcc tacatttatg aaatctgtgt ttggtgtgct 900

tattacaact cagccatgaa ccctctgatc tacgctttat tttatccatg gtttaagaaa 960

gctattaagg ttattatgag tggccaggtg ttcaagaaca gctcagccac tatgaacttg 1020

ttctctgagc aaatataa 1038

<210> 13
<211> 345
<212> PRT
<213> Mus musculus

<400> 13

Met Gly Ser Asn Ser Ser Pro Pro Thr Val Leu Gln Leu Cys Tyr Glu
1               5                   10                  15

Asn Val Thr Gly Ser Cys Val Lys Thr Pro Tyr Ser Pro Gly Ser Arg
            20                  25                  30

Val Ile Leu Tyr Ala Val Phe Gly Phe Gly Ala Val Leu Ala Val Phe
        35                  40                  45

Gly Asn Leu Met Val Met Ile Ser Ile Leu His Phe Lys Gln Leu His
    50                  55                  60

Ser Pro Thr Asn Phe Leu Ile Ala Ser Leu Ala Cys Ala Asp Phe Gly
65                  70                  75                  80

Val Gly Ile Ser Val Met Pro Phe Ser Met Val Arg Ser Ile Glu Ser
                85                  90                  95

Cys Trp Tyr Phe Gly Arg Ser Phe Cys Thr Phe His Thr Cys Cys Asp
            100                 105                 110

Val Ala Phe Cys Tyr Ser Ser Leu Phe His Leu Ser Phe Ile Ser Ile
        115                 120                 125

Asp Arg Tyr Ile Ala Val Thr Asp Pro Leu Val Tyr Pro Thr Lys Phe
    130                 135                 140

Thr Val Ser Val Ser Gly Ile Cys Ile Gly Val Ser Trp Ile Leu Pro
145                 150                 155                 160

Leu Val Tyr Ser Gly Ala Val Phe Tyr Thr Gly Val Tyr Asp Asp Gly
                165                 170                 175

Leu Glu Glu Leu Ser Ser Ala Leu Asn Cys Val Gly Gly Cys Gln Val

180                 185                 190

Val Val Asn Gln Asn Trp Val Leu Ile Asp Phe Leu Ser Phe Leu Ile
        195                 200                 205

Pro Thr Leu Val Met Ile Ile Leu Tyr Gly Asn Ile Phe Leu Val Ala
    210                 215                 220

Arg Gln Gln Ala Lys Lys Ile Glu Asn Ile Gly Ser Lys Thr Glu Ser
225                 230                 235                 240

Ser Ser Glu Ser Tyr Lys Ala Arg Val Ala Arg Arg Glu Arg Lys Ala
                245                 250                 255

Ala Lys Thr Leu Gly Ile Thr Val Val Ala Phe Met Ile Ser Trp Leu
            260                 265                 270

Pro Tyr Ser Ile Asp Ser Leu Val Asp Ala Phe Met Gly Phe Ile Thr
        275                 280                 285

Pro Ala Tyr Ile Tyr Glu Ile Cys Val Trp Cys Ala Tyr Tyr Asn Ser
    290                 295                 300

Ala Met Asn Pro Leu Ile Tyr Ala Leu Phe Tyr Pro Trp Phe Lys Lys
305                 310                 315                 320

Ala Ile Lys Val Ile Met Ser Gly Gln Val Phe Lys Asn Ser Ser Ala
                325                 330                 335

Thr Met Asn Leu Phe Ser Glu Gln Ile
            340                 345

<210>  14
<211>  1077
<212>  DNA
<213>  Mus musculus

<400>  14
atggacaaat tggttgacca ttttctcagt gatcaatcta gaaccatgaa tgaagatctg      60

ttctctgcca catctacaga gctgtgctat gagaacctga acaggtcctg tgtcaggagc     120

ccatactccc caggccctcg cctcatcctc tatgcagtct ttggctttgg ggctgcgctg     180

gctgtgtgtg gaaacctcct ggtgatgaca tcaattcttc atttcaggca gctgcactct     240

cctgccaact tcctggtggc atccctggcc tgtgctgatt tcttagtggg tctgactgtg     300

atgcccttca gcacggtgag gtctgtggag ggctgctggt actttggaga gagttattgt     360

aaattccatt cttgttttga agggtcattc tgttactctt ctatcttcca cttgtgcttc     420

atctctgttg atagatatat tgcagtcagt gaccccctga cctaccccac caggttcact     480

gcttctgttt ctggcaagtg catcaccttc tcctggctcc tgtccatcat ctacagcttt 540

tccctccttt acacaggagc caacgaagct gggctggagg atctagtgag tgtcctcacc 600

tgtgtgggtg gctgtcaaat tgcagtgaat caaagctggg tctttatcaa ctttctatta 660

tttcttatcc ccactcttgt gatgatgact gtctactcta agattttcct cattgctaaa 720

cagcaggctc agaacattga gaagatgagc aagcagactg ccagggcatc agaaagctac 780

aaggacaggg tggccaagag ggagaggaaa gcagccaaaa ccctgggcat tgcagtggct 840

gccttcctcc tttcatggct gccatacttc attgactcca tcattgatgc cttcctaggg 900

ttcatcacgc ccacgtatgt gtatgaaatc ctagtttgga tcgcttacta taactcagcc 960

atgaaccctt tgatttatgc tttcttttat ccttggtttc gaaaagccat caagctcatt 1020

gtcactggca aaatcttgag ggagaattcc tcaaccacca acttgtttcc tgagtag 1077

<210> 15
<211> 358
<212> PRT
<213> Mus musculus

<400> 15

Met Asp Lys Leu Val Asp His Phe Leu Ser Asp Gln Ser Arg Thr Met
1               5                   10                  15

Asn Glu Asp Leu Phe Ser Ala Thr Ser Thr Glu Leu Cys Tyr Glu Asn
            20                  25                  30

Leu Asn Arg Ser Cys Val Arg Ser Pro Tyr Ser Pro Gly Pro Arg Leu
        35                  40                  45

Ile Leu Tyr Ala Val Phe Gly Phe Gly Ala Ala Leu Ala Val Cys Gly
    50                  55                  60

Asn Leu Leu Val Met Thr Ser Ile Leu His Phe Arg Gln Leu His Ser
65                  70                  75                  80

Pro Ala Asn Phe Leu Val Ala Ser Leu Ala Cys Ala Asp Phe Leu Val
                85                  90                  95

Gly Leu Thr Val Met Pro Phe Ser Thr Val Arg Ser Val Glu Gly Cys
            100                 105                 110

Trp Tyr Phe Gly Glu Ser Tyr Cys Lys Phe His Ser Cys Phe Glu Gly
        115                 120                 125

Ser Phe Cys Tyr Ser Ser Ile Phe His Leu Cys Phe Ile Ser Val Asp
    130                 135                 140

Arg Tyr Ile Ala Val Ser Asp Pro Leu Thr Tyr Pro Thr Arg Phe Thr
145                 150                 155                 160

Ala Ser Val Ser Gly Lys Cys Ile Thr Phe Ser Trp Leu Leu Ser Ile
                165                 170                 175

Ile Tyr Ser Phe Ser Leu Leu Tyr Thr Gly Ala Asn Glu Ala Gly Leu
            180                 185                 190

Glu Asp Leu Val Ser Val Leu Thr Cys Val Gly Gly Cys Gln Ile Ala
        195                 200                 205

Val Asn Gln Ser Trp Val Phe Ile Asn Phe Leu Leu Phe Leu Ile Pro
    210                 215                 220

Thr Leu Val Met Met Thr Val Tyr Ser Lys Ile Phe Leu Ile Ala Lys
225                 230                 235                 240

Gln Gln Ala Gln Asn Ile Glu Lys Met Ser Lys Gln Thr Ala Arg Ala
                245                 250                 255

Ser Glu Ser Tyr Lys Asp Arg Val Ala Lys Arg Glu Arg Lys Ala Ala
            260                 265                 270

Lys Thr Leu Gly Ile Ala Val Ala Ala Phe Leu Leu Ser Trp Leu Pro
        275                 280                 285

Tyr Phe Ile Asp Ser Ile Ile Asp Ala Phe Leu Gly Phe Ile Thr Pro
    290                 295                 300

Thr Tyr Val Tyr Glu Ile Leu Val Trp Ile Ala Tyr Tyr Asn Ser Ala
305                 310                 315                 320

Met Asn Pro Leu Ile Tyr Ala Phe Phe Tyr Pro Trp Phe Arg Lys Ala
                325                 330                 335

Ile Lys Leu Ile Val Thr Gly Lys Ile Leu Arg Glu Asn Ser Ser Thr
            340                 345                 350

Thr Asn Leu Phe Pro Glu
        355

<210>  16
<211>  1077
<212>  DNA
<213>  Mus musculus

<400>  16
atggctacag ataatgacag ttttccctgg gatcaagaca gcatcctgag cagtgatatg      60

ttctctgcca catctacaga gctgtgctat gagaacctga acaggtcctg tgtcaggagc 120
ccatattccc caggccctcg cctcatcctc tatgcagtct ttggctttgg ggctgcgctg 180
gctgtgtgtg gaaacctcct ggtgatgaca tcaattcttc atttcaggca gctacactcc 240
cctgccaact tcctggtggt gtccttggcc tgtgctgatt tcttggtggg attgactgtg 300
atgcccttca gcacagtgag gtctgtggag ggatgctggt actttgggga gagttactgt 360
aaattacaca cttgttttga tgtatctttc tgctattgtt ctatcttcca cttgtgcttc 420
atctctgttg atagatatat tgcagtcagt gaccccctga cctaccccac caggtttact 480
gcatttgttt ctggcaagtg catcaccttc tcctggctcc tgtccaccat ttatgggttt 540
tctctccttt atacaggggc taatgaagct gggctggagg atctagtgag tgccctcacc 600
tgtgtgggtg gctgtcaact tgcagtgaat caaagctggg tcttcatcaa tttcctatta 660
tttcttatcc ccacccttgt gatgataact gtctactcta agattttcct cattgctaaa 720
cagcaggctc agaacattga gaagatgagc aagcagactg ccagggcatc agacagctac 780
aaggacaggg tggccaagag agagaggaaa gcagccaaaa ccctgggcat cgcagtggct 840
gccttcctcc tttcatggct gccatacttc attgactcca tcattgatgc cttcctaggg 900
ttcatcacgc ccacgtatgt gtatgaaatc ctagtttgga tcgcttacta taactcagcc 960
atgaaccctt tgatttatgc tttcttttat ccttggtttc gaaaagccat caaactcatt 1020
gtctctggca aagtcttgag ggagaattcc tcaaccacca acttgtttcc tgagtag 1077

<210> 17
<211> 358
<212> PRT
<213> Mus musculus

<400> 17

Met Ala Thr Asp Asn Asp Ser Phe Pro Trp Asp Gln Asp Ser Ile Leu
1               5                   10                  15

Ser Ser Asp Met Phe Ser Ala Thr Ser Thr Glu Leu Cys Tyr Glu Asn
            20                  25                  30

Leu Asn Arg Ser Cys Val Arg Ser Pro Tyr Ser Pro Gly Pro Arg Leu
        35                  40                  45

Ile Leu Tyr Ala Val Phe Gly Phe Gly Ala Ala Leu Ala Val Cys Gly
    50                  55                  60

Asn Leu Leu Val Met Thr Ser Ile Leu His Phe Arg Gln Leu His Ser
65                  70                  75                  80

Pro Ala Asn Phe Leu Val Val Ser Leu Ala Cys Ala Asp Phe Leu Val

                85                  90                  95

Gly Leu Thr Val Met Pro Phe Ser Thr Val Arg Ser Val Glu Gly Cys
            100                 105                 110

Trp Tyr Phe Gly Glu Ser Tyr Cys Lys Leu His Thr Cys Phe Asp Val
        115                 120                 125

Ser Phe Cys Tyr Cys Ser Ile Phe His Leu Cys Phe Ile Ser Val Asp
    130                 135                 140

Arg Tyr Ile Ala Val Ser Asp Pro Leu Thr Tyr Pro Thr Arg Phe Thr
145                 150                 155                 160

Ala Phe Val Ser Gly Lys Cys Ile Thr Phe Ser Trp Leu Leu Ser Thr
                165                 170                 175

Ile Tyr Gly Phe Ser Leu Leu Tyr Thr Gly Ala Asn Glu Ala Gly Leu
            180                 185                 190

Glu Asp Leu Val Ser Ala Leu Thr Cys Val Gly Gly Cys Gln Leu Ala
        195                 200                 205

Val Asn Gln Ser Trp Val Phe Ile Asn Phe Leu Leu Phe Leu Ile Pro
    210                 215                 220

Thr Leu Val Met Ile Thr Val Tyr Ser Lys Ile Phe Leu Ile Ala Lys
225                 230                 235                 240

Gln Gln Ala Gln Asn Ile Glu Lys Met Ser Lys Gln Thr Ala Arg Ala
                245                 250                 255

Ser Asp Ser Tyr Lys Asp Arg Val Ala Lys Arg Glu Arg Lys Ala Ala
            260                 265                 270

Lys Thr Leu Gly Ile Ala Val Ala Ala Phe Leu Leu Ser Trp Leu Pro
        275                 280                 285

Tyr Phe Ile Asp Ser Ile Ile Asp Ala Phe Leu Gly Phe Ile Thr Pro
    290                 295                 300

Thr Tyr Val Tyr Glu Ile Leu Val Trp Ile Ala Tyr Tyr Asn Ser Ala
305                 310                 315                 320

Met Asn Pro Leu Ile Tyr Ala Phe Phe Tyr Pro Trp Phe Arg Lys Ala
                325                 330                 335

Ile Lys Leu Ile Val Ser Gly Lys Val Leu Arg Glu Asn Ser Ser Thr

340                 345                 350

Thr Asn Leu Phe Pro Glu
        355

<210>  18
<211>  1055
<212>  DNA
<213>  Mus musculus

<400>  18
atgactacag gtgatgacag ttttccttgg aaccaagaca gcatcctgag cagagatctg      60

ttctctgcca catctgcaga gctgtgctat gagaacctga acaggtcctg tgtcaggagc     120

ccatactcct caggccctcg cctcatcctc tatgcagtct ttggctttgg ggctgtgctg     180

gctgtgtgtg gaaacctcct ggtgatgaca tcaattcttc atttcaggca gctgcactct     240

cctgccaact tcctggtggc atccctggcc tgtgctgact tcttggtagg ggtgatggtg     300

atgcccttta gcacagtgag gtctgtggag ggctgctggt actttgggga gatttactgt     360

aaattccatt cttgttttga agggtcattc tgttactctt ctatcttcca cttgtgtttc     420

atctctattg atagatatat tgcagtcagt gaccccctga tctaccccac caggtttact     480

gcatctgttt ctggcaagtg catcaccttc tcctgtccac catctacagc ttttccctcc     540

tttatacagg ggccaatgaa gctgggctga aggatctagt gagtgccctc acctgtgtgg     600

gtggctgtca agtcccagtg aatcaaagct gggtcttcat caacttccta ttatttctta     660

tccccaccct tgtgatgata actgtctact ctaagatttt cctcattgct aaacagcagg     720

ctcagaacat tgagaagatg agcaagcaga ctgccagggc atcagacagc tacaaggaca     780

gggtggccaa gagggagagg aaagcagcca aaacgctggg cattgcagtg gctgccttcc     840

tcctttcatg gctgccatac ttcattgact cctttattga tgccttccta gggtatgtgt     900

atgaaatcct agtttggatc gcttactata actcagccat gaaccctttg atttatgctt     960

tcttttatcc ttggtttcga aaagccatca aactcattgt cactggcaaa atcctgagag    1020

tgaattcctc aaccaccaac ctgtttcccg aatag                               1055

<210>  19
<211>  1055
<212>  DNA
<213>  Mus musculus

<400>  19
atgactacag gtgatgacag ttttccttgg aaccaagaca gcatcctgag cagagatctg      60

ttctctgcca catctgcaga gctgtgctat gagaacctga acaggtcctg tgtcaggagc     120

ccatactcct caggccctcg cctcatcctc tatgcagtct ttggctttgg ggctgtgctg     180

gctgtgtgtg gaaacctcct ggtgatgaca tcaattcttc atttcaggca gctgcactct     240

cctgccaact tcctggtggc atccctggcc tgtgctgact tcttggtagg ggtgatggtg 300
atgcccttta gcacagtgag gtctgtggag ggctgctggt actttgggga gatttactgt 360
aaattccatt cttgttttga agggtcattc tgttactctt ctatcttcca cttgtgtttc 420
atctctattg atagatatat tgcagtcagt gaccccctga tctaccccac caggtttact 480
gcatctgttt ctggcaagtg catcaccttc tcctgtccac catctacagc ttttccctcc 540
tttatacagg ggccaatgaa gctgggctga aggatctagt gagtgccctc acctgtgtgg 600
gtggctgtca agtcccagtg aatcaaagct gggtcttcat caacttccta ttatttctta 660
tccccaccct tgtgatgata actgtctact ctaagatttt cctcattgct aaacagcagg 720
ctcagaacat tgagaagatg agcaagcaga ctgccagggc atcagacagc tacaaggaca 780
gggtggccaa gagggagagg aaagcagcca aaacgctggg cattgcagtg gctgccttcc 840
tcctttcatg gctgccatac ttcattgact cctttattga tgccttccta gggtatgtgt 900
atgaaatcct agtttggatc gcttactata actcagccat gaaccctttg atttatgctt 960
tcttttatcc ttggtttcga aaagccatca aactcattgt cactggcaaa atcctgagag 1020
tgaattcctc aaccaccaac ctgtttcccg aatag 1055

<210> 20
<211> 351
<212> PRT
<213> Mus musculus

<400> 20

Met Thr Thr Gly Asp Asp Ser Phe Pro Trp Asn Gln Asp Ser Ile Leu
1               5                   10                  15

Ser Arg Asp Leu Phe Ser Ala Thr Ser Ala Glu Leu Cys Tyr Glu Asn
            20                  25                  30

Leu Asn Arg Ser Cys Val Arg Ser Pro Tyr Ser Ser Gly Pro Arg Leu
        35                  40                  45

Ile Leu Tyr Ala Val Phe Gly Phe Gly Ala Val Leu Ala Val Cys Gly
    50                  55                  60

Asn Leu Leu Val Met Thr Ser Ile Leu His Phe Arg Gln Leu His Ser
65                  70                  75                  80

Pro Ala Asn Phe Leu Val Ala Ser Leu Ala Cys Ala Asp Phe Leu Val
                85                  90                  95

Gly Val Met Val Met Pro Phe Ser Thr Val Arg Ser Val Glu Gly Cys
            100                 105                 110

Trp Tyr Phe Gly Glu Ile Tyr Cys Lys Phe His Ser Cys Phe Glu Gly
        115                 120                 125

Ser Phe Cys Tyr Ser Ser Ile Phe His Leu Cys Phe Ile Ser Ile Asp
    130                 135                 140

Arg Tyr Ile Ala Val Ser Asp Pro Leu Ile Tyr Pro Thr Arg Phe Thr
145                 150                 155                 160

Ala Ser Val Ser Gly Lys Cys Ile Thr Phe Ser Leu Ser Thr Ile Tyr
                165                 170                 175

Ser Phe Ser Leu Leu Tyr Thr Gly Ala Asn Glu Ala Gly Leu Lys Asp
            180                 185                 190

Leu Val Ser Ala Leu Thr Cys Val Gly Gly Cys Gln Val Pro Val Asn
        195                 200                 205

Gln Ser Trp Val Phe Ile Asn Phe Leu Leu Phe Leu Ile Pro Thr Leu
    210                 215                 220

Val Met Ile Thr Val Tyr Ser Lys Ile Phe Leu Ile Ala Lys Gln Gln
225                 230                 235                 240

Ala Gln Asn Ile Glu Lys Met Ser Lys Gln Thr Ala Arg Ala Ser Asp
                245                 250                 255

Ser Tyr Lys Asp Arg Val Ala Lys Arg Glu Arg Lys Ala Ala Lys Thr
            260                 265                 270

Leu Gly Ile Ala Val Ala Ala Phe Leu Leu Ser Trp Leu Pro Tyr Phe
        275                 280                 285

Ile Asp Ser Phe Ile Asp Ala Phe Leu Gly Tyr Val Tyr Glu Ile Leu
    290                 295                 300

Val Trp Ile Ala Tyr Tyr Asn Ser Ala Met Asn Pro Leu Ile Tyr Ala
305                 310                 315                 320

Phe Phe Tyr Pro Trp Phe Arg Lys Ala Ile Lys Leu Ile Val Thr Gly
                325                 330                 335

Lys Ile Leu Arg Val Asn Ser Ser Thr Thr Asn Leu Phe Pro Glu
            340                 345                 350

<210>  21
<211>  1055
<212>  DNA
<213>  Mus musculus

<400>  21
atgactacag gtgatgacag ttttccttgg aaccaagaca gcatcctgag cagagatctg      60

ttctctgcca catctgcaga gctgtgctat gagaacctga acaggtcctg tgtcaggagc     120

ccatactcct caggccctcg cctcatcctc tatgcagtct ttggctttgg ggctgtgctg     180

gctgtgtgtg gaaacctcct ggtgatgaca tcaattcttc atttcaggca gctgcactct     240

cctgccaact tcctggtggc atccctggcc tgtgctgact tcttggtagg ggtgatggtg     300

atgcccttta gcacagtgag gtctgtggag ggctgctggt actttgggga gatttactgt     360

aaattccatt cttgttttga agggtcattc tgttactctt ctatcttcca cttgtgtttc     420

atctctattg atagatatat tgcagtcagt gaccccctga tctaccccac caggtttact     480

gcatctgttt ctggcaagtg catcaccttc tcctgtccac catctacagc ttttccctcc     540

tttatacagg ggccaatgaa gctgggctga aggatctagt gagtgccctc acctgtgtgg     600

gtggctgtca agtcccagtg aatcaaagct gggtcttcat caacttccta ttatttctta     660

tccccaccct tgtgatgata actgtctact ctaagatttt cctcattgct aaacagcagg     720

ctcagaacat tgagaagatg agcaagcaga ctgccagggc atcagacagc tacaaggaca     780

gggtggccaa gagggagagg aaagcagcca aaacgctggg cattgcagtg gctgccttcc     840

tcctttcatg gctgccatac ttcattgact cctttattga tgccttccta gggtatgtgt     900

atgaaatcct agtttggatc gcttactata actcagccat gaaccctttg atttatgctt     960

tcttttatcc ttggtttcga aaagccatca aactcattgt cactggcaaa atcctgagag    1020

tgaattcctc aaccaccaac ctgtttcccg aatag                               1055

<210>  22
<211>  358
<212>  PRT
<213>  Mus musculus

<400>  22

Met Ala Thr Gly Asp Asp Ser Phe Pro Trp Asp Gln Asp Ser Ile Leu
1               5                   10                  15

Ser Arg Asp Leu Phe Ser Ala Thr Ser Thr Glu Leu Cys Tyr Glu Asn
            20                  25                  30

Leu Asn Arg Ser Cys Val Arg Ser Pro Tyr Ser Pro Gly Pro Arg Leu
        35                  40                  45

Ile Leu Tyr Ala Val Phe Gly Phe Gly Ala Val Leu Ala Val Cys Gly
    50                  55                  60

Asn Leu Leu Val Met Thr Ser Ile Leu His Phe Arg Gln Leu His Ser
65                  70                  75                  80

Pro Ala Asn Phe Leu Val Ala Ser Leu Ala Cys Ala Asp Phe Leu Val
                85                  90                  95

Gly Val Met Val Met Pro Phe Ser Met Val Arg Ser Val Glu Gly Cys
            100                 105                 110

Trp Tyr Phe Gly Glu Ser Tyr Cys Lys Phe His Ser Cys Phe Glu Gly
        115                 120                 125

Ser Phe Cys Tyr Ser Ser Leu Phe His Leu Cys Phe Ile Ser Val Asp
    130                 135                 140

Arg Tyr Ile Ala Val Ser Asp Pro Leu Thr Tyr Pro Thr Arg Phe Thr
145                 150                 155                 160

Ala Ser Val Ser Gly Lys Cys Ile Thr Phe Ser Trp Leu Leu Ser Ile
                165                 170                 175

Ile Tyr Ser Phe Ser Leu Leu Tyr Thr Gly Ala Asn Asp Ala Gly Leu
            180                 185                 190

Glu Asp Leu Val Ser Ala Leu Thr Cys Val Gly Gly Cys Gln Ile Ala
        195                 200                 205

Val Asn Gln Thr Trp Val Phe Ile Asn Phe Leu Leu Phe Leu Ile Pro
    210                 215                 220

Thr Leu Val Met Ile Thr Val Tyr Ser Lys Ile Phe Leu Ile Ala Lys
225                 230                 235                 240

Gln Gln Ala Gln Asn Ile Glu Lys Met Ser Lys Gln Thr Ala Arg Ala
                245                 250                 255

Ser Glu Ser Tyr Lys Asp Arg Val Thr Lys Arg Glu Arg Lys Ala Ala
            260                 265                 270

Lys Thr Leu Gly Ile Ala Val Ala Ala Phe Leu Leu Ser Trp Leu Pro
        275                 280                 285

Tyr Phe Ile Asp Ser Ile Ile Asp Ala Phe Leu Gly Phe Ile Thr Pro
    290                 295                 300

Thr Tyr Val Tyr Glu Ile Leu Val Trp Ile Val Tyr Tyr Asn Ser Ala
305                 310                 315                 320

Met Asn Pro Leu Ile Tyr Ala Phe Phe Tyr Ser Trp Phe Arg Lys Ala
                325                 330                 335

Ile Lys Leu Ile Val Ser Gly Lys Ile Leu Arg Glu Asn Ser Ser Thr
            340                 345                 350

Thr Asn Leu Phe Pro Glu
        355

<210>  23
<211>  1077
<212>  DNA
<213>  Mus musculus

<400>  23
atggctacag gtgatgacag ttttctctgg gaccaagaca gcatcctgag cagagatctg      60

ttctctgcca catctgcaga gctgtgctat gagaacctga acaggtcctg tgtcagaagc     120

ccatactccc caggccctcg cctcatcctc tatgcagtct ttggctttgg ggctgtgctg     180

gctgtgtgtg gaaacctcct ggtgatgaca tcaattcttc atttcaggca gctgcactct     240

cctgccaact tcctggtggc atccctggcc tgtgctgact tcttggtggg tctgactgtg     300

atgcccttca gcacagtgag gtctgtggag ggctgctggt actttgggga gatttactgt     360

aaattacaca cttgttttga tgtatctttc tgtagttctt ctattttcca cttgtgcttc     420

atctctgttg atagatatat tgcagtcagt gaccccctga tctaccccac caggtttact     480

gcatctgttt ctaacaagtg catcactttc tcctggcttc tgtccatcag ctatgggttt     540

tcccttattt acacaggagc aagtgaagct gggctagagg atctagtgag tgccctcacc     600

tgtgtgggtg gctgtcaact tgcagtgaat caaagctggg tctttatcaa ctttctatta     660

tttcttatcc ccacccttgt gatgataact gtctactcta agattttcct cattgctaaa     720

cagcaggctc agaacattga gaagatgagc aagcagactg ccagggcatc agacagctac     780

aaggacaggg tggccaagag ggagaggaaa gcagccaaaa ccctgggcat cgcagtggct     840

gccttcctcc tttcatggct gccatacttc attgactcct tcattgatgc cttcctgggg     900

ttcatcacgc ccacgtatgt gtatgaaatc ctagtttgga tcgcttacta taactcagcc     960

atgaaccctt tgatttatgc tttcttttat ccttggtttc gaaaagccat caaactcact    1020

gttactggca aaatcttgag ggaaaattcc tcaaccacca acctgtttcc tgagtag       1077

<210>  24
<211>  358
<212>  PRT
<213>  Mus musculus

<400>  24

Met Ala Thr Gly Asp Asp Ser Phe Leu Trp Asp Gln Asp Ser Ile Leu
1               5                   10                  15

Ser Arg Asp Leu Phe Ser Ala Thr Ser Ala Glu Leu Cys Tyr Glu Asn
            20                  25                  30

Leu Asn Arg Ser Cys Val Arg Ser Pro Tyr Ser Pro Gly Pro Arg Leu
        35                  40                  45

Ile Leu Tyr Ala Val Phe Gly Phe Gly Ala Val Leu Ala Val Cys Gly
    50                  55                  60

Asn Leu Leu Val Met Thr Ser Ile Leu His Phe Arg Gln Leu His Ser
65                  70                  75                  80

Pro Ala Asn Phe Leu Val Ala Ser Leu Ala Cys Ala Asp Phe Leu Val
                85                  90                  95

Gly Leu Thr Val Met Pro Phe Ser Thr Val Arg Ser Val Glu Gly Cys
            100                 105                 110

Trp Tyr Phe Gly Glu Ile Tyr Cys Lys Leu His Thr Cys Phe Asp Val
        115                 120                 125

Ser Phe Cys Ser Ser Ser Ile Phe His Leu Cys Phe Ile Ser Val Asp
    130                 135                 140

Arg Tyr Ile Ala Val Ser Asp Pro Leu Ile Tyr Pro Thr Arg Phe Thr
145                 150                 155                 160

Ala Ser Val Ser Asn Lys Cys Ile Thr Phe Ser Trp Leu Leu Ser Ile
                165                 170                 175

Ser Tyr Gly Phe Ser Leu Ile Tyr Thr Gly Ala Ser Glu Ala Gly Leu
            180                 185                 190

Glu Asp Leu Val Ser Ala Leu Thr Cys Val Gly Gly Cys Gln Leu Ala
        195                 200                 205

Val Asn Gln Ser Trp Val Phe Ile Asn Phe Leu Leu Phe Leu Ile Pro
    210                 215                 220

Thr Leu Val Met Ile Thr Val Tyr Ser Lys Ile Phe Leu Ile Ala Lys
225                 230                 235                 240

Gln Gln Ala Gln Asn Ile Glu Lys Met Ser Lys Gln Thr Ala Arg Ala
                245                 250                 255

Ser Asp Ser Tyr Lys Asp Arg Val Ala Lys Arg Glu Arg Lys Ala Ala
            260                 265                 270

Lys Thr Leu Gly Ile Ala Val Ala Ala Phe Leu Leu Ser Trp Leu Pro
        275                 280                 285

Tyr Phe Ile Asp Ser Phe Ile Asp Ala Phe Leu Gly Phe Ile Thr Pro
    290                 295                 300

Thr Tyr Val Tyr Glu Ile Leu Val Trp Ile Ala Tyr Tyr Asn Ser Ala
305                 310                 315                 320

Met Asn Pro Leu Ile Tyr Ala Phe Phe Tyr Pro Trp Phe Arg Lys Ala
                325                 330                 335

Ile Lys Leu Thr Val Thr Gly Lys Ile Leu Arg Glu Asn Ser Ser Thr
            340                 345                 350

Thr Asn Leu Phe Pro Glu
        355

<210> 25
<211> 1077
<212> DNA
<213> Mus musculus

<400> 25
atgtctatag ctgatgaaac tgtttcctgg aaccaagaca gcatcctgag cagagatctg 60

ttctctgcca catctgcaga gctgtgctat gagaacctga acaggtcctg tgtcagaagc 120

ccatactccc caggccctcg cctcatcctc tatgcagtct ttggctttgg ggctgtgctg 180

gctgtgtgtg gaaacctcct ggtgatgaca tcaattcttc atttcaggca gctgcactct 240

cctgccaact tcctggtggc atccctggcc tgtgctgact tcttggtagg ggtgatggtg 300

atgcccttta gcatggtgag gtctgtggag ggctgctggt actttggaga cagttactgt 360

aaattacaca cttgttttga tgtatctttc tgctattgtt ctctcttcca cttgtgcttc 420

atctctgttg atagatatat tgcagtcagt gaccccctgg cctaccccac caggtttact 480

gcatctgttt ctggcaagtg catcaccttc tcctggctcc tgtccatcag ctatgggttt 540

tccctcattt acacaggagc aagtgaagct gggctggagg atctagtgag ttccctcacc 600

tgtgtgggtg gctgtcaaat cgcagtgaat caaacctggg tctttatcaa cttctcagta 660

tttctcattc ccacccttgt gatgataact gtctactcta agattttcct cattgctaaa 720

cagcaggctc agaacattga gaagatgagc aagcagactg ccagggcatc agacagctac 780

aaggacaggg tggccaagag ggagaggaaa gcagccaaaa ccctgggcat cgcagtggct 840

gccttcctcc tttcatggct gccatacttc attgactcct ttattgatgc cttcctaggg 900

ttcatcacgc ccacgtatgt gtatgaaatc ctagtttgga ttgtttacta taactcagcc 960

atgaaccctt tgatttatgc tttcttttat ccttggtttc gaaaagccat caaactcact 1020

gttactggca aaatcttgag ggaaaattcc tcaaccacca atctgttttc tgagtag     1077

<210>  26
<211>  358
<212>  PRT
<213>  Mus musculus

<400>  26

Met Ser Ile Ala Asp Glu Thr Val Ser Trp Asn Gln Asp Ser Ile Leu
1               5                   10                  15

Ser Arg Asp Leu Phe Ser Ala Thr Ser Ala Glu Leu Cys Tyr Glu Asn
            20                  25                  30

Leu Asn Arg Ser Cys Val Arg Ser Pro Tyr Ser Pro Gly Pro Arg Leu
        35                  40                  45

Ile Leu Tyr Ala Val Phe Gly Phe Gly Ala Val Leu Ala Val Cys Gly
    50                  55                  60

Asn Leu Leu Val Met Thr Ser Ile Leu His Phe Arg Gln Leu His Ser
65                  70                  75                  80

Pro Ala Asn Phe Leu Val Ala Ser Leu Ala Cys Ala Asp Phe Leu Val
                85                  90                  95

Gly Val Met Val Met Pro Phe Ser Met Val Arg Ser Val Glu Gly Cys
            100                 105                 110

Trp Tyr Phe Gly Asp Ser Tyr Cys Lys Leu His Thr Cys Phe Asp Val
        115                 120                 125

Ser Phe Cys Tyr Cys Ser Leu Phe His Leu Cys Phe Ile Ser Val Asp
    130                 135                 140

Arg Tyr Ile Ala Val Ser Asp Pro Leu Ala Tyr Pro Thr Arg Phe Thr
145                 150                 155                 160

Ala Ser Val Ser Gly Lys Cys Ile Thr Phe Ser Trp Leu Leu Ser Ile
                165                 170                 175

Ser Tyr Gly Phe Ser Leu Ile Tyr Thr Gly Ala Ser Glu Ala Gly Leu
            180                 185                 190

Glu Asp Leu Val Ser Ser Leu Thr Cys Val Gly Gly Cys Gln Ile Ala
        195                 200                 205

Val Asn Gln Thr Trp Val Phe Ile Asn Phe Ser Val Phe Leu Ile Pro
    210                 215                 220

Thr Leu Val Met Ile Thr Val Tyr Ser Lys Ile Phe Leu Ile Ala Lys
225                 230                 235                 240

Gln Gln Ala Gln Asn Ile Glu Lys Met Ser Lys Gln Thr Ala Arg Ala
                245                 250                 255

Ser Asp Ser Tyr Lys Asp Arg Val Ala Lys Arg Glu Arg Lys Ala Ala
            260                 265                 270

Lys Thr Leu Gly Ile Ala Val Ala Ala Phe Leu Leu Ser Trp Leu Pro
        275                 280                 285

Tyr Phe Ile Asp Ser Phe Ile Asp Ala Phe Leu Gly Phe Ile Thr Pro
    290                 295                 300

Thr Tyr Val Tyr Glu Ile Leu Val Trp Ile Val Tyr Tyr Asn Ser Ala
305                 310                 315                 320

Met Asn Pro Leu Ile Tyr Ala Phe Phe Tyr Pro Trp Phe Arg Lys Ala
                325                 330                 335

Ile Lys Leu Thr Val Thr Gly Lys Ile Leu Arg Glu Asn Ser Ser Thr
            340                 345                 350

Thr Asn Leu Phe Ser Glu
        355

<210>  27
<211>  1035
<212>  DNA
<213>  Mus musculus

<400>  27
atgaccagca acttttccca accagccctg cagctctgct atgagaacac gaatggatcc      60

tgtattaaaa ctccctattc tccagggccc cgggtcatcc tgtacatggt ctatggcttt     120

ggggctgtgc tggcagtgtg tgggaacctc ctggtggtga tttcagttct ccatttcaag     180

cagctgcact ctccagccaa ttttctcatc gcctctctgg ccagtgctga cttcttggtg     240

ggcatctctg tgatgccctt cagcatggtc aggtccattg agagctgctg gtactttgga     300

gatgcatttt gtagccttca cagttgttgt gatgtggctt tttgttactc ttctgtcctc     360

cacctctgct tcatctcagt ggacaggtac atcgctgtca cggaccctct ggtctatccc     420

accaagttca cagtgtctgt gtctggaatt tgcatcagca tctcctggat tctgcccctg     480

gtgtacagca gtgcagtgtt ttacacaggc atcagtgcta aggggattga aagcttagta     540

agtgctctaa attgtgtagg gggctgccaa attgttatca atcaagactt tgttttgata     600

agttttcttc tattcttcat acctaccctt gttatgatca ttctttacag caaaatattt 660

ttggtagcca aacagcaagc tgtaaaaatt gaaacttctg taagtggcaa cagaggtgaa 720

tcatcctcag agagtcacaa agccagagtg gccaagagag agaggaaggc tgcaaaaacc 780

ttgggggtca ctgtggtggc ttttatggtc tcgtggctcc catacacaat tgacgcattg 840

gttgatgctt tcatgggctt catcactcct gcctatgtct atgaaatctg ttgctggggt 900

acctattata actcagccat gaaccctttg atttatgctt tcttttttcc ttggtttaag 960

aaagccataa agcttatttt aagtggggaa attctaaagg gacattcatc aactgcaaat 1020

ttattttcag agtaa 1035

<210> 28
<211> 344
<212> PRT
<213> Mus musculus

<400> 28

Met Thr Ser Asn Phe Ser Gln Pro Ala Leu Gln Leu Cys Tyr Glu Asn
1               5                   10                  15

Thr Asn Gly Ser Cys Ile Lys Thr Pro Tyr Ser Pro Gly Pro Arg Val
            20                  25                  30

Ile Leu Tyr Met Val Tyr Gly Phe Gly Ala Val Leu Ala Val Cys Gly
        35                  40                  45

Asn Leu Leu Val Val Ile Ser Val Leu His Phe Lys Gln Leu His Ser
    50                  55                  60

Pro Ala Asn Phe Leu Ile Ala Ser Leu Ala Ser Ala Asp Phe Leu Val
65                  70                  75                  80

Gly Ile Ser Val Met Pro Phe Ser Met Val Arg Ser Ile Glu Ser Cys
                85                  90                  95

Trp Tyr Phe Gly Asp Ala Phe Cys Ser Leu His Ser Cys Cys Asp Val
            100                 105                 110

Ala Phe Cys Tyr Ser Ser Val Leu His Leu Cys Phe Ile Ser Val Asp
        115                 120                 125

Arg Tyr Ile Ala Val Thr Asp Pro Leu Val Tyr Pro Thr Lys Phe Thr
    130                 135                 140

Val Ser Val Ser Gly Ile Cys Ile Ser Ile Ser Trp Ile Leu Pro Leu
145                 150                 155                 160

Val Tyr Ser Ser Ala Val Phe Tyr Thr Gly Ile Ser Ala Lys Gly Ile
                165                 170                 175

Glu Ser Leu Val Ser Ala Leu Asn Cys Val Gly Gly Cys Gln Ile Val
            180                 185                 190

Ile Asn Gln Asp Phe Val Leu Ile Ser Phe Leu Leu Phe Phe Ile Pro
        195                 200                 205

Thr Leu Val Met Ile Ile Leu Tyr Ser Lys Ile Phe Leu Val Ala Lys
    210                 215                 220

Gln Gln Ala Val Lys Ile Glu Thr Ser Val Ser Gly Asn Arg Gly Glu
225                 230                 235                 240

Ser Ser Ser Glu Ser His Lys Ala Arg Val Ala Lys Arg Glu Arg Lys
                245                 250                 255

Ala Ala Lys Thr Leu Gly Val Thr Val Val Ala Phe Met Val Ser Trp
            260                 265                 270

Leu Pro Tyr Thr Ile Asp Ala Leu Val Asp Ala Phe Met Gly Phe Ile
        275                 280                 285

Thr Pro Ala Tyr Val Tyr Glu Ile Cys Cys Trp Gly Thr Tyr Tyr Asn
    290                 295                 300

Ser Ala Met Asn Pro Leu Ile Tyr Ala Phe Phe Phe Pro Trp Phe Lys
305                 310                 315                 320

Lys Ala Ile Lys Leu Ile Leu Ser Gly Glu Ile Leu Lys Gly His Ser
                325                 330                 335

Ser Thr Ala Asn Leu Phe Ser Glu
            340

<210>  29
<211>  1035
<212>  DNA
<213>  Mus musculus

<400>  29
atgaccagca acttttccca accagccctg cagctctgct atgagaacac gaatggatcc      60

tgtattaaaa ctccctattc tccagggccc cgggtcatcc tgtacatggt ctttggcttt     120

ggggctgtgc tggcagtgtg tgggaacctc ctggtggtga tttcagttct ccatttcaag     180

cagctgcact ctccagccaa ttttctcatc gcctctctgg ccagcgctga cttcttggtg     240

ggcatctctg tgatgccctt cagcatggtc aggtccattg agagctgctg gtactttgga     300

gatgcatttt gtagccttca cagttgctgt gatgtggctt tttgttactc ttctgccctc 360
cacctgtgct tcatctcagt ggacaggtac attgctgtca cagaccctct ggtctatccc 420
accaagttca cagtgtctgt gtctggaatt tgcatcagca tctcctggat tctgcccctg 480
gtatacagca gtgcagtgtt ctacacaggc atcagtgcta aggggattga aagcttagta 540
agtgctctga attgtgtagg gggctgccaa atcgttgtca atcaagactg ggttttgata 600
gattttcttc tatttttcat acctaccctt gttatgatca ttctttacag caaaatattt 660
ttggtagcca aacagcaagc tgtaaaaatt gaaacttctg taagtgacaa cagaggtgaa 720
tcatcctcag agagtcacaa agccagagtg gccaagagag agaggaaggc tgcaaaaacc 780
ttgggggtca ctgtggtggc ttttatggtc tcgtggctcc catacacaat tgactcattg 840
gttgatgctt tcgtgggctt cattactcct gcctatgtct atgaaatctg ttgctggagt 900
gcctattata actcagccat gaaccctttg atttatgctt tcttttatcc ttggtttagg 960
aaagccataa agcttatttt aagtggggag attctaaaga gtcattcatc taccatgagt 1020
ttgttttcag agtaa 1035

<210> 30
<211> 344
<212> PRT
<213> Mus musculus

<400> 30

Met Thr Ser Asn Phe Ser Gln Pro Ala Leu Gln Leu Cys Tyr Glu Asn
1               5                   10                  15

Thr Asn Gly Ser Cys Ile Lys Thr Pro Tyr Ser Pro Gly Pro Arg Val
            20                  25                  30

Ile Leu Tyr Met Val Phe Gly Phe Gly Ala Val Leu Ala Val Cys Gly
        35                  40                  45

Asn Leu Leu Val Val Ile Ser Val Leu His Phe Lys Gln Leu His Ser
    50                  55                  60

Pro Ala Asn Phe Leu Ile Ala Ser Leu Ala Ser Ala Asp Phe Leu Val
65                  70                  75                  80

Gly Ile Ser Val Met Pro Phe Ser Met Val Arg Ser Ile Glu Ser Cys
                85                  90                  95

Trp Tyr Phe Gly Asp Ala Phe Cys Ser Leu His Ser Cys Cys Asp Val
            100                 105                 110

Ala Phe Cys Tyr Ser Ser Ala Leu His Leu Cys Phe Ile Ser Val Asp
        115                 120                 125

Arg Tyr Ile Ala Val Thr Asp Pro Leu Val Tyr Pro Thr Lys Phe Thr
    130                 135                 140

Val Ser Val Ser Gly Ile Cys Ile Ser Ile Ser Trp Ile Leu Pro Leu
145                 150                 155                 160

Val Tyr Ser Ser Ala Val Phe Tyr Thr Gly Ile Ser Ala Lys Gly Ile
                165                 170                 175

Glu Ser Leu Val Ser Ala Leu Asn Cys Val Gly Gly Cys Gln Ile Val
            180                 185                 190

Val Asn Gln Asp Trp Val Leu Ile Asp Phe Leu Leu Phe Phe Ile Pro
        195                 200                 205

Thr Leu Val Met Ile Ile Leu Tyr Ser Lys Ile Phe Leu Val Ala Lys
    210                 215                 220

Gln Gln Ala Val Lys Ile Glu Thr Ser Val Ser Asp Asn Arg Gly Glu
225                 230                 235                 240

Ser Ser Ser Glu Ser His Lys Ala Arg Val Ala Lys Arg Glu Arg Lys
                245                 250                 255

Ala Ala Lys Thr Leu Gly Val Thr Val Val Ala Phe Met Val Ser Trp
            260                 265                 270

Leu Pro Tyr Thr Ile Asp Ser Leu Val Asp Ala Phe Val Gly Phe Ile
        275                 280                 285

Thr Pro Ala Tyr Val Tyr Glu Ile Cys Cys Trp Ser Ala Tyr Tyr Asn
    290                 295                 300

Ser Ala Met Asn Pro Leu Ile Tyr Ala Phe Phe Tyr Pro Trp Phe Arg
305                 310                 315                 320

Lys Ala Ile Lys Leu Ile Leu Ser Gly Glu Ile Leu Lys Ser His Ser
                325                 330                 335

Ser Thr Met Ser Leu Phe Ser Glu
            340

<210>  31
<211>  1035
<212>  DNA
<213>  Mus musculus

<400>  31

atgaccagca acttttccca accagccctg cagctctgct atgagaacac gaatggatcc 60

tgtattaaaa ctccctattc tccagggccc cgggtcatcc tgtacatggt ctatggcttt 120

ggggctgtgc tggcagtgtg tgggaacctc ctggtggtga tttcagttct ccatttcaag 180

cagctgcact ctccagccaa ttttctcatc gcctctctgg ccagtgctga cttcttggtg 240

ggcatctctg tgatgccctt cagcatggtc aggtccattg agagctgctg gtactttgga 300

gatgcatttt gtagccttca cagttgctgt gatgtggctt tttgttactc ttctgccctc 360

cacctgtgct tcatctcagt ggacaggtac atagctgtca cggaccctct ggtctatccc 420

accaagttca cagtgtctgt gtctggaatt tgcatcagca tctcctggat tctgcccctg 480

gtatacagca gtgcagtgtt ctacacaggc atcagtgcta aggggattga aagcttagta 540

agtgctctga attgtgtagg gggctgccaa gttgttgtca atcaagactg ggttttgata 600

agtttctttt tattcttcat acctaccgtt gttatgatca ttctttacag caaaatattt 660

ttggtagcca aacagcaagc tgtaaaaatt gaaacttctg taagtggcaa cagaggtgaa 720

tcatcctcag agagtcacaa agccagagtg gccaagagag agaggaaggc tgcaaaaacc 780

ttgggggtca ctgtggtggc ttttatggtc tcgtggctcc catacacaat tgatgcattg 840

gttgatgctt tcatgggctt catcactcct gcctatgtct atgaaatctg ttgctggagt 900

gcctattata actcagccat gaaccctttg atttatgctt tcttttatcc ttggtttagg 960

aaagccataa agcttatttt aagtgggaaa attctaaagg gacattcatc aactacaaat 1020

ttattttcag agtaa 1035

<210> 32
<211> 344
<212> PRT
<213> Mus musculus

<400> 32

Met Thr Ser Asn Phe Ser Gln Pro Ala Leu Gln Leu Cys Tyr Glu Asn
1               5                   10                  15

Thr Asn Gly Ser Cys Ile Lys Thr Pro Tyr Ser Pro Gly Pro Arg Val
            20                  25                  30

Ile Leu Tyr Met Val Tyr Gly Phe Gly Ala Val Leu Ala Val Cys Gly
        35                  40                  45

Asn Leu Leu Val Val Ile Ser Val Leu His Phe Lys Gln Leu His Ser
    50                  55                  60

Pro Ala Asn Phe Leu Ile Ala Ser Leu Ala Ser Ala Asp Phe Leu Val
65                  70                  75                  80

Gly Ile Ser Val Met Pro Phe Ser Met Val Arg Ser Ile Glu Ser Cys
                85                  90                  95

Trp Tyr Phe Gly Asp Ala Phe Cys Ser Leu His Ser Cys Cys Asp Val
            100                 105                 110

Ala Phe Cys Tyr Ser Ser Ala Leu His Leu Cys Phe Ile Ser Val Asp
        115                 120                 125

Arg Tyr Ile Ala Val Thr Asp Pro Leu Val Tyr Pro Thr Lys Phe Thr
    130                 135                 140

Val Ser Val Ser Gly Ile Cys Ile Ser Ile Ser Trp Ile Leu Pro Leu
145                 150                 155                 160

Val Tyr Ser Ser Ala Val Phe Tyr Thr Gly Ile Ser Ala Lys Gly Ile
                165                 170                 175

Glu Ser Leu Val Ser Ala Leu Asn Cys Val Gly Gly Cys Gln Val Val
            180                 185                 190

Val Asn Gln Asp Trp Val Leu Ile Ser Phe Leu Leu Phe Phe Ile Pro
        195                 200                 205

Thr Val Val Met Ile Ile Leu Tyr Ser Lys Ile Phe Leu Val Ala Lys
    210                 215                 220

Gln Gln Ala Val Lys Ile Glu Thr Ser Val Ser Gly Asn Arg Gly Glu
225                 230                 235                 240

Ser Ser Ser Glu Ser His Lys Ala Arg Val Ala Lys Arg Glu Arg Lys
                245                 250                 255

Ala Ala Lys Thr Leu Gly Val Thr Val Val Ala Phe Met Val Ser Trp
            260                 265                 270

Leu Pro Tyr Thr Ile Asp Ala Leu Val Asp Ala Phe Met Gly Phe Ile
        275                 280                 285

Thr Pro Ala Tyr Val Tyr Glu Ile Cys Cys Trp Ser Ala Tyr Tyr Asn
    290                 295                 300

Ser Ala Met Asn Pro Leu Ile Tyr Ala Phe Phe Tyr Pro Trp Phe Arg
305                 310                 315                 320

Lys Ala Ile Lys Leu Ile Leu Ser Gly Lys Ile Leu Lys Gly His Ser
                325                 330                 335

Ser Thr Thr Asn Leu Phe Ser Glu
            340

<210>  33
<211>  1047
<212>  DNA
<213>  Mus musculus

<400>  33
atgacaagcg acttctcccc agaaccgccc atggagctct gctatgagaa tgtgaacgga      60

tcctgcatta aaagctctta cgcaccctgg cctcgagcca tcctctatgg ggtcctcggt     120

ttgggagccc tgctggcagt gtttgggaac ttactggtca tcattgccat cctccacttt     180

aaacagctgc acacgcctac gaacttcctg gtggcctccc tggcctgtgc tgacttcttg     240

gtgggggtga cagtgatgcc cttcagcacg gtgcgttctg tggagagctg ctggtacttt     300

ggggagagtt actgtaagtt ccacacgtgt ttcgacacct ccttctgttt tgcgtctctg     360

tttcatttat gctgcatctc cattgaccgg tacattgcag ttacagatcc actgacctat     420

ccgaccaagt tcactgtatc ggtttctgga ctgtgcattg ctctctcttg gttcttttct     480

gtcacataca gcttttccat cttttacacg ggagccaatg aggaagggat tgaggaatta     540

gtggttgctc tgacctgtgt gggaggctgc caggctccac tgaatcaaaa ttgggtttta     600

ctttgtttcc ttttgttctt tctgcccact gtggtcatgg tgtttctata tggtcggata     660

tttttggtgg caaagtatca ggctaggaag atagagggta cagccaacca agctcaggcc     720

tcctctgaga gctataagga aagagtagcc aaaagagaga ggaaggctgc caagaccttg     780

gggatcgcca tggctgcatt tctcgtgtcc tggctgccat acattattga tgcggttatt     840

gacgcttaca tgaacttcat aactcctgcg tacgtctatg agatattagt gtggtgtgtt     900

tactacaatt cagctatgaa ccccttgata tacgcctttt tttatccttg gtttcgtaag     960

gcaataaaac ttattgtgag tggcaaagtc ttcagggctg attcatcaac aactaatcta    1020

ttctctgaag aggcaggtgc aggttaa                                        1047

<210>  34
<211>  348
<212>  PRT
<213>  Mus musculus

<400>  34

Met Thr Ser Asp Phe Ser Pro Glu Pro Pro Met Glu Leu Cys Tyr Glu
1               5                   10                  15

Asn Val Asn Gly Ser Cys Ile Lys Ser Ser Tyr Ala Pro Trp Pro Arg
            20                  25                  30

Ala Ile Leu Tyr Gly Val Leu Gly Leu Gly Ala Leu Leu Ala Val Phe
        35                  40                  45

Gly Asn Leu Leu Val Ile Ile Ala Ile Leu His Phe Lys Gln Leu His
    50                  55                  60

Thr Pro Thr Asn Phe Leu Val Ala Ser Leu Ala Cys Ala Asp Phe Leu
65                  70                  75                  80

Val Gly Val Thr Val Met Pro Phe Ser Thr Val Arg Ser Val Glu Ser
                85                  90                  95

Cys Trp Tyr Phe Gly Glu Ser Tyr Cys Lys Phe His Thr Cys Phe Asp
            100                 105                 110

Thr Ser Phe Cys Phe Ala Ser Leu Phe His Leu Cys Cys Ile Ser Ile
        115                 120                 125

Asp Arg Tyr Ile Ala Val Thr Asp Pro Leu Thr Tyr Pro Thr Lys Phe
    130                 135                 140

Thr Val Ser Val Ser Gly Leu Cys Ile Ala Leu Ser Trp Phe Phe Ser
145                 150                 155                 160

Val Thr Tyr Ser Phe Ser Ile Phe Tyr Thr Gly Ala Asn Glu Glu Gly
                165                 170                 175

Ile Glu Glu Leu Val Val Ala Leu Thr Cys Val Gly Gly Cys Gln Ala
            180                 185                 190

Pro Leu Asn Gln Asn Trp Val Leu Leu Cys Phe Leu Leu Phe Phe Leu
        195                 200                 205

Pro Thr Val Val Met Val Phe Leu Tyr Gly Arg Ile Phe Leu Val Ala
    210                 215                 220

Lys Tyr Gln Ala Arg Lys Ile Glu Gly Thr Ala Asn Gln Ala Gln Ala
225                 230                 235                 240

Ser Ser Glu Ser Tyr Lys Glu Arg Val Ala Lys Arg Glu Arg Lys Ala
                245                 250                 255

Ala Lys Thr Leu Gly Ile Ala Met Ala Ala Phe Leu Val Ser Trp Leu
            260                 265                 270

Pro Tyr Ile Ile Asp Ala Val Ile Asp Ala Tyr Met Asn Phe Ile Thr
        275                 280                 285

Pro Ala Tyr Val Tyr Glu Ile Leu Val Trp Cys Val Tyr Tyr Asn Ser
    290                 295                 300

Ala Met Asn Pro Leu Ile Tyr Ala Phe Phe Tyr Pro Trp Phe Arg Lys
305                 310                 315                 320

Ala Ile Lys Leu Ile Val Ser Gly Lys Val Phe Arg Ala Asp Ser Ser
                325                 330                 335

Thr Thr Asn Leu Phe Ser Glu Glu Ala Gly Ala Gly
            340                 345

<210>  35
<211>  18
<212>  DNA
<213>  Mus musculus

<400>  35
atgaatacac ccgacccc                                                    18

<210>  36
<211>  19
<212>  DNA
<213>  Mus musculus

<400>  36
ctaaggatgt gcaggatgc                                                   19

<210>  37
<211>  18
<212>  DNA
<213>  Mus musculus

<400>  37
atggcatctt ttgaagcc                                                    18

<210>  38
<211>  27
<212>  DNA
<213>  Mus musculus

<400>  38
ctattctgtt tctttttgag taaacaa                                          27

<210>  39
<211>  25
<212>  DNA
<213>  Mus musculus

<400>  39
atggatctaa tatacattcc cgaag                                            25

<210>  40
<211>  22
<212>  DNA
<213>  Mus musculus

<400>  40
ttaatgtgct tcaggaaaaa ga                                                22

<210>  41
<211>  18
<212>  DNA
<213>  Mus musculus

<400>  41
atgaatacac ccgacccc                                                     18

<210>  42
<211>  19
<212>  DNA
<213>  Mus musculus

<400>  42
ctaaggatgt gcaggatgc                                                    19

<210>  43
<211>  19
<212>  DNA
<213>  Mus musculus

<400>  43
atgagagctg tcctcctcc                                                    19

<210>  44
<211>  25
<212>  DNA
<213>  Mus musculus

<400>  44
tcagtcatgg tataaatcaa cagtc                                             25

<210>  45
<211>  20
<212>  DNA
<213>  Mus musculus

<400>  45
atgggcagta actcgtctcc                                                   20

<210>  46
<211>  25
<212>  DNA
<213>  Mus musculus

<400>  46
ttatatttgc tcagagaaca agttg                                             25

<210>  47
<211>  19
<212>  DNA
<213>  Mus musculus

<400>  47

atggacaaat tggttgacc                                                19

<210>  48
<211>  22
<212>  DNA
<213>  Mus musculus

<400>  48
ctactcagga aacaagttgg tg                                             22

<210>  49
<211>  24
<212>  DNA
<213>  Mus musculus

<400>  49
tggctacaga taatgacagt tttc                                           24

<210>  50
<211>  22
<212>  DNA
<213>  Mus musculus

<400>  50
ctactcagga aacaagttgg tg                                             22

<210>  51
<211>  25
<212>  DNA
<213>  Mus musculus

<400>  51
atgactacag gtgatgacag ttttc                                          25

<210>  52
<211>  20
<212>  DNA
<213>  Mus musculus

<400>  52
ctattcggga aacaggttgg                                                20

<210>  53
<211>  20
<212>  DNA
<213>  Mus musculus

<400>  53
atggctacag gtgatgacag                                                20

<210>  54
<211>  20
<212>  DNA
<213>  Mus musculus

<400>  54
ctattcagga aacaggttgg                                                20

<210>  55
<211>  20
<212>  DNA
<213>  Mus musculus

<400>  55
atggctacag gtgatgacag                                                    20


<210>  56
<211>  20
<212>  DNA
<213>  Mus musculus

<400>  56
ctattcagga aacaggttgg                                                    20


<210>  57
<211>  26
<212>  DNA
<213>  Mus musculus

<400>  57
atgtctatag ctgatgaaac tgtttc                                             26


<210>  58
<211>  23
<212>  DNA
<213>  Mus musculus

<400>  58
ctactcagaa aacagattgg tgg                                                23


<210>  59
<211>  19
<212>  DNA
<213>  Mus musculus

<400>  59
atgaccagca acttttccc                                                     19


<210>  60
<211>  26
<212>  DNA
<213>  Mus musculus

<400>  60
ttactctgaa aataaatttg cagttg                                             26


<210>  61
<211>  19
<212>  DNA
<213>  Mus musculus

<400>  61
atgaccagca acttttccc                                                     19

<210>  62
<211>  26
<212>  DNA
<213>  Mus musculus

<400>  62
ttactctgaa aacaaactca tggtag                                          26


<210>  63
<211>  19
<212>  DNA
<213>  Mus musculus

<400>  63
atgaccagca acttttccc                                                  19


<210>  64
<211>  28
<212>  DNA
<213>  Mus musculus

<400>  64
ttactctgaa aataaatttg tagttgat                                        28


<210>  65
<211>  19
<212>  DNA
<213>  Mus musculus

<400>  65
atgacaagcg acttctccc                                                  19


<210>  66
<211>  18
<212>  DNA
<213>  Mus musculus

<400>  66
ttaacctgca cctgcctc                                                   18

## Description

The present invention provides a fingerprint sequence which is specific and selective for trace amine associated receptors (TAAR) in mouse, forming a subfamily of G protein coupled receptors. The invention also provides the mouse polypeptides identified as members of this family, nucleic acids encoding said polypeptides, and vectors, host cells and non-human animals comprising the novel family members. In addition, the invention provides methods of identifying mouse TAARs.

Trace amines (TA) are endogenous compounds structurally related to biogenic amines and are found in the mammalian nervous system in trace amounts. TAs are stored in nerve terminals and released together with classical biogenic amines. To date there is no evidence for the existence of synapses using TAs as their exclusive transmitter. Recently, receptors specifically binding trace amines were reported by Borowski et al (Trace amines: identification of a family of mammalian G protein-coupled receptors. Proc Natl Acad Sci USA (2001) 98(16):8966-71) and Bunzow et al (Amphetamine, 3,4-methylenedioxymethamphetamine, lysergic acid diethylamide, and metabolites of the catecholamine neurotransmitters are agonists of a rat trace amine receptor. Mol Pharmacol. 2001, 60(6):1181-8.). These receptors apparently represent a novel GPCR-family.

The dysregulation of trace amines were linked to various psychiatric disorders like depression (Sandler M. et al., Decreased cerebrospinal fluid concentration of free phenylacetic acid in depressive illness. Clin Chim Acta. 1979, 93(1):169-71; Davis BA and Boulton AA. The trace amines and their acidic metabolites in depression an overview. Prog Neuropsychopharmacol Biol Psychiatry. 1994, 18(1):17-45.), schizophrenia (Potkin SG et al., Phenylethylamine in paranoid chronic schizophrenia. Science. 1979, 206(4417):470-1; Sandler M and Reynolds GP. Does phenylethylamine cause schizophrenia? Lancet. 1976, 1(7950):70-1.) and bipolar disorder (Boulton AA.: Some aspects of basic psychopharmacology: the trace amines. Prog Neuropsychopharmacol Biol Psychiatry. 1982;6(4-6):563-70; Sabelli HC et al., Clinical studies on the KM/29.04.2005 phenylethylamine hypothesis of affective disorder: urine and blood phenylacetic acid and phenylalanine dietary supplements. J Clin Psychiatry. 1986 Feb;47(2):66-70.). There has also been made a link between dysregulation of trace amines and attention-deficit hyperactivity disorder (Baker et al., Phenylethylaminergic mechanisms in attention-deficit disorder. Biol Phychiatry. 1991, 29(1):15-22), Parkinson's disease (Heller B and Fischer E., Diminution of phenethylamine in the urine of Parkinson patients. Arzneimittelforschung. 1973, 23(6):884-6.), migraine (D'Andrea G. et al., Elusive amines and primary headaches: historical background and prospectives. Neurol Sci. 2003, 24 Suppl 2:S65-7; D'Andrea, G. et al., Elevated levels of circulating trace amines in primary headaches. Neurology. 2004, 62(10):1701-1705.) and eating disorders (Wolf ME and Mosnaim AD. Phenylethylamine in neuropsychiatric disorders. Gen Pharmacol. 1983, 14(4):385-90; Branchek TA and Blackburn TP. Trace amine receptors as targets for novel therapeutics: legend myth and tact. Curr. Opin Pharmacol. 2003. 3(1):90-97.) such as i.e. obesity and anorexia as suggested by the very high structural similarity between PEA and amphetamin, which is considered the strongest anorexic compound known to date. (Samanin R, and Garattini S.: Neurochemical mechanism of action of anorectic drugs. Pharmacol Toxicol. 1993 Aug;73(2):63-8; Popplewell DA et al., A behavioural and pharmacological examination of phenylethylamine-induced anorexia and hyperactivitycomparisons with amphetamine. Pharmacol Biochem Behav. 1986 Oct;25(4):711-6.).

Therefore, there is a broad interest to increase the knowledge about trace amine receptors, especially to identify further trace amine receptors. However, examination of the literature and public database entries revealed inconsistencies in the naming of the TA receptors, e.g. the human receptor GPR102 (Lee et al., Discovery and mapping of ten novel G protein-coupled receptor genes. Gene. 2001, 275(1):83-91.) is also referred to as TA5 (Borowski et al Trace amines: identification of a family of mammalian G protein-coupled receptors. Proc Natl Acad Sci USA (2001) 98(16):8966-71)), and human 5-HT4Ψ (Liu et al., A serotonin-4 receptor-like pseudogene in humans. Brain Res Mol Brain Res. 1998, 53(1-2):98-103.) has also been named TA2Ψ (Borowski et al., Trace amines: identification of a family of mammalian G protein-coupled receptors. Proc Natl Acad Sci USA (2001) 98(16):8966-71). In addition, GPR57 (Lee et al., Cloning and characterization of additional members of the G protein-coupled receptor family. Biochim Biophys Acta. 2000, 1490(3):311-23.), GPR58 (Lee et al., Cloning and characterization of additional members of the G protein-coupled receptor family. Biochim Biophys Acta. 2000, 1490(3):311-23.) and PNR (Zeng et al., Cloning of a putative human neurotransmitter receptor expressed in skeletal muscle and brain. Biochem Biophys Res Commun. 1998, 242(3):575-8.) were so far not generally recognized as TA receptor. This inconsistencies cause confusion and uncertainty. Therefore, there is a strong need for a clear definition of this receptor family.

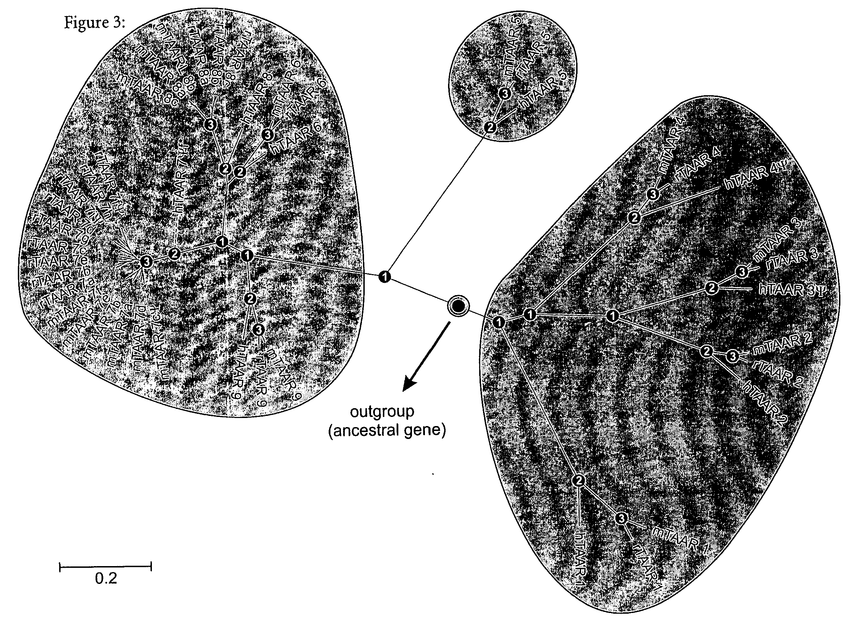

In order to resolve any ambiguity of the current naming a novel, uniform nomenclature is proposed with this invention which refers to these receptors as Trace Amine Associated Receptors (TAARs). This novel nomenclature reflects the finding that at least some TAARs do not respond to TAs at all, hence the term "associated". It covers all receptors of this GPCR family and is compatible with the different number of receptor genes in different species. The novel nomenclature is strictly based on the sequential order of the receptor genes on the respective human chromosome as well as a detailed phylogenetic analysis (Fig. 3) of the receptor genes across different species. The nomenclature adheres to the following rules:
a) Any two (or three) genes that are orthologues, *i.e*. that were generated through a speciation event, should be labeled with the same number. Vice versa, two genes must not have the same number if they are not orthologous.
b) Genes that are paralogues, *i.e*. that were generated through a gene duplication event within the lineage of one species, should be distinguished by a letter suffix.
c) Examples: Genes hTAAR 5, rTAAR 5, mTAAR 5 are all orthologues.
   Genes mTAAR 8a,b,c in mouse are paralogues.
   Genes mTAAR 8a,b,c are all orthologues to hTAAR 8.

The present invention provides novel polypeptides identified as TAAR family members, the use of said polypeptides as drug target, the polynucleotide sequences encoding said polypeptide, and vectors, host cells and non-human animals comprising said polynucleotides. Furthermore, the present invention pertains the fingerprint motif specific and selective for the TAAR family and the use of it.

The present invention provides fingerprint motif comprising the sequence NSXXNPXXZXXXBXWF (SEQ. ID NO: 1), wherein X is any natural occurring amino acid and Z may be a tyrosine or histidine and B may be a tyrosine or phenylalanine. The term "fingerprint", "fingerprint motif" or "fingerprint sequence" as used herein relates to an amino acid sequence which is specific and selective for the mouse GPCR subfamily TAAR. Preferably, the fingerprint motif is specific for functional TAARs. The tryptophan residue in the context of the fingerprint motif is found exclusively in TAARs and not in any other known GPCR; rather, the corresponding sequence position is almost invariably occupied by polar or even charged amino acids in other GPCRs. The fingerprint motif may be used for identifying TAARs, preferably for identifying functional TAARs.

The present invention also provides a method of identifying mouse TAARs using the fingerprint sequence (SEQ. ID NO: 1). To be identified as a member of the mouse TAARs family a polypeptide may have 100% identity.

Furthermore, the present invention pertains a method of identifying TAARs of other species, preferably of mammalians, using the fingerprint sequence (SEQ. ID NO: 1). To be identified as a member of the TAARs family a polypeptide may have at least 75 % identity with the fingerprint sequence, preferably more than 87% identity, more preferably 100% identity.

The fingerprint sequence may be used as a "query sequence" to perform a search against sequence databases to, for example to identify TAAR family members. Such searches can be performed using a pattern recognition program as for example fuzzpro of EMBOSS (Rice et al., EMBOSS: the European Molecular Biology Open Software Suite. Trends in Genetics, 2000, 16(6):276-277). A search for mouse TAAR may be performed with fuzzpro, NSXXNPXX[HY]XXX[YF]XWF as the required sequence, number of mismatches = 0, database = swissprot (release 43).

The mouse TAAR family contains sixteen members (mTAAR1 to mTAAR 9) wherein one of them is a pseudogene: mTAAR7cΨ. All genes encoding the mouse TAAR family members are located in the same region of chromosome 10 (10A4). With the exception of mTAAR 2, which is encoded by two exons, the coding sequences of all TAAR genes are located in a single exon.

The term "gene" as used herein refers to any segment of DNA associated with a biological function. A gene is an ordered sequence of nucleotides located in a particular position on a particular chromosome that encodes a specific functional product.

The term "pseudogene" as used herein relates to an inactive gene which may have evolved by mutation events from an active ancestor gene. Inactive means that the gene is not translated to functional polypeptide.

The present invention provides an isolated or recombinant polypeptide comprising sequence SEQ. ID NO: 5. Said polypeptide was identified as trace amine associated receptor (TAAR) and was named mTAAR2.

The term "polypeptide sequence" (e.g., a protein, polypeptide, peptide, etc.) as used herein relates to a polymer of amino acids comprising naturally occurring amino acids.

The term "isolated" means altered " by the hand of man" form the natural state. If an "isolated" composition or substance occures in nature, it has been changed or removed from its orignial environment, or both. For example, a polynucleotide or a polypeptide naturally present in a living animal is not "isolated", but the same polynucleotide or polypeptide separated form the coexisting materials of its natural state is "isolated" as the term is employed herein.

The term "recombinant" when used with reference, e.g. to a polynucleotide or polypeptide typically indicates that the polynucleotide or polypeptide has been modified by the introduction of a heterologuous (or foreign) nucleic acid of the alteration of a native nucleic acid, or that the protein or polypeptide has been mdified by the introduction of a hteerologous amino acid.

The present invention further pertains the polynucleotide sequence comprising SEQ. ID NO: 4 that encodes the polypeptide mTAAR2.

The term "polynucleotide sequence" (e.g., a nucleic acid, polynucleotide, oligonucleotide, etc.) as used herein relates to a polymer of nucleotides comprising nucleotides A,C,T,U,G.

The present invention provides further an isolated or recombinant polypeptide comprising sequence SEQ. ID NO: 7. Said polypeptide was identified as trace amine associated receptor and was named mTAAR3.

The present invention also pertains the polynucleotide sequence comprising SEQ. ID NO: 6 that encodes the polypeptide mTAAR3.

The present invention provides further an isolated or recombinant polypeptide comprising sequence SEQ. ID NO: 9. Said polypeptide was identified as trace amine associated receptor and was named mTAAR4.

The present invention also pertains the polynucleotide sequence comprising SEQ. ID NO: 10 that encodes the polypeptide mTAAR4.

The present invention provides further an isolated or recombinant polypeptide comprising sequence SEQ. ID NO: 11. Said polypeptide was identified as trace amine associated receptor and was named mTAAR5.

The present invention also pertains the polynucleotide sequence comprising SEQ. ID NO: 10 that encodes the polypeptide mTAAR5.

The present invention provides further an isolated or recombinant polypeptide comprising sequence SEQ. ID NO: 13. Said polypeptide was identified as trace amine associated receptor and was named mTAAR6.

The present invention also pertains the polynucleotide sequence comprising SEQ. ID NO: 12 that encodes the polypeptide mTAAR6.

The present invention provides further an isolated or recombinant polypeptide comprising sequence SEQ. ID NO: 15. Said polypeptide was identified as trace amine associated receptor and was named mTAAR7a.

The present invention also pertains the polynucleotide sequence comprising SEQ. ID NO: 14 that encodes the polypeptide mTAAR7a.

The present invention provides further an isolated or recombinant polypeptide comprising sequence SEQ. ID NO: 17. Said polypeptide was identified as trace amine associated receptor and was named mTAAR7b.

The present invention also pertains the polynucleotide sequence comprising SEQ. ID NO: 16 that encodes the polypeptide mTAAR7b.

The present invention provides further an isolated or recombinant polypeptide comprising sequence SEQ. ID NO: 22. Said polypeptide was identified as trace amine associated receptor and was named mTAAR7d.

The present invention also pertains an isolated or recombinant polynucleotide sequence comprising SEQ. ID NO: 21 that encodes the polypeptide mTAAR7d.

The present invention provides further an isolated or recombinant polypeptide comprising sequence SEQ. ID NO: 24. Said polypeptide was identified as trace amine associated receptor and was named mTAAR7e.

The present invention also pertains an isolated or recombinant polynucleotide sequence comprising SEQ. ID NO: 23 that encodes the polypeptide mTAAR7e.

The present invention provides further an isolated or recombinant polypeptide comprising sequence SEQ. ID NO: 26. Said polypeptide was identified as trace amine associated receptor and was named mTAAR7f.

The present invention also pertains an isolated or recombinant polynucleotide sequence comprising SEQ. ID NO: 25 that encodes the polypeptide mTAAR7f.

The present invention provides further an isolated or recombinant polypeptide comprising sequence SEQ. ID NO: 28. Said polypeptide was identified as trace amine associated receptor and was named mTAAR8a.

The present invention also pertains an isolated or recombinant polynucleotide sequence comprising SEQ. ID NO: 27 that encodes the polypeptide mTAAR8a.

The present invention provides further an isolated or recombinant polypeptide comprising sequence SEQ. ID NO: 30. Said polypeptide was identified as trace amine associated receptor and was named mTAAR8b.

The present invention also pertains an isolated or recombinant polynucleotide sequence comprising SEQ. ID NO: 29 that encodes the polypeptide mTAAR8b.

The present invention provides further an isolated or recombinant polypeptide comprising sequence SEQ. ID NO: 32. Said polypeptide was identified as trace amine associated receptor and was named mTAAR8c.

The present invention also pertains an isolated or recombinant polynucleotide sequence comprising SEQ. ID NO: 31 that encodes the polypeptide mTAAR8c.

The present invention provides further an isolated or recombinant polypeptide comprising sequence SEQ. ID NO: 34. Said polypeptide was identified as trace amine associated receptor and was named mTAAR9.

The present invention also pertains an isolated or recombinant polynucleotide sequence comprising SEQ. ID NO: 33 that encodes the polypeptide mTAAR9.

The other member of the TAARs family mTAAR1 having the polynucleotide sequence SEQ. ID NO: 2 and the polypeptide sequence SEQ. ID NO: 3, was previously described (Borowsky, B et al., Trace amines: identification of a family of mammalian G protein-coupled receptors, Proc. Natl. Acad. Sci. U.S.A. (2001)98 (16), 8966-8971).

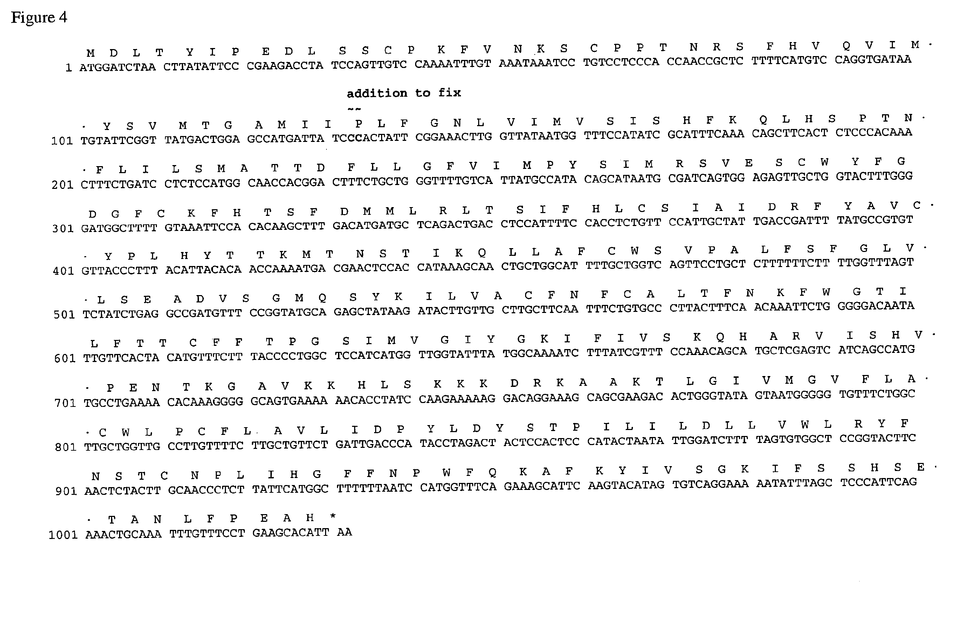

The present invention also provides the nucleotide sequences of mTAAR7cΨ (SEQ. ID NO: 18). Furthermore, the present invention provides the repaired nucleotide sequences of mTAAR7cΨ (SEQ. ID NO: 19). This invention further provides the polynucleotide sequences encoded by the fixed nucleotide sequence mTAAR7c (SEQ. ID NO: 20). The term "repaired gene" or "fixed gene" as used herein relates to a pseudogene whose sequence was altered in a way that the gene becomes active. The polynucleotide sequence of i.e. hTAAR3 is inactive due to a mutation that caused an early stop codon. The sequence was repaired by insertion of two nucleotides at position 133-134 (see Figure 4).

The present invention also provides isolated or recombinant polypeptides comprising an amino acid sequence which comprise the fingerprint sequence (SEQ. ID NO: 1) and differs from the sequence SEQ. ID NO: 3.

In a further aspect the invention relates to the use of the polypeptides of the invention as drug target. Preferably, the polypeptides of the invention are used as a drug target for identifying compounds useful in depression therapy, in schizophrenia therapy, in migraine therapy, or in therapy of attention-deficit hyperactivity disorder or eating disorder as anorexia or obesity. The drug target may be suitable for the design, screening and development of pharmaceutically active compounds.

One embodiment of the invention relates to the polypeptide having a polypeptide sequence SEQ. ID NO: 5, 7, 9, 11, 13, 15, 17, 22, 24, 26, 28, 30, 32 or 34 as drug target. Preferably, the polypeptides of the invention are used as a drug target for identifying compounds useful in depression therapy, in schizophrenia therapy, in migraine therapy, or in therapy of attention-deficit hyperactivity disorder or eating disorder as anorexia or obesity.

Another embodiment of the invention relates to mouse receptors having an amino acid sequence which comprise the fingerprint sequence and differs from the sequences SEQ. ID NOs: 3 as drug target. Preferably, these receptors are drug target for identifying compounds useful in depression therapy, in schizophrenia therapy, in migraine therapy, or in therapy of attention-deficit hyperactivity disorder or eating disorder as anorexia or obesity.

The term "polypeptide of the invention" relates to the novel polypetides provided in the present invention, i.e. mTAAR2 to mTAAR9.

The present invention also pertain vectors comprising polynucleotides of the invention, host cells which are transduced with said vectors and the production of the polypeptides of the invention by recombinant techniques. Preferably, the vector comprises the polynucleotide comprising SEQ. ID NO: 4, SEQ. ID NO: 6, SEQ. ID NO: 8, SEQ. ID NO: 10, SEQ. ID NO: 12, SEQ. ID NO: 14, SEQ. ID NO: 16, SEQ. ID NO: 19, SEQ. ID NO: 21, SEQ. ID NO: 23, SEQ. ID NO: 25, SEQ. ID NO: 27, SEQ. ID NO: 29, SEQ. ID NO: 31 or SEQ. ID NO: 33 and preferably, the host cells are transduced with said vector.

Host cells can be genetically engineered (i.e., transduced, transformed or transfected) to incorporate expression systems or portion thereof for polynucleotides of the present invention. Introduction of the vector into host cells can be effected by methods described in many standard laboratory manuals, such as Davis et al., Basic methods in molecular biology Elsevier, New York (1986); Davis JM (ed.): Basic cell culture: a practical approach, sec. edition. Oxford University Press (2002); R. Ian Freshney: Culture of Animal Cells: A Manual of Basic Technique, fourth edition. John Wiley & Sons (Sd) 1999; and Sambrook et al., Molecular cloning: a laboratory manual, 2. Ed., Cold Spring Harbour Laboratory Press, Cold Spring Harbor, N.Y (1989), such as calcium phosphate transfection, DEAE-dextran mediated transfection, transvectin, microinjection, cationic lopid-mediated transfection, electroporation, transduction or infection.

A host cell may be mammalian cell as HEK 293, CHO, COS, HeLa, neuronal, neuroendicrinal, neuroblastomal or glial cell lines like SH-SY5Y, PC12, HN-10 (Lee HJ, Hammond DN, Large TH, Roback JD, Sim JA, Brown DA, Otten UH, Wainer BH.: Neuronal properties and trophic activities of immortalized hippocampal cells from embryonic and young adult mice. J Neurosci. 1990 Jun;10(6):1779-87.), IMR-32, NB41A3, Neuro-2a, TE 671, primary neuronal or glia cells from mammals like rat or mouse, Xenopus oocytes, bacterial cells such as streptococci, staphylocooci, *E*. *coli, Streptomyces* and *Bacillus subtilis* cells; fungal cells such as *Saccharomyces cerevisiae* and *Aspergillus* cell; insect cells such as *Drosophila* S2 and *Spodoptera* Sf9 cells and plant cells.

A great variety of expression systems can be used. Such a system include, among others, chromosomal, episomal and virus -derived systems, i.e. vectors derived from bacterial plasmids, from bacteriophage, from transposons, from yeast episoms, from yeast chromosomal elements, from viruses such as *baculovirus,* papova viruses, such as SV40, vaccinia viruses, adenovirus, fowl pox virus, pseudorabies, retroviruses and vectors derived from combinations thereof, such as those derived from plasmids and bacteriophage genetic elements, such as cosmids and phagemids. The expression systems may contain control regions that regulate as well as engender expression. Generally, any system or vector suitable to maintain, progagate or express polynucleotides to produce a polypeptide in a host may be used. The appropriate nucleotide sequence may be inserted into an expression system by any of a variety of well-known and routine techniques, such as, for example, those set forth on Sambrook et al., Molecular cloning: a laboratory manual, 2. Ed., Cold Spring Harbour Laboratory Press, Cold Spring Harbour, N.Y. (1989) or Borowski et al Trace amines: identification of a family of mammalian G protein-coupled receptors. Proc Natl Acad Sci USA (2001) 98(16):8966-71).

The present invention further provides a transgenic non-human animal comprising a polynucleotide encoding a polypeptide of the invention. Preferably, the transgenic non-human animal comprises a polynucleotide comprising SEQ. ID NO: 4, SEQ. ID NO: 6, SEQ. ID NO: 8, SEQ. ID NO: 10, SEQ. ID NO: 12, SEQ. ID NO: 14, SEQ. ID NO: 16, SEQ. ID NO: 19, SEQ. ID NO: 21, SEQ. ID NO: 23, SEQ. ID NO: 25, SEQ. ID NO: 27, SEQ. ID NO: 29, SEQ. ID NO: 31 or SEQ. ID NO: 33.

The transgenic non-human animal may be any non-human animal known in the art. Preferably, the transgenic non-human animal is a mammal, more preferably the transgenic non-human animal is a rodent. Most preferably, the transgenic animal of the invention is a mouse.

Methods of producing a transgenic non-human animal are well known in the art such as for example those set forth on Hogan, B.C., F; Lacy, E, *Manipulating the Mouse Embryo: A Laboratory Manual.* 1986, New York: Cold Spring Harbor Laboratory Press; Hogan, B., et al., Manipulating the mouse embryo. 1994, Second Edition, Cold Spring Harbor Press, Cold Spring Harbor and Joyner, A. (ed.): Gene Targeting - A Practical Approach Second Edition. Practical Approach Series, Oxford University Press 1999.

The polypeptides of the invention may be employed in a screening process for compounds which bind the receptor and which activate (agonists) or inhibit (antagonists) activation of the receptor polypeptide of the present invention or compounds which modulate receptor function e.g by acting on receptor trafficking..

The present invention provides a method for identifying compounds which bind to a polypeptide of the invention comprising:
a) contacting the polypeptide of the invention with a candidate compound and
b) determing whether compound binds to the polypeptide of the invention.

The invention also provides a method for identifying compounds which have a stimulatory or inhibitory effect on the biological activity of a polypeptide of the invention or its expression comprising
a) contacting the polypeptide of the invention with a candidate compound and
b) determining if said compound has modulated the function or activity of the polypeptide of the invention.

In general, such screening procedures as described above involve producing appropriate cells which express the receptor polypeptide of the present invention on the surface thereof, however, the receptor expressed in appropiate cells may also be localized intracellularly. Such cells include i.e. cells from mammals, Xenopus, yeast, Drosophila or E. coli. Cells expressing the receptor (or cell membrane containing the expressed receptor) are then contacted with a test compound to measure binding, or stimulation or inhibition of a functional response.

One screening technique includes the use of cells which express receptor of this invention (for example, transfected CHO cells or HEK293) in a system which measures extracellular pH or intracellular calcium changes caused by receptor activation. In this technique, compounds may be contacted with cells expressing the receptor polypeptide of the present invention. A second messenger response, e.g., signal transduction, pH changes, PI hydrolyse, GTP-γ-[³⁵S] release or changes in calcium level, is then measured to determine whether the potential compound activates or inhibits the receptor.

Another method involves screening for receptor inhibitors by determining inhibition or stimulation of receptor-mediated cAMP and/or adenylate cyclase accumulation. Such a method involves transfecting a eukaryotic cell with the receptor of this invention to express the receptor on the cell surface. The cell is then exposed to potential antagonists in the presence of the receptor of this invention. The amount of cAMP accumulation is then measured. If the potential antagonist binds the receptor, and thus inhibits receptor binding, the levels of receptor-mediated cAMP, or adenylate cyclase, activity will be reduced or increased.

Another methods for detecting agonists or antagonists for the receptor of the present invention is the yeast based technology as described in U.S. Patent 5,482,835.

The assays may simply test binding of a candidate compound wherein adherence to the cells bearing the receptor is detected by means of a label directly or indirectly associated with the candidate compound or in an assay involving competition with a labeled competitor. Further, these assays may test whether the candidate compound results in a signal generated by activation of the receptor, using detection systems appropriate to the cells bearing the receptor at their surfaces. Inhibitors of activation are generally assayed in the presence of a known agonist and the effect on activation by the agonist by the presence of the candidate compound is observed. Standard methods for conducting such screening assays are well understood in the art.

Examples of potential polypeptide of the invention antagonists include antibodies or, in some cases, oligonucleotides or proteins which are closely related to the ligand of the polypeptide of the invention, e.g., a fragment of the ligand, or small molecular weight molecules such as e.g. metabolites of neurotransmitters or of amino acids, which bind to the receptor but do not elicit a response, so that the activity of the receptor is prevented.

Having now generally described this invention, the same will become better understood by reference to the specific examples, which are included herein for purpose of illustration only and are not intended to be limiting unless otherwise specified, in connection with the following figures.

### Figures

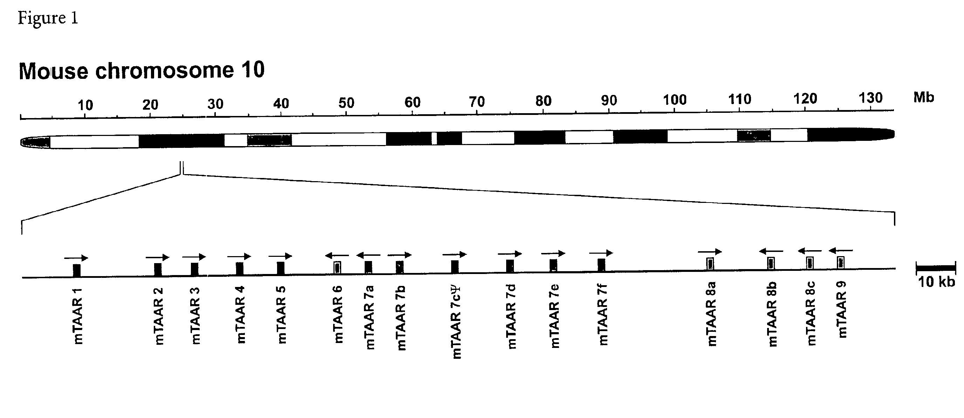

Figure 1 shows the chromosomal localization of TAARs genes in mouse. The relative position of TAAR genes on the chromosomes is indicated by boxes, representing the single coding exons of the respective genes. For mTAAR 2, which is encoded by two exons, the box indicates the position of the second coding exon harboring more than 95% of the open reading frame. The width of the boxes is not to scale with regard to the length of the respective coding sequences, and the colors indicate a distinction in receptor subgroups as discussed in the text. Arrows on top of the boxes indicate the orientation of the mTAAR genes (→: ORF in direction of the forward strand, ←: ORF in direction of the reverse strand). (Ψ= Pseudogene)

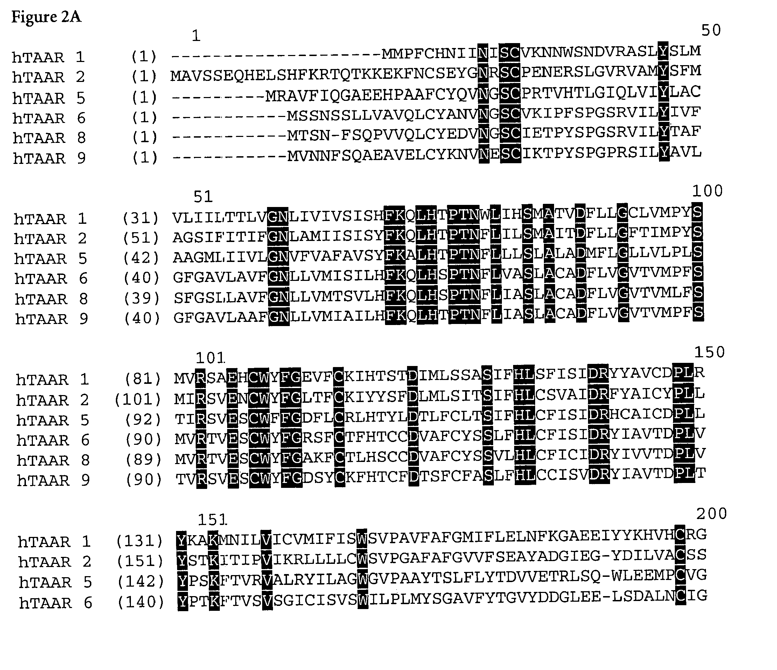

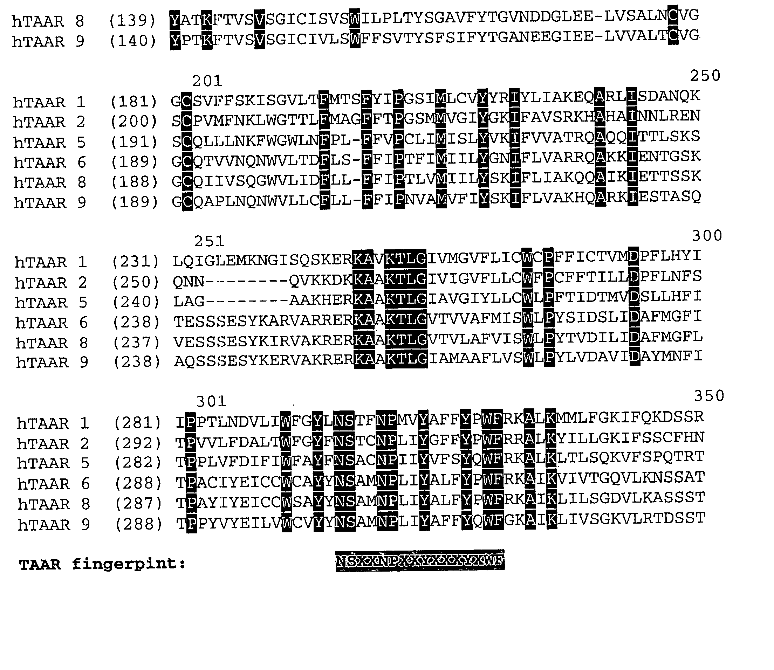

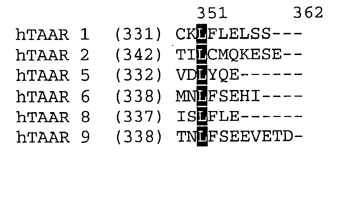

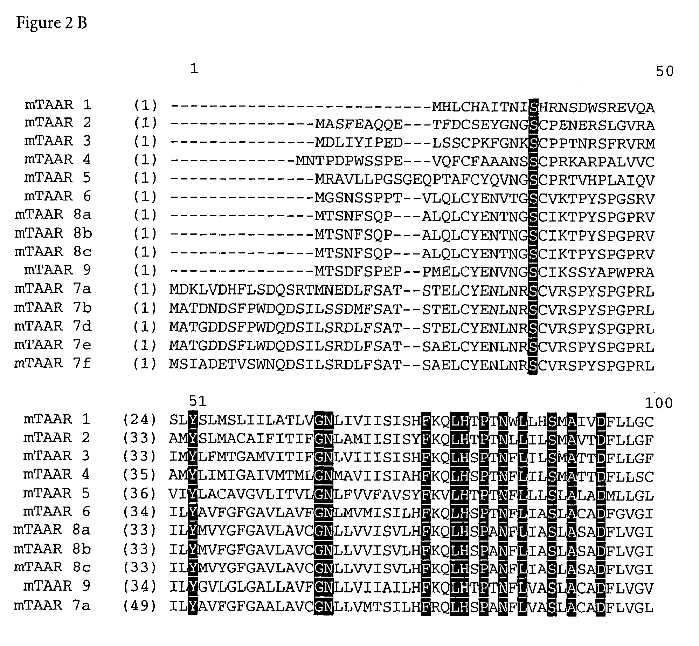

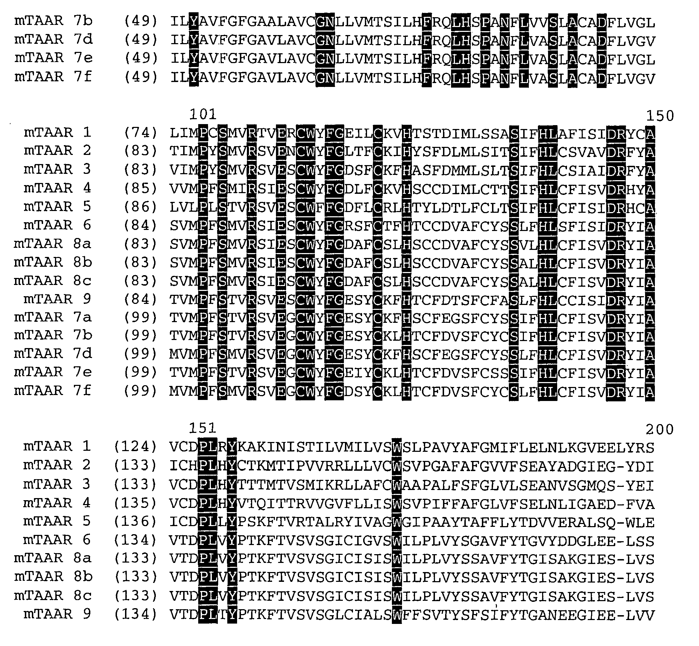

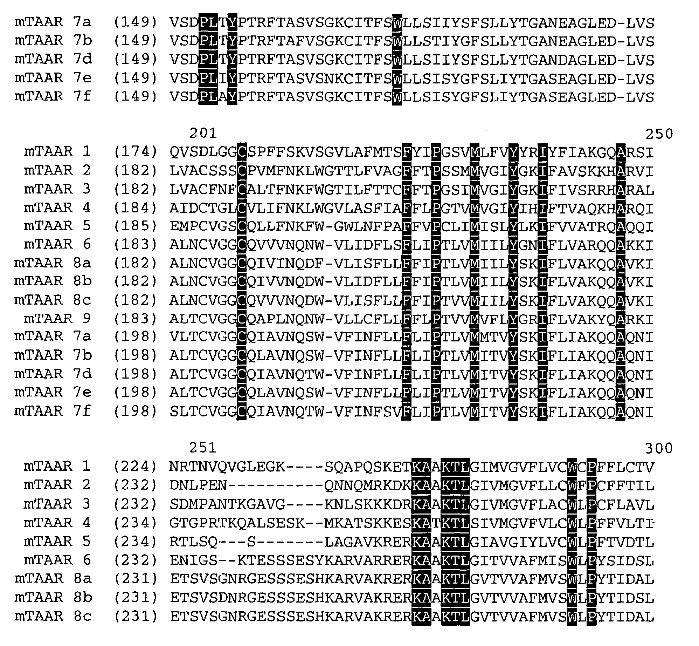

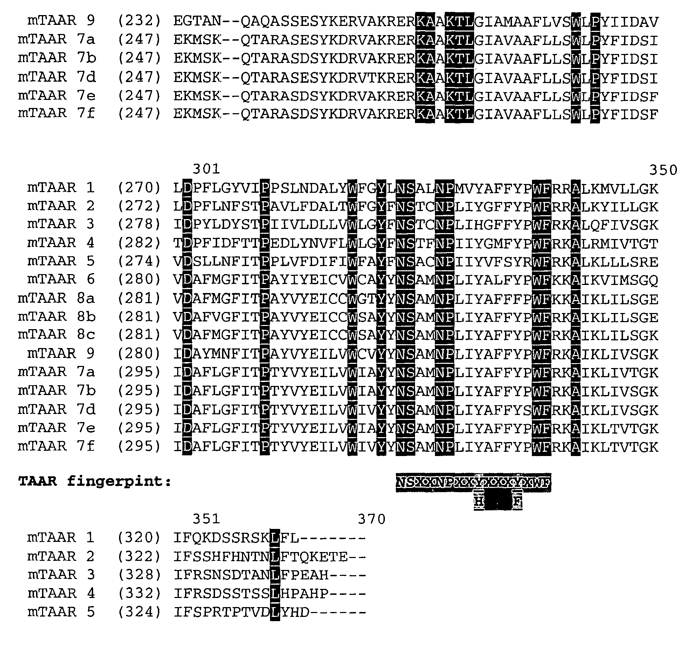

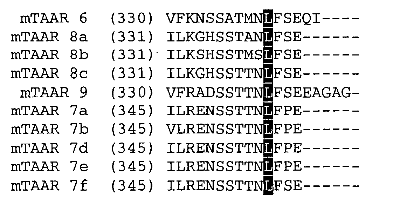

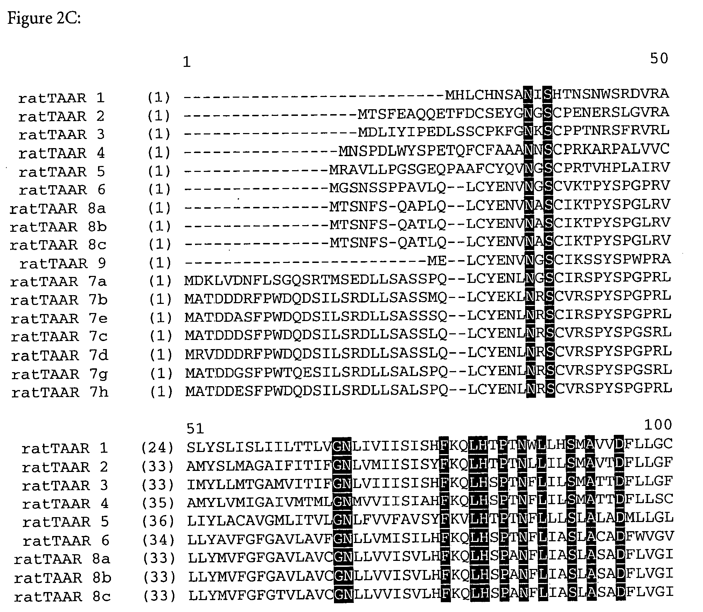

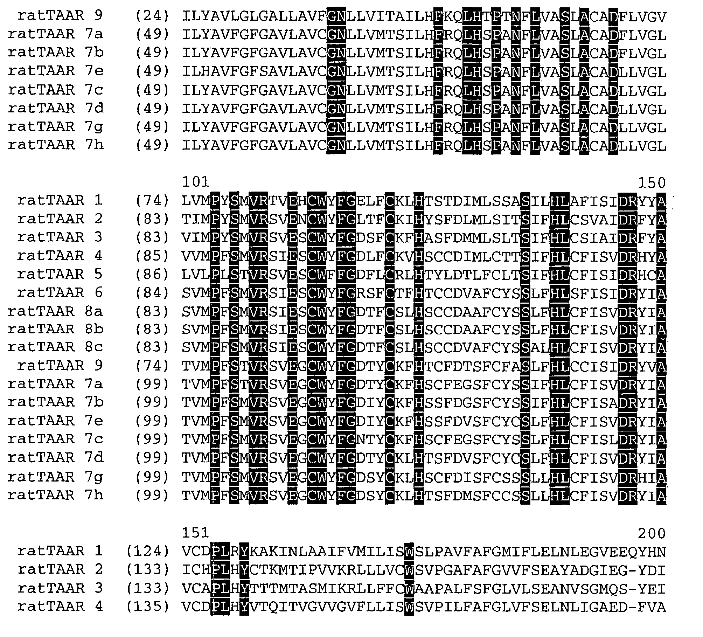

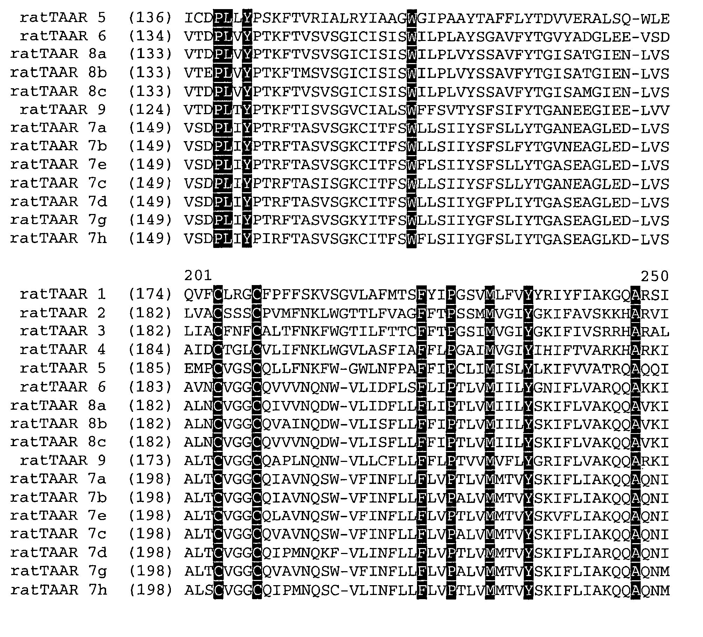

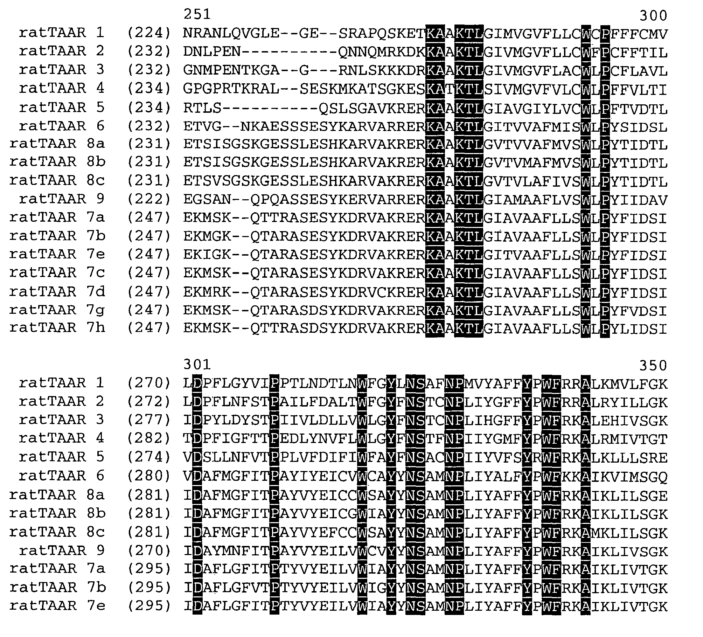

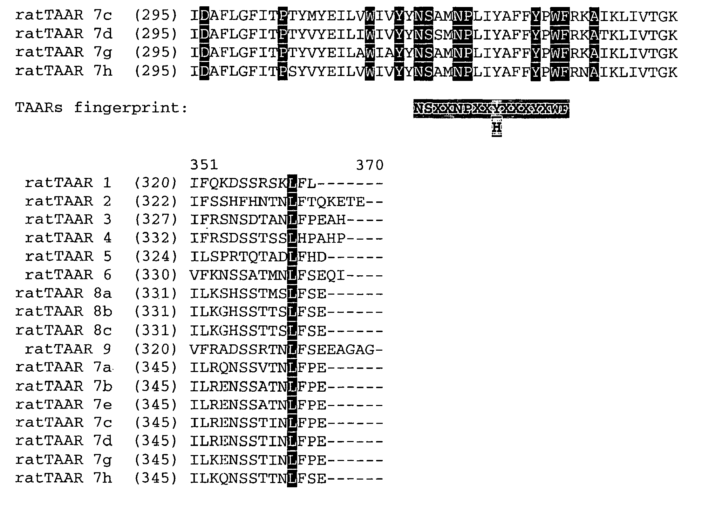

Figure 2 shows an alignment of all functional TAARs. Amino acid residues conserved in all human, rat and mouse TAARs are highlighted by black shading. The characteristic TAAR fingerprint motif is located in TM VII.
   A: alignment of functional human TAARs. **Predicted TM Positions: hTAAR 1** (TM I: 26-46, TM II: 60-80, TM III: 99-109, TM IV: 137-157, TM V: 189-209, TM VI: 253-273, TM VII: 288-308); **hTAAR 2** (TM I: 49-71, TM II: 80-102, TM III: 129-151, TM IV: 163-185, TM V: 208-230, TM VI: 263-285, TM VII: 300-322); **hTAAR 5** (TM I: 36-58, TM II: 71-93, TM III: 108-130, TM IV: 150-172, TM V: 203-225, TM VI: 253-270, TM VII: 285-307); **hTAAR 6** (TM I: 33-53, TM II: 69-89, TM III: 108-128, TM IV: 148-168, TM V: 203-223, TM VI: 260-276, TM VII: 283-302); **hTAAR 8** (TM I: 37-59, TM II: 66-88, TM III: 103-125, TM IV: 145-167, TM V: 198-220, TM VI: 258-280, TM VII: 290-312); **hTAAR 9** (TM I: 38-58, TM II: 69-89, TM III: 107-128, TM IV: 148-168, TM V: 198-218, TM VI: 260-280, TM VII: 295-315)
   B: alignment of functional mouse TAARs.
   C: alignment of functional rat TAARs.
Figure 3 shows the phylogenetic relationship of TAAR genes in human, rat and mouse based on their DNA sequence.
   Subsequent gene duplication events are indicated by numbers at the branching points ( 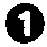 : Gene duplication from a putative common ancestor; 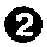 : Speciation leading to separate primates and rodent lineages; 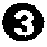 : Gene duplication leading to mouse and rat species). The outgroup was placed based on sequence comparison to the human 5-HT₄ receptor. The summary of TAAR genes into distinct subfamilies is indicated by boxes with grey shading. Ψ: Pseudogene; *: Gene fragment with closest similarity to rat TAAR 7h in the human genome resembling remnants of rodent TAAR 7 paralogues. Scale: JTT protein distances.
Figure 4 shows the "repair" of the pseudogene hTAAR 3 (old name GPR57ψ): The two bps CC in position 133-134 of the fixed ORF were added, which repairs the otherwise early stop codon TCA.

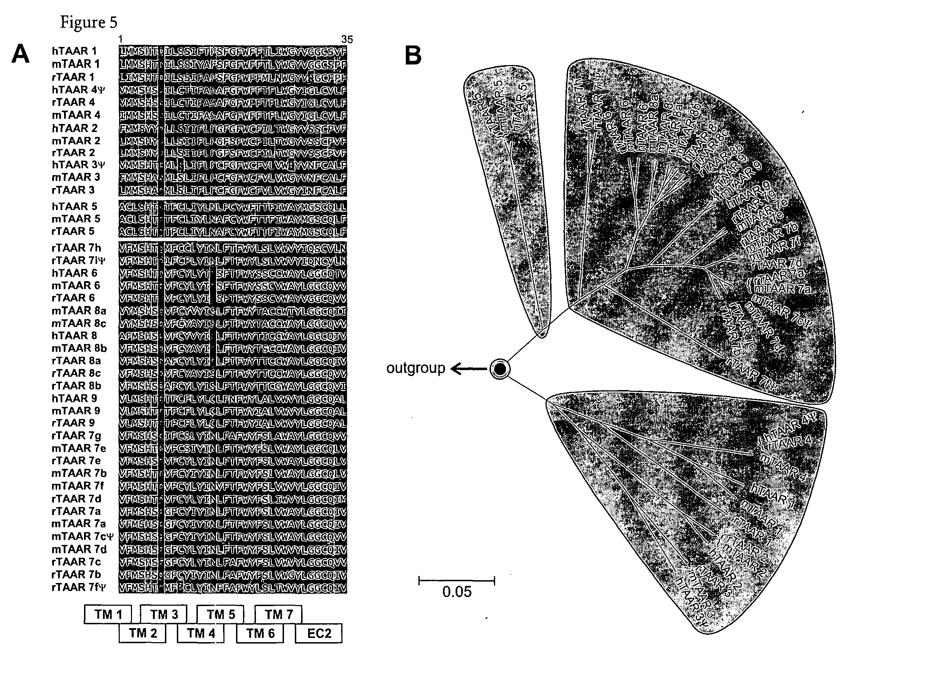

Figure 5 shows the relationship of TAARs based on the properties of the ligand pocket vectors (amino acid whose residues probably take part in the ligand binding, determined by calculation model).
   A: The Ligand Pocket Vectors (LPVs) of human, rat and mouse TAAR proteins, as predicted according to Kratochwil et al. (2004). Individual TAARs are ordered such that proteins with closest similar ligand binding pockets are placed next to each other. The physicochemical properties of the amino acid residues are indicated by colored shading (blue: hydrophobic/aromatic residues; green: aliphatic polar residues; red/plum: positively/negatively charged residues; orange/yellow: glycine/proline residues, potentially inducing kinks in the TM domains; pink: histidine; light blue: tyrosine). The position of the respective amino acids in the transmembrane domains (TM 1-7) as well as the extracellular loop II (EC II) is indicated by boxes in the lower part of the figure. For the precise position of predicted TM regions please refer to legend of figure 2.
   B: Hierarchical tree representation of the pharmacophore similarity of the ligand binding pockets of TAAR proteins. The following proteins were included into the analysis as "repaired pseudogenes", modification in parenthesis: hTAAR 3Ψ (insertions: C135-, A136-), hTAAR 4Ψ (pointmutation: T411A, deletions: -605G, -750A), mTAAR 7cΨ (insertion: C513-), rTAAR 7fΨ (pointmutation: G515A), rTAAR 7iΨ (insertion: A90-). Scale bar: Pharmacophore diversity units.

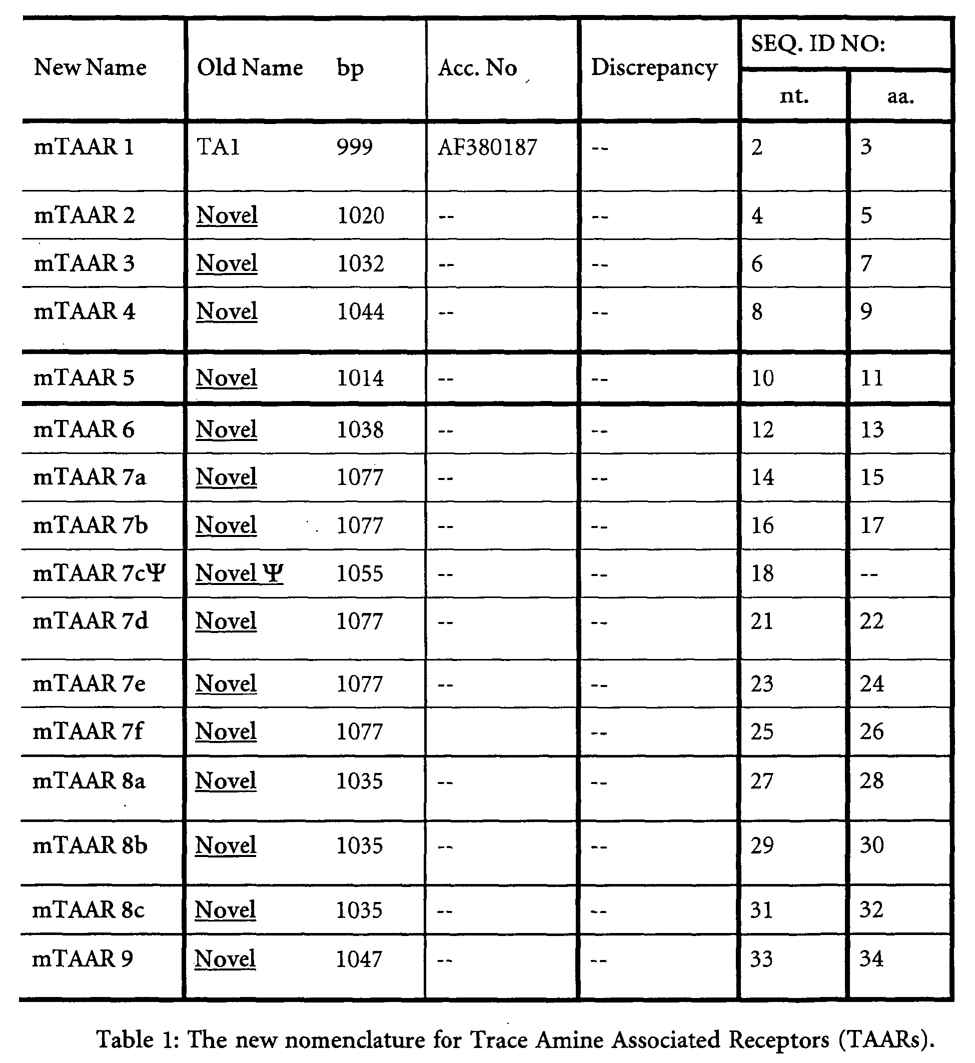

All genes for which discrepancies between published sequence data and the sequences provided by this invention were detected, as well as newly identified TAARs are underligned. Genbank accession numbers refers to published genes and the discrepancies between the published sequences and the sequences provided by the present invention. Cloning and sequence analysis of TAAR genes was carried out according to standard protocols employing genomic or cDNA from human (derived from total human brain total RNA, Stratagene). (nt=nucleotide sequence; aa= amino acid sequence).

### Examples

Commercially available reagents referred to in the examples were used according to manufacturer's instructions unless otherwise indicated.

### Example 1: Identification of TAAR family members

The TAAR genes were identified by comparison of the previously published TAAR receptor genes with the human, rat and mouse genomic sequence information available from Genbank (...see also Table 1) employing standard algorithms like BLAST.

Based on this sequence information the TAAR genes were amplified by means of PCR from either genomic DNA or cDNA of the respective species. Were not further specified, all procedures were carried out basically described in: Sambrook, J., Fritsch, E.F., und Maniatis, T. (1989). Molecular Cloning: A laboratory manual (New York: Cold Spring Harbor Laboratory Press). All reagents were of highest available purity, and all solutions and reagents were sterilized prior to use unless otherwise stated.

For this purpose oligonucleotide primers (table 2) were designed based on the TAAR coding sequences which had been derived from the genomic sequence information available at Genbank. The primers were designed such that the amplicons contain the entire open reading frames. The primer were designed using the VectorNTI software (Vector NTI version 9.0.0, Informax) following standard rules for PCR primers, mainly: a) Primers should have a length of 18-25 nt b) a G/C content of about 50% c) should not contain inverted repeats longer than 3 nt d) should carry an G or C at least at the 5' end e) should have no simple repetitions of the same nts more than 3, and f) the annealing temperatures (TM) of primers used in the same PCR reaction should differ as little as possible (for details see for example: McPherson M.J., Hames B.D., Taylor G.R. (eds.): PCR 2 - A Practical Approach. The practical approach series, Oxford University Press, 1995, ISBN: 0-19-963424-6). The annealing temperatures of the primer oligonucleotides were calculated based on rules described in: Breslauer KJ, Frank R, Blocker H, Marky LA.: Predicting DNA duplex stability from the base sequence. Proc Natl Acad Sci U S A. 1986 Jun;83(11):3746-50 (These rules assume that the sequences are not symmetric and contain at least one G or C and have a length of minimally 8 nts). Oligonucleotides were ordered from Microsynth (Microsynth AG, Schützenstrasse 15, 9436 Balgach, Switzerland) in HPLC purified quality.

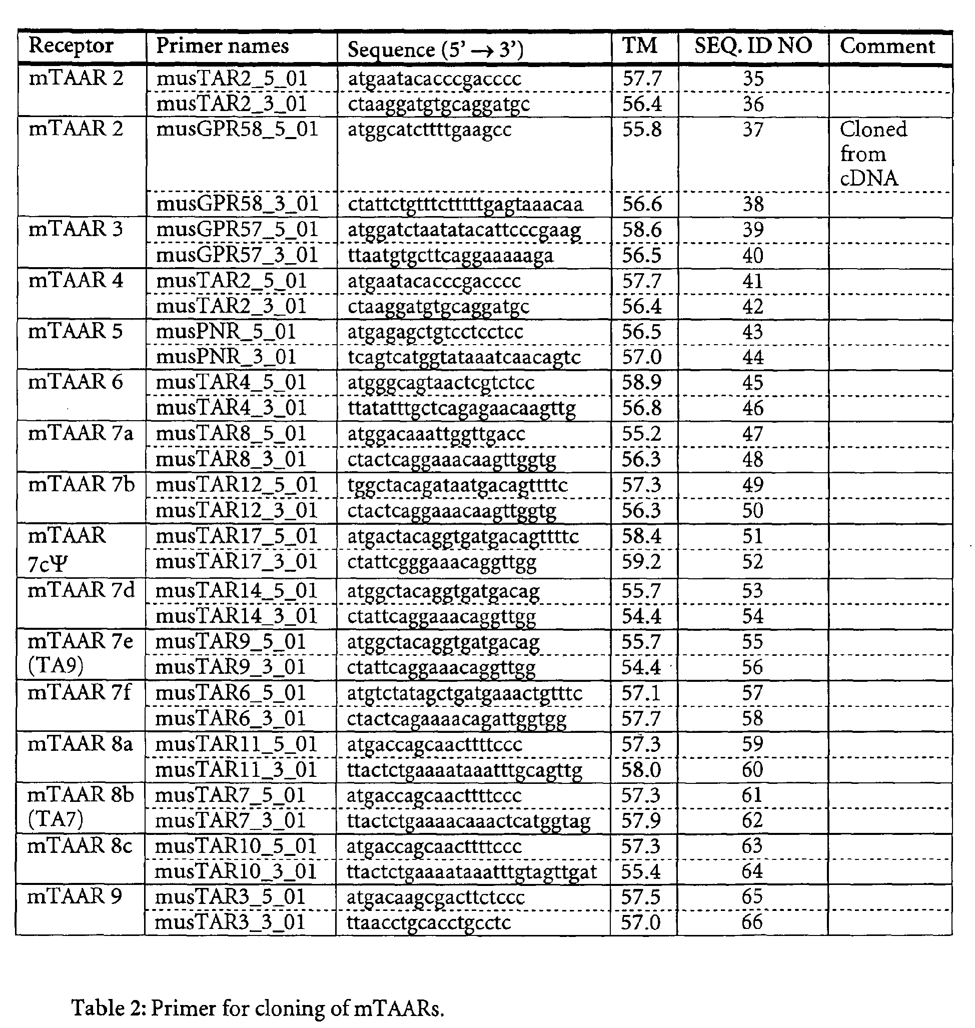

mTAAR2: The main reason of cloning the mTAAR 2 from cDNA was to assure whether the transcript of the corresponding genes would in fact contain the two exons. Independent from these experiments, the two coding exons have been cloned separately from genomic DNA. (Tm: melting temperature in °C calculated according to Breslauer KJ, Frank R, Blocker H, Marky LA.: Predicting DNA duplex stability from the base sequence. Proc Natl Acad Sci U S A. 1986 Jun;83(11):3746-50; 5 in the primer name indicates a 5' primer (i.e. musTAR3_5_01) and 3 in the primer name indicates a 3' primer).

The actual PCR reactions had a total volume of 50 µl and the following composition: Template equaling 5-100 ng genomic DNA or cDNA equivalent to 100-200 ng of total RNA (sources specified below), 200 nM oligonucleotide primer, 1.5 mM MgCl₂ (Invitrogen), 200 mM dNTPs each (Invitrogen), 1x concentrated PCR reaction buffer (Invitrogen) and 5 U/reaction recombinant Taq DNA polymerase (Inditrogen). The PCR reactions were assembled under a sterile working bench with UV irradiated equipment, and a) preparation of template material, b) assembly of PCR reactions, c) running the PCR reactions and agarose gel electrophoresis, and d) plasmid preparation were each performed in separated rooms in order to avoid any possible cross contamination of reagents with PCR amplified material or plasmid DNA. The PCR reactions were assembled in 200 µl PCR cups (Eppendorf) including Taq DNA polymerase at room temperature (RT) and transferred to the thermocycler (Geneamp 9700 with gold block, Applied Biosystems) preheated to 95°C. The temperature profile was adjusted as follows:

| | |
|---|---|
| 95°C | 2 min |
| (95 °C: 30 sec, annealing temperature: 30 sec, 72°C: extension time) x cycle number: | |
| 72°C | 5 min |
| 4°C | max. 6 hrs |

**annealing temperature**: the Tm (melting temperature) of the oligonucleotide primer with the lower Tm value for the PCR reaction (calculated as specified above) -1°C was used as annealing temperature. Example: 2 primers with Tm primer 1 = 55°C, Tm primer 2 = 57°C ⇒ annealing temperature = 54°C.
**extension time** was calculated by length of amplicon (in kb) x 1 min.
**cycle number**: For each gene several PCR reactions were run in parallel with cycle numbers between 25 - 40. All PCR reactions were subsequently analyzed by agarose gel electrophoresis, and the PCR reaction with the lowest cycle number still producing a clearly visible PCR product of the correct size was used for subsequent cloning.

The PCR products were analyzed by agarose gel electrophoresis using 1% ultraPure agarose (GibcoBRL) made up in TAE buffer (Invitrogen). Agarose gels were run in PerfectBlue Horizontal Mini Electrophoresis Systems (Pequlab Biotechnologie GmbH, Erlangen, Germany) according to standard protocols (Sambrook et al., 1989). Agarose gels were stained with ethidium bromide, PCR products were visualized on a UV transilluminator (Syngene, Cambridge, UK), and the size of the PCR products was analyzed in comparison with the molecular weight standard 1 kb DNA ladder (Invitrogen). PCR products of the expected sizes were cut out from the gels with sterile scalpels (Bayha, Tuttlingen, Germany) and extracted from the agarose gel slices using the QIAquick Gel Extraction Kit (QIAGEN AG, Basel, Switzerland) following the instructions of the manufacturer.

The extracted PCR products were precipitated with a cold ethanol/sodium acetate precipitation (Sambrook et al., 1989), and DNA pellets were dissolved in a volume of 10 µl of 10 mM Tris/HCl pH 7.5. The PCR products in this solutions were subsequently cloned using the TOPO cloning kit for sequencing (Invitrogen; kits containing the vectors pCR4-TOPO and pCR2.1-TOPO were used) following the instructions of the manufacturer. Ligations were transformed into TOP10 chemically competent bacteria (included in the TOPO cloning kit for sequencing) following the instructions of the manufacturer and subsequently plated on LB agar plates containing 100 µg/ml ampicillin (Sigma, Division of FLUKA Chemie GmbH, Buchs, Switzerland) and incubated at 37°C over night. The bacterial colonies were analyzed by a method called "miniprep PCR": Colonies were picked with a sterile inoculation loop (Copan Diagnostic S.P.A., Brescia, Italy) and transferred to new LB agar plates containing 100 µg/ml ampicillin. The same inoculation loops were subsequently transferred individually into 50 µl 10 mM Tris/HCl pH 7.5 in 1.5 ml Eppendorf Cups (Eppendorf) in order to transfer the bacteria which had remained on the loop into the solution. This bacterial suspensions were subsequently heated to 95°C for 5 min, and 1 µl pf the resulting bacterial lysates per 50 µl PCR reaction was used as template for PCR reactions with primers flanking the multiple cloning site of the plasmids pCR4-TOPO and pCR2.1-TOPO (T7 and M13 reverse, sequences: Primer "T7": 5' - cgggatatcactcagcataat - 3', primer "M13 reverse": 5' - caggaaacagctatgacc - 3'). PCR reactions were assembled as described above and run with the following temperature profile:

| | |
|---|---|
| 95°C | 2 min |
| (95 °C: 30 sec, 50°C: 30 sec, 72°C: 1 min) x 30 cycles | |
| 72°C | 5 min |
| 4°C | max. 6 hrs. |

The PCR products were analyzed by agarose gel electrophoresis as described above. Bacterial colonies for which cloned DNA fragments of the expected sizes could be detected by the method outlined above were used for subsequent plasmid preparation.

For plasmid preparations bacterial colonies identified to carry inserts of the expected sizes by "miniprep PCR" were used to inoculate bacterial liquid cultures in LB medium containing 100 µg/ml ampicillin, and cultures were incubated on a horizontal shaker at 37°C over night (Sambrook et al., 1989). Plasmids were isolated from the bacterial liquid cultures using the HiSpeed Plasmid Maxi Kit (QIAGEN AG, Basel, Switzerland) following the instructions of the manufacturer. Plasmid concentrations were determined by measuring the OD at 260 nm using an UV/VIS spectrophotometer ultrospec 3300 pro (Amersham Biosciences Europe GmbH, Otelfingen, Switzerland). The size of inserts in the isolated plasmids was analyzed by means of restriction digests using an restriction endonuclease i.e. EcoRI (New England Biolabs, products distributed in Switzerland by Bioconcept, Allschwil, Switzerland); EcoRI restriction sites are flanking the multiple cloning sites in both vectors pCR4-TOPO and pCR2.1-TOPO, therefore the cloned inserts can be released from the plasmids by EcoRI restriction digests. Of each plasmid 0.5 µg were digested with EcoRI in a total volume of 20 µl following the recommendations of the manufacturer. Restriction digests were analyzed by agarose gel electrophoresis as described above. Plasmids which turned out to carry inserts of the expected sizes as revealed by restriction analysis were subjected to DNA sequence analysis, carried out by an external company (Microsynth AG, Schützenstrasse 15, 9436 Balgach, Switzerland).

The DNA sequences of the cloned TAAR gene open reading frames, as revealed by DNA sequence analysis, were compared to either previously published sequences and/or to the sequence of the TAAR genes retrieved from the genome sequence information. Potential errors in the DNA sequence information which could have been introduced by the PCR reaction were eliminated by comparing the sequences of the two to three DNA plasmids which were cloned each from independent PCR reactions per TAAR gene: Since the probability of the same PCR error to occur at the very same position in independent PCR reactions is basically zero, the alignment of the independent sequences for each gene revealed the very correct DNA sequence.

**Template material**: We paid particular attention to use standardized template material derived, were applicable, from the same inbred strains from which the genome sequence information available at Genbank was derived. Mice of the inbred strain C57BL/6, or genomic DNA derived from this mouse strain, was purchased from Jackson Laboratory (600 Main Street, Bar Harbor, Maine 04609 USA), rats of the inbred strain Crl:Wi was purchased from Charles River Laboratories France (Les Oncins, France; belongs to Charles River Laboratories, Inc.,251 Ballardvale Street,Wilmington, MA 01887-1000,USA). Genomic DNA of tail biopsies taken from adult Crl:Wi rats was prepared basically according to: Laird PW, Zijderveld A, Linders K, Rudnicki MA, Jaenisch R, Berns A.: *Simplified mammalian DNA isolation procedure.* Nucleic Acids Res. 1991 Aug 11;19(15):4293. The DNA concentration was determined by measuring the OD at 260 nm as described above and the DNA concentration was adjusted to 100 ng/µl. The DNA was stored in aliquots at 4°C. Human genomic DNA was purchased from Stratagene.

cDNAs were either commercially purchased (human: Human brain, cerebellum Marathon-Ready cDNA, BD Biosciences Clontech), or made from total RNA. RNA was either commercially purchased (human: human adult total brain, male, total RNA, Stratagene, Stratagene Europe, P.O. Box 12085 1100 AB Amsterdam, The Netherlands) or extracted from postnatal day 3 C57BL/6 mouse or Crl:Wi rat pups as described below.

### RNA isolation:

RNA was isolated from tissue samples basically according to: Chomczynski, P. und Sacchi, N.: Single-step method of RNA isolation by acid guanidinium thiocyanate-phenol-chloroform extraction. (Chomzynski P, Sacchi N., Analytical Biochemistry (1987) Single-Step Method of RNA Isolation by Acid Guanidinium Thiocyanate-PhenolChloroform Extraction 162, 156-159. Basic rules and principles for working with RNA are described in detail in Sambrook et al. (1989).

Rat or mice pups were killed by decapitation. The total brains were removed, weighed, and transferred to a 5 ml glass douncer and homogenized in the 10x volume of Trizol Reagent (Invitrogen). Total RNA was extracted from this homogenate following the instructions of the manufacturer. The RNA pellet derived from each half brain was dissolved on 200 µl 10 mM Tris/HCl pH 7.0. Traces of genomic DNA were removed by DNAseI digest (addition of 10 µl 100 µl MgCl₂ and 5 µl RNAse free DNAse I, 10³ U/µl, Roche; incubation at 37°C for 1 hr). DNAse I was removed from the solution by phenol/chloroform extraction (H₂O saturated phenol pH 7.0, Sigma) according to Sambrook et al. (1989). The RNA was precipitated by cold ethanol/sodium acetate precipitate (Sambrook et al., 1989), dissolved in 10 mM Tris/HCl pH 7.0, and the RNA concentration was determined by measuring the OD at 260 nm as described above. This RNA preparation was used for the synthesis of cDNA.

cDNA synthesis: In an 1.5 ml Eppendorf cup was mixed: 2 µg total RNA in a total volume of 11µl, 1 µl 0.5 µg/µl oligo dT12-18 (Invitrogen). The mixture was heated to 70°C for 10 min, placed on ice, and the following reagents were added:

4 µl 5x reaction buffer (supplied with enzyme), 2 µl DTT (supplied with enzyme), 1 µl dNTPs (10 mM each, Amersham), 0.5 µl RNAse inhibitor RNAse-OUT (Invitrogen), 0.8 µl Superscript II Reverse Transcriptase (Invitrogen). The reaction was briefly vortexed, incubated for 1 hr at 42°C, the enzyme was inactivated by incubating at 70°C for 10 min, and the reaction was adjusted to a final volume of 100 µl with 10 mM Tris/HCl pH 7.0. The cDNA was stored in aliquots at -80°C prior to use.

In general, particular measures were taken to rule out sequence errors by the PCR reaction and by polymorphisms introduced by the template material: **a) Cycle number**: For each TAAR gene several PCR reactions with different cycle numbers were run, and the PCR reactions with lowest possible cycle number which still delivered a specific product in amounts sufficient for cloning were used. **b) Several independent clones per gene**: Each TAAR genes was cloned 2 or 3 times from independent PCR reactions, and the correct sequence was deduced from an alignment of the sequences of these independently generated plasmids: Since the likelihood of a PCR error to occur at the very same position in several independent PCR reactions is basically zero, the alignment of the sequence of these plasmids generates the very correct sequence of the gene. **c) Standardized template material:** Since differences between inbred strains can introduce significant inconsistencies in sequence information, we also paid particular attention in the choice of the template material employed for the cloning of TAAR genes: Mouse genomic DNA was purchased from Jackson Laboratory (600 Main Street, Bar Harbor, Maine 04609 USA) genomic DNA of the inbred strain C57BL/6 was used, since this strain was employed for the mouse genomic sequence database at Genbank), human genomic DNA was purchased from Stratagene, and rat genomic DNA was isolated from a rat of the inbred strain Crl:Wi purchased from Charles River Laboratories France (Les Oncins, France; belongs to Charles River Laboratories, Inc.,251 Ballardvale Street,Wilmington, MA 01887-1000,USA; the rat strain Crl:Wi was selected, since this strain was employed for the rat genomic sequence database at Genbank). For the synthesis of cDNA rat and mouse whole brain total RNA was isolated from a postnatal day 3 pup of either a Crl:Wi rat or a C57BL/6 mouse, employing the Trizol reagent following the recommendations of the manufacturer. Human total brain RNA was purchased from Stratagene. The cDNA was synthesized using Superscript II Reverse Transcriptase from Invitrogen and dNTPs from Amersham following the recommendations of the manufacturers.

### Example 2: Cell culture & generating stable cell lines

a) Subcloning: For expression of TAAR receptors in mammalian cell lines, DNA fragments carrying the entire TAAR open reading frames were subcloned from the pCR4-TOPO or pCR2.1-TOPO vectors (Invitrogen) to pIRES-NEO2 (Rees S et al., Bicistronic vector for the creation of stable mammalian cell lines that predisposes all antibioticresistant cells to express recombinant protein. Biotechniques. 1996 Jan;20(1):102-4, 106, 108-10).
   To this end, DNA fragments carrying the entire TAAR open reading frames were removed from the respective TOPO vectors by restriction digest with i.e. EcoRI and subsequent purification of the ~1kb fragments by agarose gel electrophoresis and gel extraction as described above. In parallel, the pIRES-NEO2 vector was linearized with EcoRI and dephosphorylated with shrimp alkaline phosphatase (Roche) following the instructions of the manufacturer. The DNA fragments carrying the entire TAAR open reading frames were ligated into the linearized pIRES-NEO2 vector using T4 DNA ligase (New England Biolabs) by mixing 30 ng of linearized pIRES-NEO2 vector, 100-300 ng of DNA fragments carrying the respective TAAR coding sequence, 1x ligation buffer (final concentration; provided with the enzyme as 10x concentrate) and 1 µl of T4 DNA ligase in a total volume of 20 µl. The reaction was proceeded at room temperature for 2-3 hrs, and the ligation was transformed into chemically competent TOP10 cells as described for the TOPO ligations. Plating of transformed bacteria and picking of bacterial clones was performed as described earlier for the PCR cloning of TAAR genes from genomic and cDNA. The picked bacterial clones were used to inoculate 5 ml liquid cultures in LB containing 100 µg/ml ampicillin. These liquid cultures were used for mini plasmid preparations using the QIAprep Miniprep Kit (QIAGEN), following the instructions of the manufacturer. The purified pIRES-NEO2 derived plasmid constructs were analyzed for the presence of inserts of the expected sizes by restriction analysis using EcoRI and agarose gel electrophoresis. Plasmids which were identified to carry inserts of the expected sizes were subjected to DNA sequence analysis carried out by Microsynth in order to make sure for the correct orientation and sequence of the inserts. Plasmids carrying the correct inserts in the correct orientation were used for expression of the respective TAARs in mammalian cell lines.
b) Cell culture: Basic cell culture handling and techniques used are described in Davis JM (ed.): Basic cell culture, sec. edition. Oxford University Press 2002, ISBN: 0199638535. TAARs were expressed in HEK cells (ATCC number: CRL-1573; described in Graham, F.L., Smiley, J., Russell, W.C., und Nairn, R. (1977). Characteristics of a human cell line transformed by DNA from human adenovirus type 5. Journal of General Virology 36, 59-74). The culture medium consisted of DMEM with Glutamax I, with sodium pyruvate, with pyridoxine, with 4500 mg/l glucose, Invitrogen Cat. # 31966-021; Penicillin/Streptomycin; fetal calf serum heat inactivated 10%. Cells were passaged at a ratio of 1:10 -1:30 using Trypsin/EDTA (Invitrogen Cat. # 25300-062).
c) Stable cell lines: For the generation of stably transfected cell lines HEK cells were transfected with the pIRES-NEO2 expression vectors of the respective TAARs by using Lipofectamin 2000 transfection reagent and Optimem 1 reduced serum medium with Glutamax (Invitrogen Cat. # 51985-026) following the recommendations given in the Lipofectamine 2000 datasheet. After 24 hr post transfection cells were trypsinated and transferred into 90 mm tissue culture dishes (Nunc) at dilutions between 1:10 -1:300 in culture medium supplemented with 1 mg/ml G418 which was renewed on a daily basis. After 7-10 days pot transfections well isolated clones were observed which were picked when they had reached a diameter of about 3 mm. Picking of clones was performed by detaching the entire clone from the culture dish using a 1 ml Gilson pipette, trypsinizing the clone in a 1.5 ml Eppendorf cup using Trypsin/EDTA (Invitrogen Cat. # 25300-062) and plating them into a 48 well dish, from where the cells were subsequently expanded by transferring them to increasing culture surface areas (48 well -12 well - 60 mm etc.). Whenever cells were transferred to a new plate they were carefully trypsinized. As soon as single clones were selected, the concentration of G418 in the culture medium was reduced to 500 µg/ml.

After sufficient expansion of the individual clones they were tested in a functional assay with the Amersham cAMP Biotrak Enzymeimmunoassay (EIA) System, using either the trace amines (p-tyramine, β-phenylethylamine (β-PEA), tryptamine, octopamine) or a selection of compounds (5-HT Serotonin, dopamine, norepinephrine and histamine). The success rate for generating stably transfected cell lines was about 1 out of 3 clones tested. This high success rate was made possible by the use of the pIRES vector, from which the TAARs are expressed in bicistronic transcripts carrying the TAAR CDSs and the NEO^{R} CDS in the same mRNA molecule.

**Table 3: Pharmacology of mTAAR1.**

| Mouse TAAR 1 | | |
|---|---|---|
| | EC₅₀(µM) | Max (%) |
| β-PEA | 0.66 +/- 0.21 | 100 |
| p-TYR | 1.37 +/- 0.33 | 100 |
| OCT | 19.71 +/- 4.78 | 100 |
| TRY | 1.99 +/- 0.38 | 100 |
| Dopamine | 11.76 +/- 5.43 | 50 |
| Serotonin, 5-HT | > 50.000 | |
| Norepinephrine | > 50.000 | |
| Histamine | > 50.000 | |
| N-methyl-β-PEA | 0.15 +/- 0.03 | 100 |
| N-methyl-p-tyramine | 1.02 +/- 0.39 | 100 |

The amount of cAMP produced by HEK293 cells stably expressing the indicated receptor in response to stimulation by each of the listed compounds was measured using the cAMP Biotrak EIA System (Amersham). Shown are the EC50 values in µM (means of at least three independent experiments; EC50 values are calculated from experiments carried out with 12 concentrations each in duplicates evenly distributed in the range of 30 µM - 0.1 nM). The maximal response is represented as percentage of the maximal cAMP levels induced by p-TYR. In the experiments with dopamine the EC50 values were calculated based on a maximal cAMP level of 50% compared to the level reached by p-Tyramine.

For all other mTAARs than mTAAR 1 a minimum of 10 independent clones were tested. If no responder to either trace amines or selected top compounds could be detected it was concluded that the respective TAAR was not responsive to the compounds at all under the defined test conditions.

**cAMP assay:** cAMP was assayed using the cAMP Biotrak Enzymeimmunoassay (EIA) System from Amersham as described in the product manual, following the so-called Non-acetylation EIA procdure. The general procedure is also described i.e. in Notley-McRobb L et al. (The relationship between external glucose concentration and cAMP levels inside Escherichia coli: implications for models of phosphotransferase-mediated regulation of adenylate cyclase. Microbiology. 1997 Jun;143 ( Pt 6):1909-18) and Alfonso A, de la Rosa L et al. (Yessotoxin, a novel phycotoxin, activates phosphodiesterase activity. Effect of yessotoxin on cAMP levels in human lymphocytes. Biochem Pharmacol. 2003 Jan 15;65(2):193-208.)

In the protocol the following minor modifications were introduced: After stimulation of HEK cells in 96 well plates the reaction was stopped by applying equal volume of pre cooled (-20°C) ethanol. Lysis of cells was not achieved by applying the lysis buffer supplied with the kit, but with transferring the 96 well plates to -80°C for a minimum of 6 hrs (up to several days). Thereafter the contents of the 96 well plates was dried at 50°C over night, and the material remaining in the 96 well plates was resuspended in 200 µl of the assay buffer (the buffer used for resuspending all reagents of the kit). The resuspension were assayed in serial dilutions in assay buffer.

### Phylogenetic Analysis

The human, mouse and rat genome sequences (human: NCBI reference draft 34, July 2003; mouse: NCBI draft 32, October 2003; rat: Rat Genome Sequencing Consortium draft 3, June 2003) were screened, employing an in-house genomic sequence analysis resource for the identification of all TAAR genes and in particular for the presence of previously unknown potential members of the TAAR family. This screen led to the identification of 44 TAAR genes overall in human, mouse and rat, among them 22 novel genes (including 2 pseudogenes and a human TAAR 7 gene fragment) and 11 genes with discrepancies to previously published sequences. The comparison of simple DNA and protein sequence, as well as of more complex parameters like the GPCR pharmacophore relationship of the ligand pocket vectors and the phylogenetic relationship of the TAAR genes with the genome sequences strongly support the conclusion that the TAAR receptor listed in Figure 3 represents the complete TAAR receptor family in human, mouse and rat. The identification of TAAR genes based on the genome sequence information was largely facilitated by the fact that all TAARs except TAAR 2 are encoded by single exons..

In this context the arrangements of the TAAR genes in the human, mouse and rat genomes were analyzed (for human see Fig. 1). The genes mapped to very compact regions spanning a total of about 109 kb in human (chromosome 6q23.1), 192 kb in mouse (chromosome 10A4) and 216 kb in rat (chromosome 1p12; all chromosomal regions as assigned by LocusLink), with no other annotated or predicted genes in the same chromosomal regions. With the exception of TAAR 2, which is encoded by two exons, the coding sequences of all TAAR genes are located in a single exon and are very similar in their length of about 1 kb throughout all three species (range: 999 - 1089 bp). It is interesting to note that the region 6q23.1 on human chromosome no. 6 is known as schizophrenia susceptibility locus (Levinson et al.,: *Multicenter linkage study of schizophrenia candidate regions on chromosomes 5q, 6q, 10p, and 13q: schizophrenia linkage collaborative group III.* Am J Hum Genet. 2000 Sep;67(3):652-63. Epub 2000 Aug 02.; Martinez et al., *Follow-up study on a susceptibility locus for schizophrenia on chromosome* 6q. Am J Med Genet. 1999 Aug 20;88(4):337-43.) which underlines the potential of TAARs as drug targets for psychiatric disorders.

In human TAAR 1-5, as well as TAAR 6, 8 and 9 were found to be arranged in two blocks of genes with an inverse orientation of the open reading frames. The most striking difference between human and rodents is the complete absence of any functional TAAR 7 paralogues from the human genome, which represent almost half of the entire TAAR family in rat and for which we detected only a degenerate gene fragment in human with closest similarity to rTAAR 7h. In addition, TAAR 3 and 4 are functional receptors in rodents, but pseudogenes in human. The pseudogenization of hTAAR 3Ψ and 4Ψ likely is a recent event, since these genes are overall very well preserved with only two and three bp changes in the coding sequences, respectively, rendering them non-functional. The different total number of nine TAAR 7 paralogues in rat (including two pseudogenes, rTAAR 7fΨ and rTAAR 7iΨ) and six TAAR 7 paralogues in mouse (including one pseudogene, mTAAR 7cΨ) represents a remarkable inter-rodent difference within a family of such closely related genes, which is reflected in strong predicted ligand binding differences between individual mouse and rat TAARs determined by the GPCR pharmacophore relationship of the ligand pocket vector analysis (see below). The strong differences detected in the predicted ligand binding pockets of receptors with highly similar overall protein sequence indicates that the overall amino acid sequence identity is no suitable measure for predicting the degree of similarity of mouse and rat TAARs with regard to their pharmacological profile, a fact requiring close attention for the development of behavioral animal models.

Triggered by the compact chromosomal arrangement of TAAR genes in human and rodents and the high number of very similar genes within each species, the phylogenetic relationship of TAAR genes was analyzed across all three species. The phylogenetic analysis shows that the TAAR genes originate in a putative common ancestor from which they evolved through a total of eight gene duplication events, leading to a set of nine genes before the primate and rodent lineage split (Fig. 2; gene duplication events from the common ancestor, speciations leading to human and primate lineage, and gene duplications within the rodent lineage are each collectively indicated by numbers 1, 2 and 3, respectively). In seven cases, these genes were further duplicated into orthologeous sets of three by the two speciation events leading to human, mouse and rat. The rodent TAAR 7 and 8 orthologues evolved by further, independent duplications within the rat and mouse lineages. It is important to note that these events can only have occurred in the sequential order outlined above.

The overall structure of the phylogenetic tree immediately suggested to us the distinction of the TAAR family into three subgroups (indicated by grey boxes in Fig. 3). As discussed in detail below it turned out that the concept of subgroups is not an arbitrary distinction inferred just by the overall DNA sequences, but it reflects significant functional differences.

There are a total number of five pseudogenes in human, rat and mouse. All pseudogenes could be transformed into functional proteins by introduction of minor changes. Following a description of the most obvious and likely mutational events that might have caused the pseudogenizations: hTAAR 3Ψ became a pseudogene most likely due to a deletion of two bps (CA) at position 135 causing an early termination. A pointmutation (A411T) turning a Cys into a stop codon and two insertions (G605, A750) might have caused the pseudogenisation of hTAAR 4Ψ. rTAAR 7fΨ has two obvious differences to all the other TAAR 7 members of 12 additional bps at position 726, probably caused by an insertion event and of a pointmutation (A515G) turning a Trp into a stop codon. The second rat pseudogene rTAAR 7iΨ misses 10 bps at position 90 causing an early termination, and mTAAR 7cΨ misses 15 bps at position 895 (intracellular loop 3) and 7 bps at position 513 causing an early termination. In summary, within the TAAR family pseudogenisation seems to be caused by all possible mutational events like pointmutations, deletion and insertions. The events seem to have occurred fairly recently due to the highly conserved overall sequence. The pharmacology of such repaired pseudogenes might give insights into the importance of TAARs in the context of adaptation processes during evolution.

## Claims

1. An isolated or recombinant polypeptide comprising SEQ ID NO: 5.

2. An isolated or recombinant polypeptide comprising SEQ ID NO: 7

3. An isolated or recombinant polypeptide comprising SEQ ID NO: 9.

4. An isolated or recombinant polypeptide comprising SEQ ID NO: 11.

5. An isolated or recombinant polypeptide comprising SEQ ID NO: 13.

6. An isolated or recombinant polypeptide comprising SEQ ID NO: 15.

7. An isolated or recombinant polypeptide comprising SEQ ID NO: 17.

8. An isolated or recombinant polypeptide comprising SEQ ID NO: 22.

9. An isolated or recombinant polypeptide comprising SEQ ID NO: 24.

10. An isolated or recombinant polypeptide comprising SEQ ID NO: 26.

11. An isolated or recombinant polypeptide comprising SEQ ID NO: 28.

12. An isolated or recombinant polypeptide comprising SEQ ID NO: 30.

13. An isolated or recombinant polypeptide comprising SEQ ID NO: 32.

14. An isolated or recombinant polypeptide comprising SEQ ID NO: 34.

15. Use of the polypeptide according any one of claims 1 to 14 as drug target.

16. Use of the polypeptide according to any one of the claims 1 to 14 as drug target for identifying compounds useful in schizophrenia therapy.

17. Use of the polypeptide according to any one of the claims 1 to 14 as drug target for identifying compounds useful in migraine therapy.

18. Use of the polypeptide according any one of the claims 1 to 14 as drug target for identifying compounds useful in depression therapy.

19. Use of the polypeptide according to any one of the claims 1 to 14 as drug target for identifying compounds useful in therapy of eating disorders.

20. Use of the polypeptide according to any one of the claims 1 to 14 as drug target for identifying compounds useful in therapy of attention deficit hyperactivity disorder.

21. An isolated or recombinant polynucleotide comprising SEQ. ID NO: 4.

22. An isolated or recombinant polynucleotide comprising SEQ. ID NO: 6.

23. An isolated or recombinant polynucleotide comprising SEQ. ID NO: 8.

24. An isolated or recombinant polynucleotide comprising SEQ. ID NO: 10.

25. An isolated or recombinant polynucleotide comprising SEQ. ID NO: 12.

26. An isolated or recombinant polynucleotide comprising SEQ. ID NO: 14.

27. An isolated or recombinant polynucleotide comprising SEQ. ID NO: 16.

28. An isolated or recombinant polynucleotide comprising SEQ. ID NO: 21.

29. An isolated or recombinant polynucleotide comprising SEQ. ID NO: 23.

30. An isolated or recombinant polynucleotide comprising SEQ. ID NO: 25.

31. An isolated or recombinant polynucleotide comprising SEQ. ID NO: 27.

32. An isolated or recombinant polynucleotide comprising SEQ. ID NO: 29.

33. An isolated or recombinant polynucleotide comprising SEQ. ID NO: 31.

34. An isolated or recombinant polynucleotide comprising SEQ. ID NO: 33.

35. A vector comprising the polynucleotide according to any one of claims 21 to 34.

36. A host cell comprising the vector according to claim 35.

37. A transgenic non-human animal comprising the polynucleotide according to 36.

38. A fingerprint motif comprising the sequence NSXXNPXXZXXXBXWF wherein X is any natural occurring amino acid, Z may be a tyrosine or a histidine and B may be a tyrosine or phenylalanine.

39. Use of a sequence comprising the fingerprint motif according to claim 38 for identifying TAARs.

40. An isolated or recombinant polypeptide having a sequence which comprises the fingerprint motif of claim 38 and differs from the sequences SEQ. ID NOs: 3.

41. A polypeptide according to claim 40 as drug target.

42. A polypeptide according to claim 40 as drug target for identifying compounds useful in schizophrenia therapy, in migraine therapy, in depression therapy, in therapy of eating disorder or in therapy of attention deficit hyperactivity disorder.

43. A method for identifying compounds which bind to a polypeptide according to any one of claim 1 to 14 or claim 40 comprising:
a) contacting the polypeptide according to any one of claim 1 to 14 or claim 40 with a candidate compound and
b) determining whether compound binds to said polypeptide.

44. A method for identifying compounds which have a stimulatory or inhibitory effect on the biological activity of a polypeptide according to any one of claim 1 to 14 or claim 40 comprising
a) contacting a polypeptide according to any one of claim 1 to 14 or claim 40 with a candidate compound and
b) determining if said compound has modulated the function or activity of said polypeptide.
